# EUROPEAN PATENT SPECIFICATION

(11) **EP 1 260 498 B1**
(45) Date of publication and mention of the grant of the patent: **09.07.2014**
(21) Application number: 01908206.4
(22) Date of filing: 01.03.2001
(51) Int. Cl.: C07C 275/40, C07C 317/42, C07C 317/22, C07C 311/47, C09K 9/02, C07D 229/00, C07D 493/10, C07D 307/85, C07D 405/04, B41M 5/26, B41M 5/30, B41M 5/333, B41M 5/337

(54) **NOVEL COLOR FORMER AND RECORDING MATERIAL**
FARBBILDNER UND AUFZEICHNUNGSMATERIAL
SUBSTANCE CHROMOGENE ET MATERIAU D'ENREGISTREMENT

(30) Priority: 02.03.2000 JP 2000057538; 03.03.2000 JP 2000059047; 03.03.2000 JP 2000059298; 04.09.2000 JP 2000267467
(43) Date of publication of application: 27.11.2002
(73) Proprietor: Chemipro Kasei Kaisha, Ltd., Hyogo (JP)
(72) Inventor: KABASHIMA, Kazuo, Yokohama-shi, Kanagawa 230-0075 (JP); KOBAYASHI, Hiroshi, Yokohama-shi, Kanagawa 233-0007 (JP); IWAYA, Tetsurou, Kawasaki-shi, Kanagawa 211-0041 (JP)
(74) Representative: Strehl Schübel-Hopf & Partner
(86) International application number: PCT/JP2001/001577
(87) International publication number: WO 2001/066515

(56) References cited:
- EP-A- 0 633 145
- EP-A- 0 709 225
- EP-A1- 0 832 757
- EP-A2- 0 160 402
- WO-A1-00/14058
- WO-A1-90/06975
- WO-A1-92/20747
- WO-A1-99/51692
- DE-A1- 3 938 972
- JP-A- 8 311 357
- JP-A- 9 263 624
- JP-A- 10 310 633
- JP-A- 10 337 951
- JP-A- 11 005 026
- JP-A- 61 035 444
- JP-B1- 45 002 594
- US-A- 4 384 102
- US-A- 5 055 567
- US-A- 5 780 483
- J. PARKER ET AL: THE JOURNAL OF ORGANIC CHEMISTRY, vol. 22, no. 5, 1 May 1957 (1957-05-01), pages 594-596, XP55023698, ISSN: 0022-3263, DOI: 10.1021/jo01356a624
- GAVRILENKO ET AL: "Synthesis and properties of 3-amino-3-pyrazolin-5-ones", JOURNAL OF ORGANIC CHEMISTRY, AMERICAN CHEMICAL SOCIETY, EASTON.; US, vol. 40, no. 19, 1 January 1975 (1975-01-01), pages 2720-2724, XP002294768, ISSN: 0022-3263, DOI: 10.1021/JO00907A004
- LALEZARI I ET AL: "Synthesis and investigation of effects of 2-[4-[[(aryl- amino)carbonyl]amino]phenoxy]-2-methyl- propionic acids on the affinity of Hemoglobin for Oxagen: Structure-Activity Relationships", JOURNAL OF MEDICINAL CHEMISTRY, AMERICAN CHEMICAL SOCIETY, US, vol. 32, no. 10, 1 October 1989 (1989-10-01), pages 2352-2357, XP002250595, ISSN: 0022-2623, DOI: 10.1021/JM00130A021
- KNORR ET AL: CHEM. BER., vol. 36, 1903, pages 1278-1283, XP002673380,
- 'ep0832757 - D16' 01 January 1998, XP055043641
- 'jp45002594 - D5' 01 January 1970, XP055043644
- 'us4384102 - D4' 01 January 1983, XP055043645
- 'ep0160402 - D3' 01 January 1985, XP055043646
- 'wo9951692 - D1' 01 January 1999, XP055043647

## Description

### TECHNICAL FIELD

The present invention relates to novel urea-urethane compounds. The present invention relates also to a novel color-producing composition using the urea-urethane compounds.

The color-producing composition of the present invention is useful as a color-producing composition for recording materials which use heat as a recording energy.

### BACKGROUND ART

Various chemical color-producing systems which use a recording energy such as heat, pressure or the like have been known. Of these systems, color-producing systems usually composed of a two-component color-producing system consisting of a colorless or light-colored dye precursor and a developer capable of causing color development on contact with the dye precursor have been known since early times and are commonly utilized in recording materials. There are, for example, pressure-sensitive recording materials which record using pressure, heat-sensitive recording materials which record using heat, and light-sensitive recording materials which record using light.

Pressure-sensitive recording materials have been generally used in the planar forms, similar to paper. In general, the pressure-sensitive recording material is obtained by dissolving a dye precursor in a suitable solvent, emulsifying the resulting solution to several microns, and forming the emulsion into microcapsules. A first layer (also referre to herein as upper paper) of paper obtained by coating a substrate with the microcapsules and a second layer (also referred to herein as under paper) of paper obtained by coating another substrate with a developer layer containing a developer are placed one upon the other so that the microcapsule-coated surface and the developer-coated surface face each other. When a pressure is applied to the resulting assembly by writing, striking or the like, the microcapsules are destroyed to release the contents including the dye precursor. The dye precursor transfers to the developer layer to come into contact with the developer, so that color development reaction occurs, resulting in recording of an image.

In recent years, a heat-sensitive recording method comprising recording by means of heat energy has been often adopted in various information machines such as facsimiles, printers, recorders and the like. A heat-sensitive recording material used in the heat-sensitive recording method has many excellent characteristics such as a high whiteness, appearance and feel which are similar to those of ordinary planar paper, and an excellent aptitude for recording, for example, a high color development sensitivity. The heat-sensitive recording method is advantageous, for example, in that an apparatus used in the method is small, requires no maintenance and produces no noise. Therefore, the range of use of the heat-sensitive recording method has been increased in various fields of, for instance, recorders for measurement, facsimiles, printers, terminals of computer, labels, and automatic vending machines for railroad tickets or the like.

In the heat-sensitive recording method, a recording material obtained by forming on a substrate a color-producing layer containing a two-component color-producing composition is mainly used, and the components of the heat-sensitive composition are brought into contact with each other by treating the recording material with heat supplied as the recording energy from a thermal head, a hot stamp, laser beams or the like, whereby color development and recording are carried out. Many compositions used as the color-producing composition are those obtained by using a colorless or light-colored, electron-donating dye precursor (in particular, a leuco dye) and an acidic developer such as a phenolic compound. An example of a recording material obtained by using a dye precursor is thermal paper obtained by using a combination of Crystal Violet lactone and 4,4'-isopropylidenediphenol (bisphenol A) as a heat-sensitive color-producing composition (see U.S. Patent 3539375, etc.).

As the dye precursor and developer used in each of the recording methods described above, an electron-donating compound and an electron-accepting compound, respectively, are mainly used. This is because the electron-donating compound and the electron-accepting compound have, for example, the following excellent characteristics: the dye precursor as electron-donating compound and the developer as electron-accepting compound come into contact with each other to give a nearly instantaneous developed color image with a high density; and a nearly white appearance can be obtained and various hues such as red, orange, yellow, green, blue, black, etc. can be obtained. However, the developed color image obtained is so poor in chemical resistance that the record disappears easily on contact with a plasticizer contained in a plastic sheet or an eraser, or a chemical contained in food or cosmetics. Also the developed color image is so poor in record storage stability that the record fades or, worse yet, disappears when exposed to sunlight for a relatively short period of time. Therefore, color-producing compositions comprising the dye precursor and the developer are limited in their use to a considerable extent, and their improvement is eagerly desired.

In recent years, phenolic compounds represented by bisphenol A are considered unsuitable for use because they are likely to be endocrine disrupters, and hence a non-phenolic developer is preferred.

For fulfilling such a request, for example, JP-A-59-115887 and U.S. Patent 4521793 disclose recording materials comprising a combination of color-producing compositions comprising an aromatic isocyanate compound and an imino compound, as recording materials having a high shelf stability. These references disclose various recording materials in which the two color-producing compositions are brought into contact with each other to be reacted, by application of recording energy such as heat, pressure, light or the like. The references describe the fact that various colors such as red, orange, yellow, light brown, dark brown, etc. can be developed by properly selecting the color-producing compositions. However, in the inventions disclosed in the references, the development of a black color is not yet sufficient and is eagerly desired in the case of recording materials commonly used at present.

JP-A-8-2111 and JP-A-8-2112 disclose heat-sensitive recording materials having a color-producing layer containing a colorless or light-colored dye precursor and a urea compound, as heat-sensitive recording materials obtained by using a non-phenolic developer. These recording materials, however, give a low coloring density and have an insufficient shelf stability.

JP-A-5-116459 discloses a heat-sensitive recording material having a heat-sensitive color-producing layer containing a colorless or light-colored dye precursor and a sulfonylurea compound. This recording material, however, gives a low whiteness and has an insufficient shelf stability.

EP 0 832 757 A1 discloses a thermosensitive recording material having a layer comprising an aromatic urea compound.

### DISCLOSURE OF THE INVENTION

The present invention is intended to provide a novel urea-urethane compound that exhibits excellent performance characteristics when used as developer in a color-producing composition.

The present invention is also intended to provide a novel color-producing composition excellent in image preservability and coloring density, and a the use of the color-producing composition for the production of a heat-sensitive recording material.

The present inventors earnestly investigated the synthesis of various compounds for color-producing composition and consequently found that specific compounds exhibit surprisingly excellent performance characteristics, whereby the present invention has been accomplished. Furthermore, the present inventors found that specific compounds exhibit surprisingly excellent performance characteristics in combination with a specific dye precursor, whereby the present invention has been accomplished.

That is, the present invention is described by the claims.

### BEST MODE FOR CARRYING OUT THE INVENTION

The present invention is explained below in detail.

Each of the urea-urethane compounds of the present invention has both at least one urea group and at least one urethane group.

In the urea-urethane compounds of the present invention, an aliphatic compound residue is bonded to the terminal oxygen atom of the urethane group. Therefore, the urea-urethane compounds can be obtained as compounds excellent in physical and chemical stability, in particular, heat stability. Moreover, the urea-urethane compounds can be obtained by using relatively inexpensive materials.

In addition, a starting alcohol compound for the aliphatic compound residue bonded to the terminal oxygen atom of the urethane group can easily be removed even if it remains unreacted in the synthetic reaction of the urea-urethane compound. Therefore, the urea-urethane compounds can be obtained with a high purity.

Although a concrete mechanism by which the urea-urethane compounds of the present invention function as a developer is unknown, it is conjectured that the function is due to the interaction between the urea group and the urethane group in the urea-urethane structure portion.

A process for synthesizing each of the urea-urethane compounds of the present invention is not particularly limited. A process in which the urea-urethane compound is synthesized by the reaction of an isocyanate compound with an alcohol compound and an amine compound is preferable because of its ease.

A process for producing the urea-urethane compounds of the present invention is not limited. This compound can be obtained, for example, by reacting an alcohol compound of the following formula (m) with an isocyanate compound of the following formula (n) and an amine compound of the following formula (o) according to, for example, the reaction formula (A') or (B') shown below:

**R-OH** (m)

wherein R is an aliphatic compound residue, R-OH being defined below;

**OCN-A₁-NCO** (n)

wherein A₁ is an aromatic compound residue; and

A₂-NH₂ (o)

wherein A₂ is an aromatic compound residue, compounds (n) and (o) being defined below.

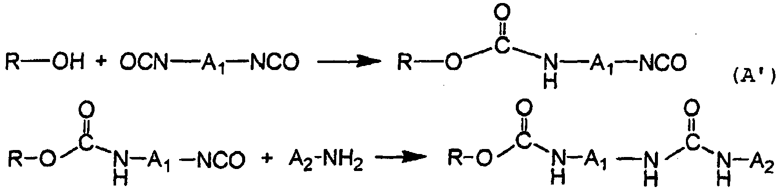

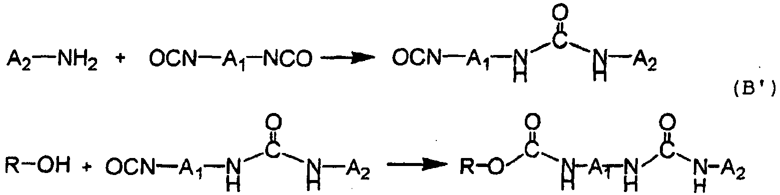

Compounds of the present invention also can be obtained, for example, by reacting an alcohol compound of the formula (m) with an isocyanate compound of the following formula (p) and an amine compound of the following formula (q) according to, for example, the reaction formula (C') or (D') shown below: 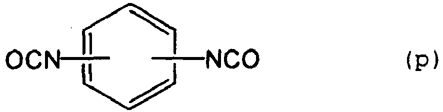 ; and 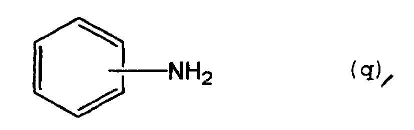 compounds (p) and (q) being defined below.

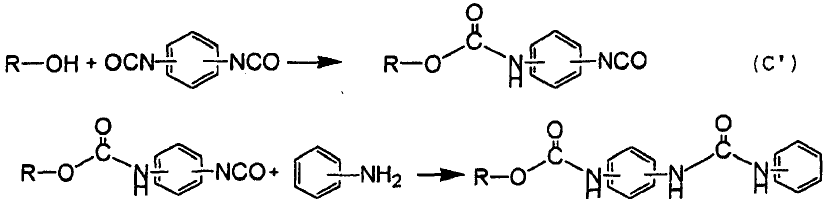

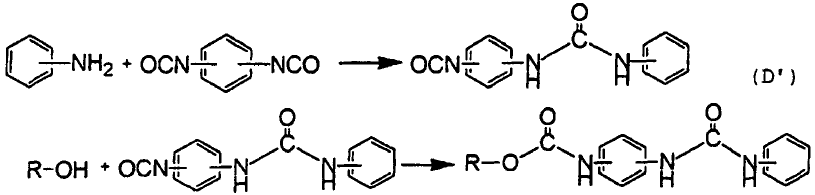

Compounds of the present invention also can be obtained, for example, by reacting an alcohol compound of the general formula (m) with an isocyanate compound of the following general formula (r) and an amine compound of the following general formula (s) according to, for example, the reaction formula (E') or (F') shown below:

**OCN-Y-NCO** (r)

; and 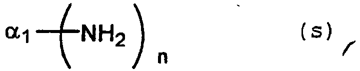 compounds (r) and (s) being defined below.

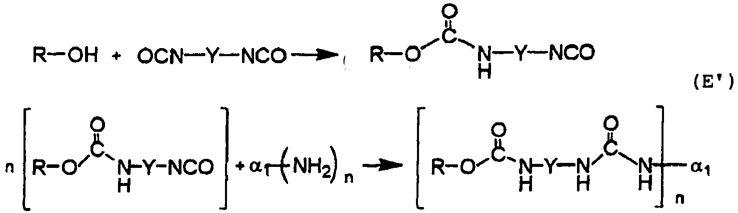

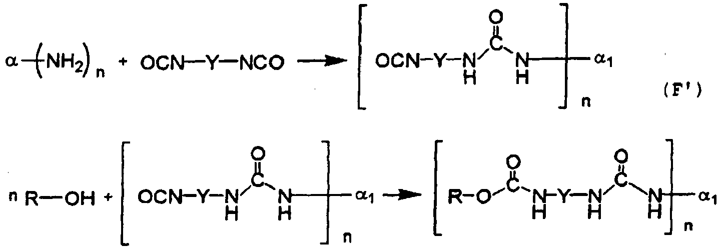

Compounds of the present invention also can be obtained, for example, by reacting an amine compound of the following general formula (t) with an isocyanate compound of the general formula (r) and an alcohol compound of the following general formula (u) according to, for example, the reaction formula (G') or (H') shown below:

**Z₁-NH₂** (t)

; and 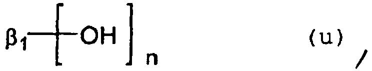 compounds (t) and (u) being defined below.

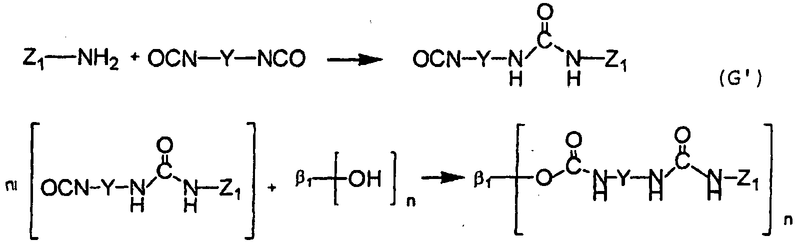

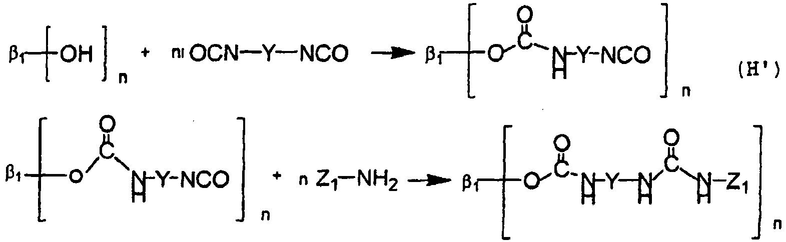

Compounds of the present invention also can be obtained, for example, by reacting an alcohol compound of the formula (u) with an isocyanate compound of the formula (p) and an amine compound of the formula (q) according to, for example, the reaction formula (i') or (J') shown below:

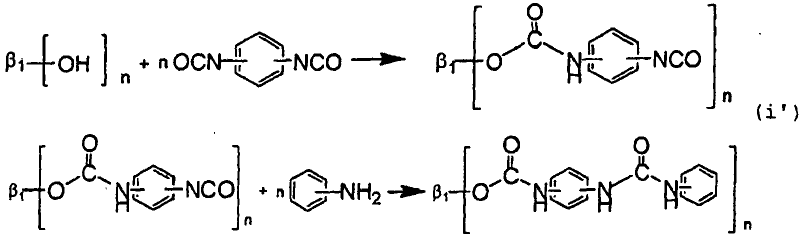

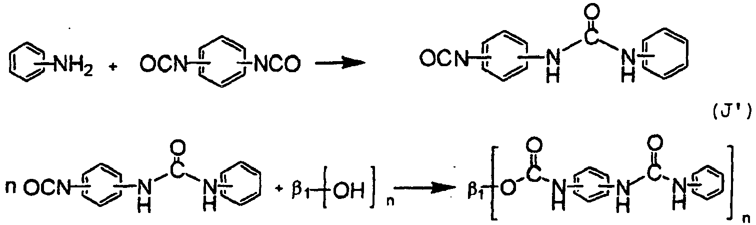

Compounds of the present invention also can be obtained, for example, by reacting an amine compound of the following formula (XVII) with an isocyanate compound of the formula (p) and an alcohol compound of the general formula (m) according to, for example, the reaction formula (K') or (L') shown below: 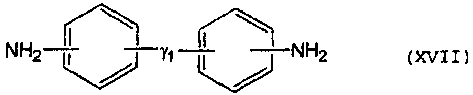 wherein compound (XVIII) is defined below.

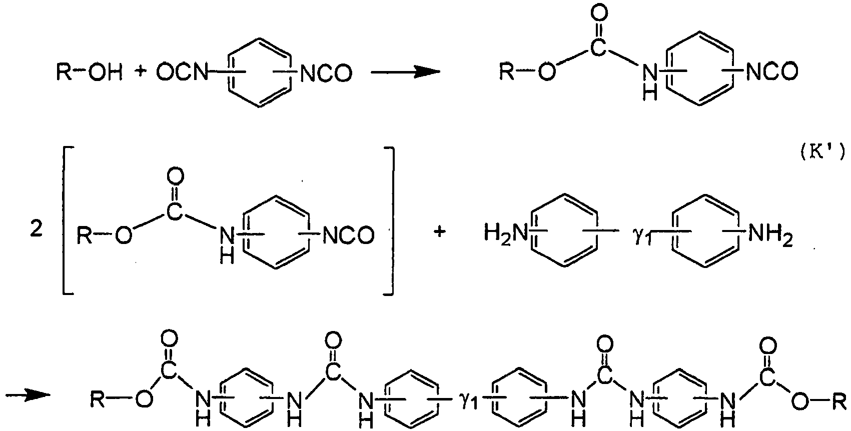

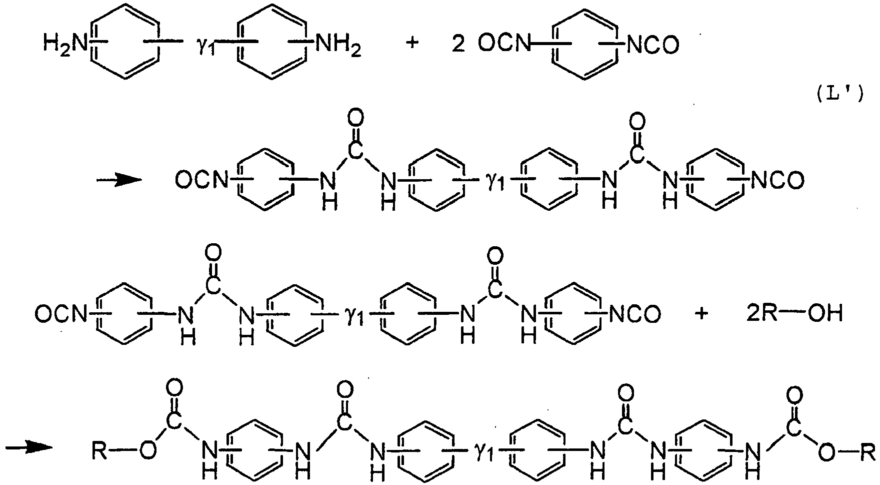

The compounds of the formulas (m) to (u) are explained below in further detail.

As the alcohol compound of the general formula (m), any of the following alcohols may be used so long as a compound of the present invention is obtained: methanol, ethanol, propanol, isopropanol, n-butanol, isobutanol, sec-butanol, hexanol, cyclohexanol, decanol, octadecyl alcohol, benzyl alcohol, ethylene glycol monobutyl ether, ethylene glycol monophenyl ether, dipropylene glycol monomethyl ether.

The isocyanate compound of the formula (n) is 2,4-toluene diisocyanate.

As the amine compound of the general formula (o), a compound is used by which a compound of the present invention can be obtained, i.e. aniline.

The aromatic isocyanate compound of the formula (p) is 2,4-toluene diisocyanate.

The aromatic amine compound of the formula (q) is aniline.

The isocyanate compound of the formula (r) is 2,4-toluene diisocyanate.

The amine compound of the general formula (s) is 4, 4'-diaminodiphenyl-methane or 4,4'-diaminodiphenyl sulfone.

The amine compound of the general formula (t) is aniline.

The alcohol compound of the general formula (u) is a polyol compound having two or more OH groups selected to provide a compound of the present invention. The alcohol is selected from ethylene glycol, diethylene glycol, 1,2-propanediol, dipropylene glycol, glycerin, trimethylolpropane, pentaerythritol and triethanolamine.

The amine compound of the general formula (XVII) is 4,4'-diaminodiphenylmethane or 4,4'-diaminodiphenyl sulfone.

For obtaining each of the urea-urethane compounds of the present invention, the isocyanate and the corresponding reactants are mixed to be reacted in an organic solvent or without a solvent, after which filtration, crystallization, desolvation, etc. are carried out to collect crystals, whereby the desired compound is obtained. The reaction may be carried out by a method in which a material having two or more groups in the molecule is used in large excess and another material to be reacted with the groups is added thereto in small portions. When this method is adopted, it is possible to react only one of the two or more groups. In the addition, it is preferable to stir the system thoroughly to effect sufficient dispersion of the added material immediately after the addition. The above applies to any of the urea-urethane compounds exemplified herein. A method for the reaction is not limited to the above method and any method may be adopted so long as the same result as above can be obtained. As each of the reactants, one or more compounds may be used depending on purposes. As the solvent, any solvent may be used so long as it does not react with an isocyanate group and the functional groups of the reactants. The solvent includes, for example, aliphatic hydrocarbons, alicyclic hydrocarbons, aromatic hydrocarbons, chlorinated aliphatic hydrocarbons, chlorinated aromatic hydrocarbons, chlorinated alicyclic hydrocarbons, and ketones. Methyl ethyl ketoneand toluene are especially preferable which dissolve the isocyanate and in which the reaction product has a low solubility. The reaction product obtained by the above reaction procedure is not always a single compound, but is obtained as a mixture of compounds different in the position of a substituent, in some cases.

The urea-urethane compounds of the present invention are the following compounds ((S-1) to (S-25), (S-28) to (S-60) and (S-63) to S-70)).

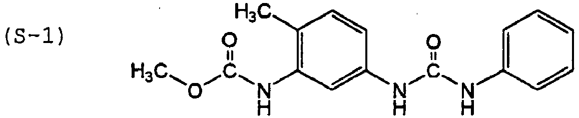

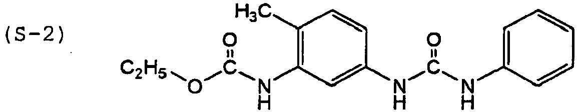

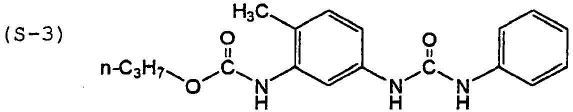

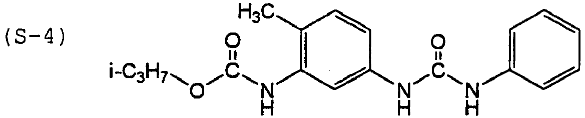

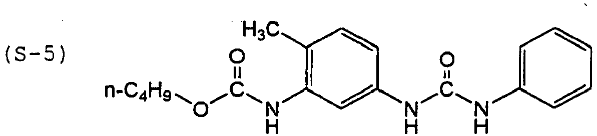

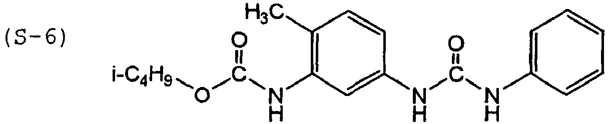

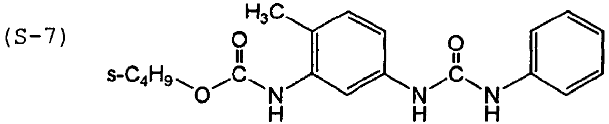

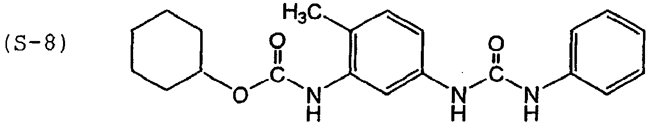

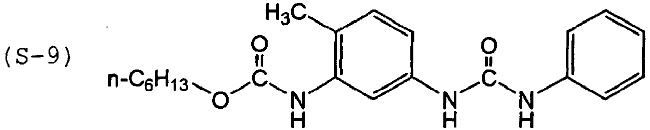

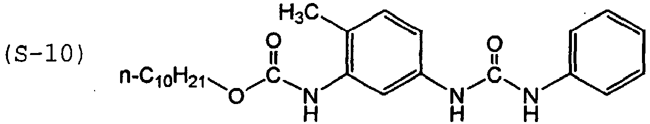

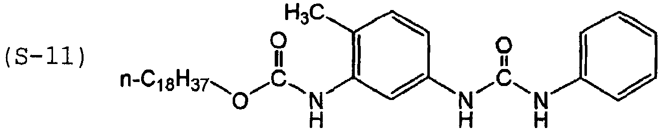

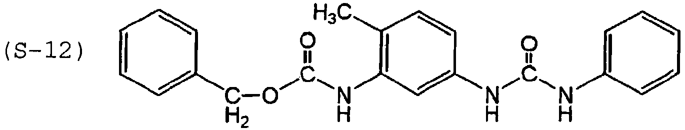

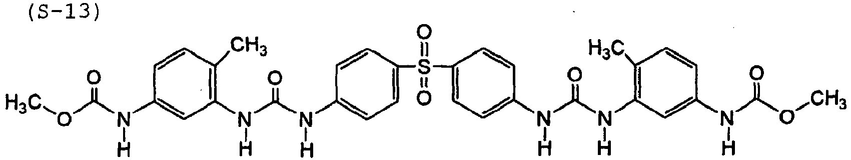

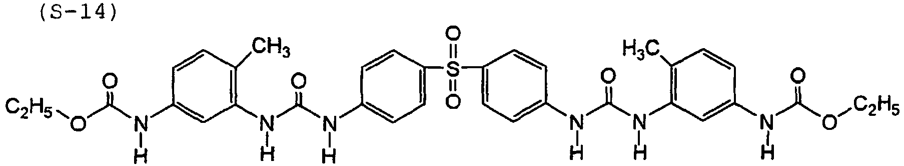

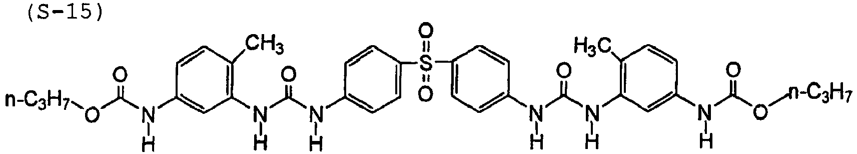

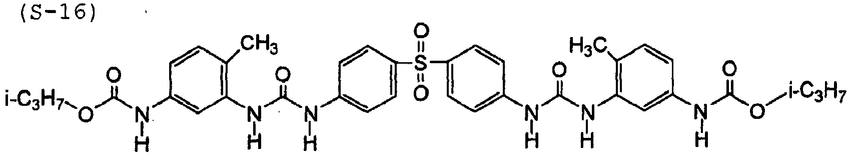

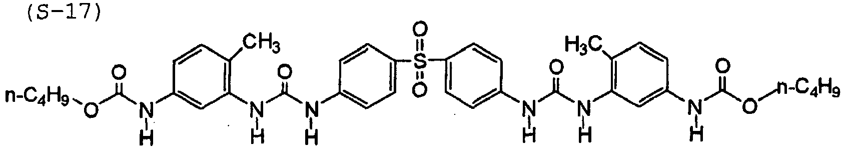

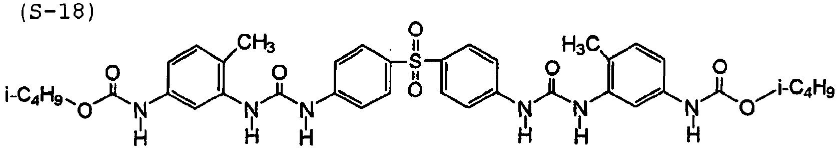

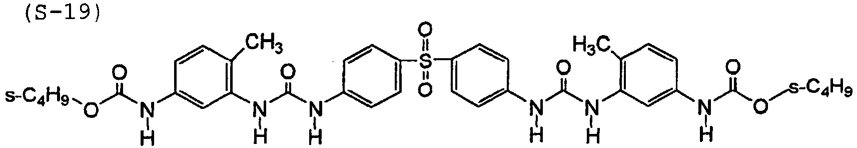

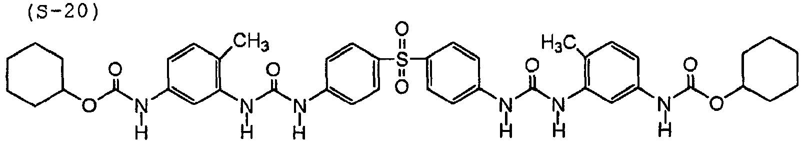

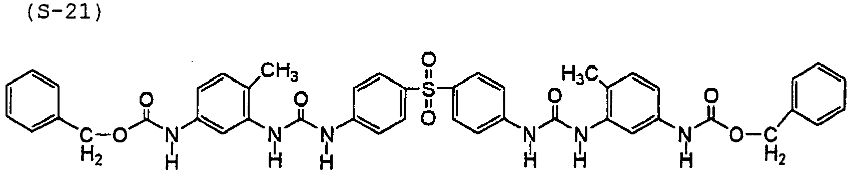

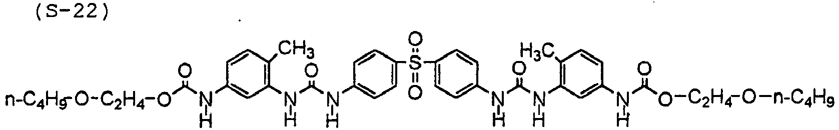

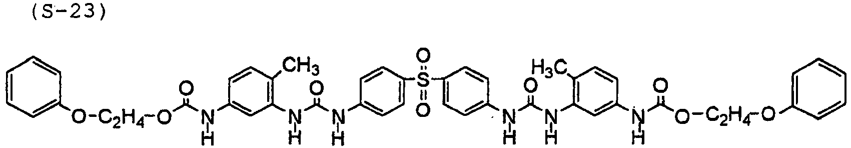

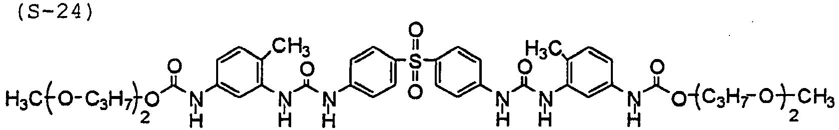

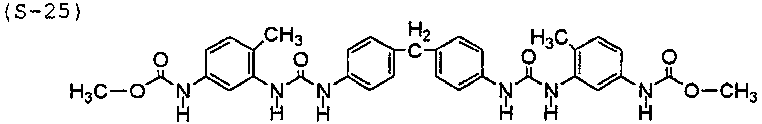

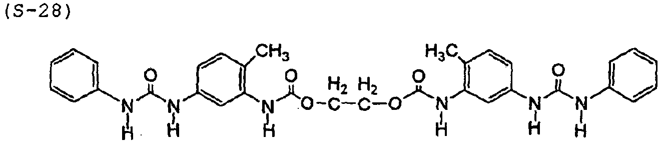

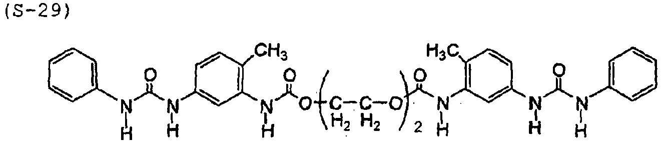

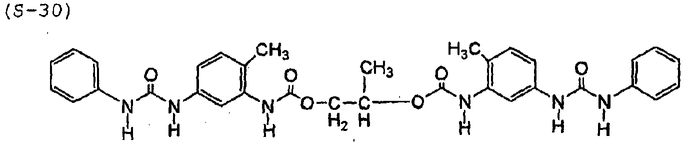

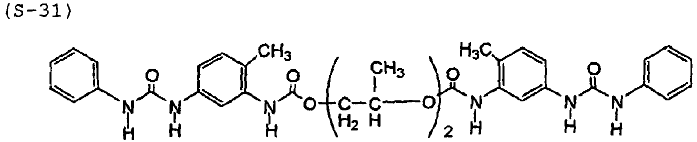

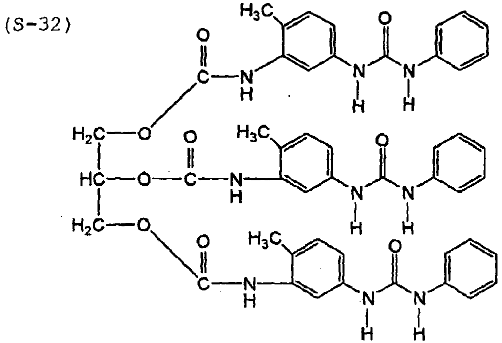

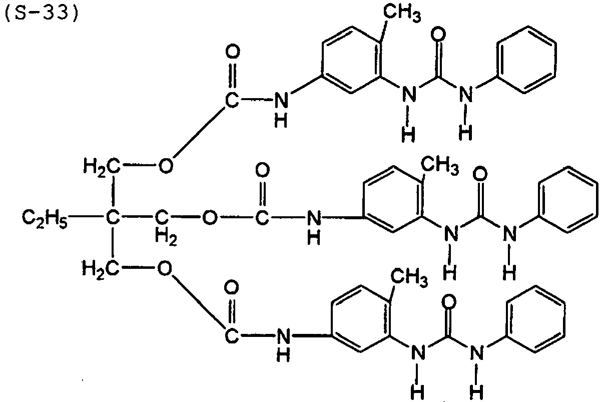

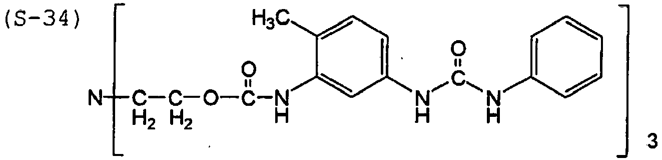

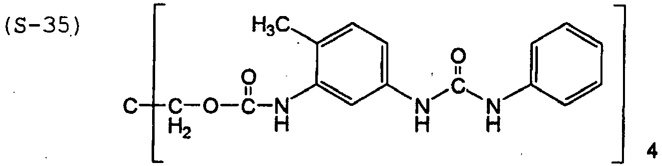

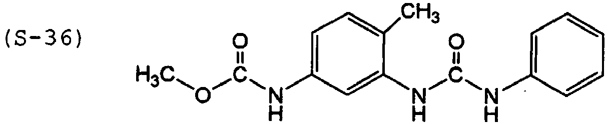

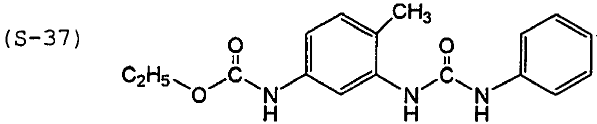

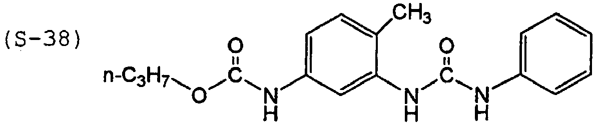

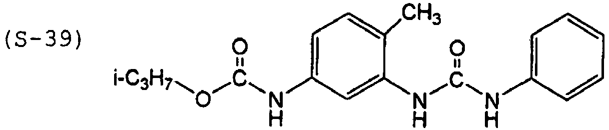

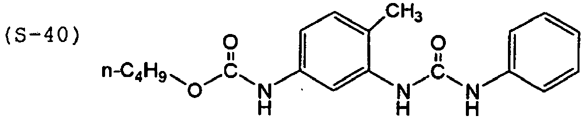

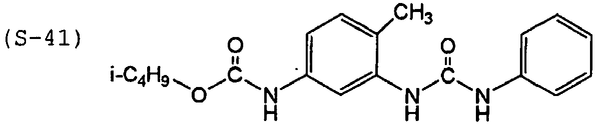

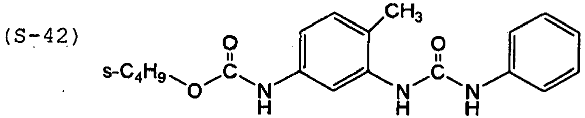

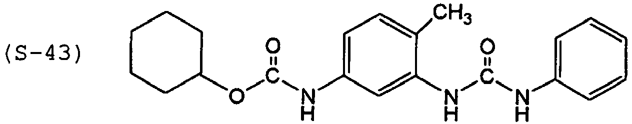

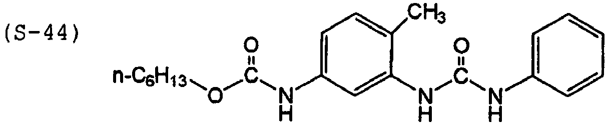

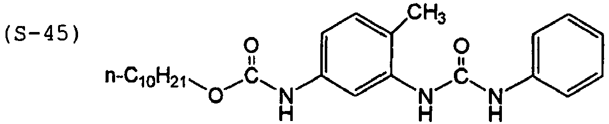

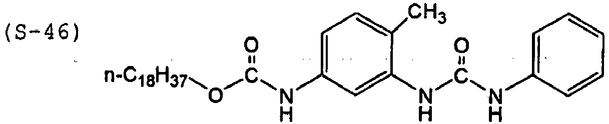

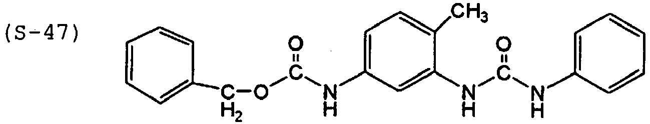

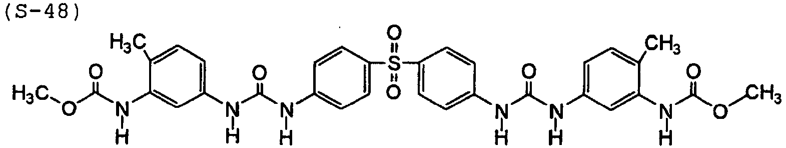

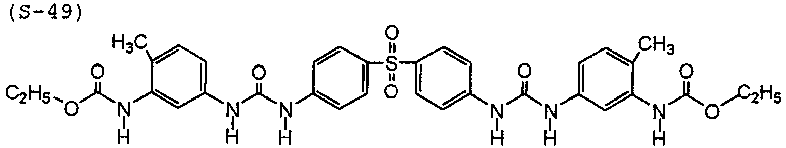

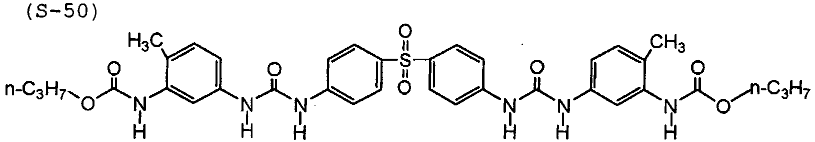

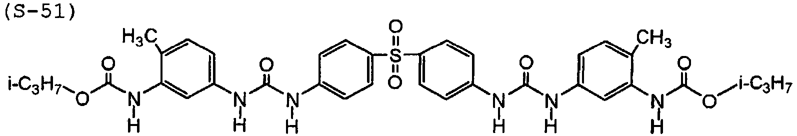

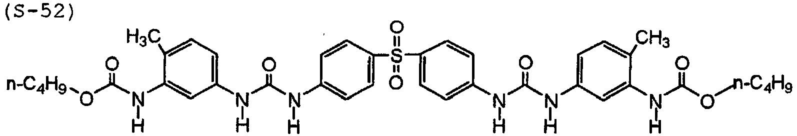

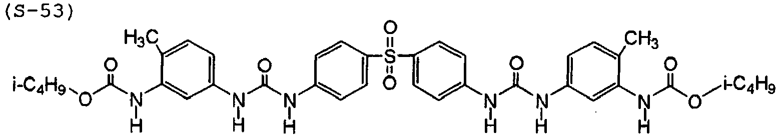

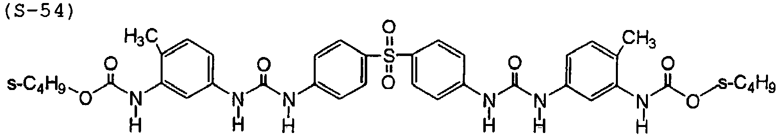

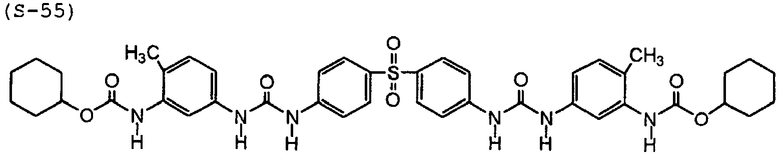

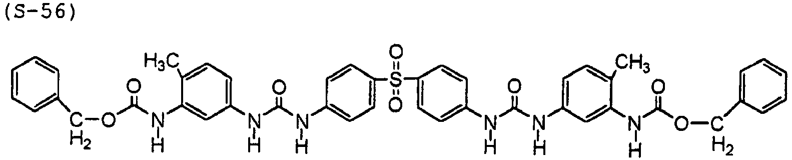

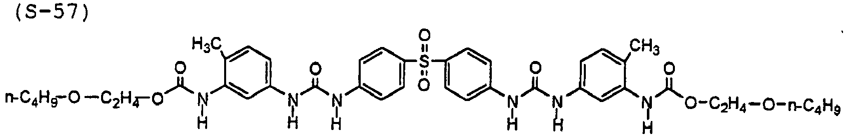

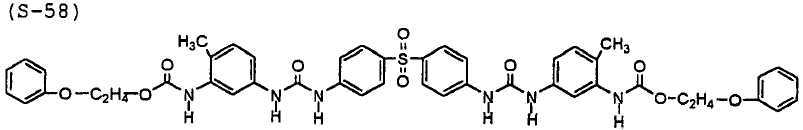

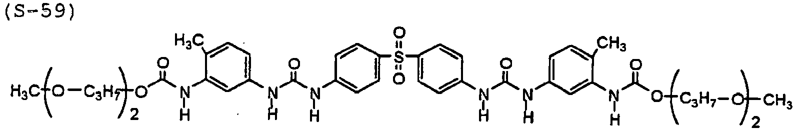

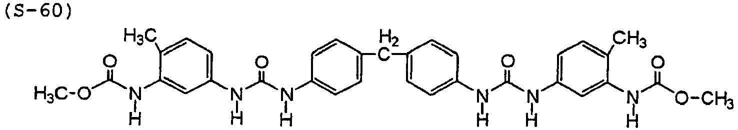

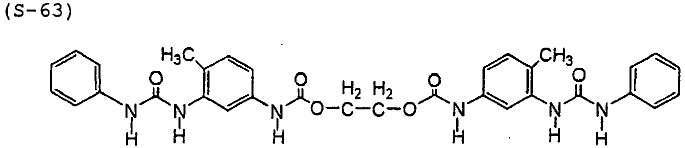

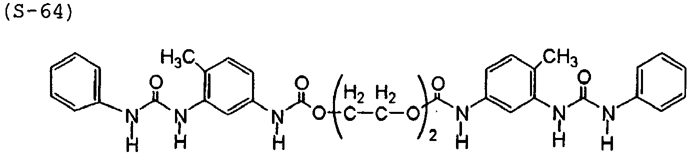

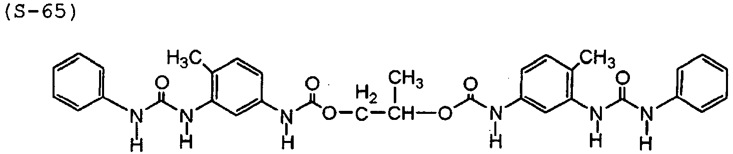

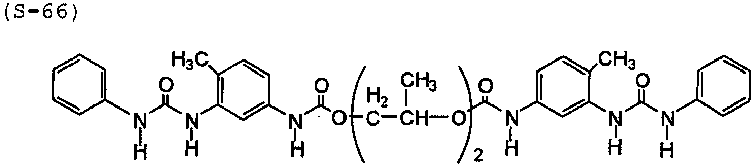

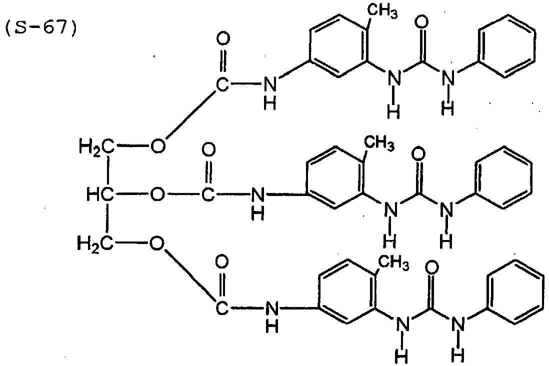

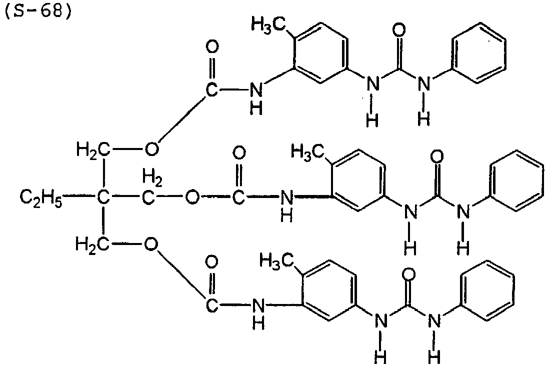

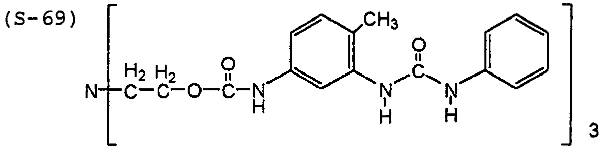

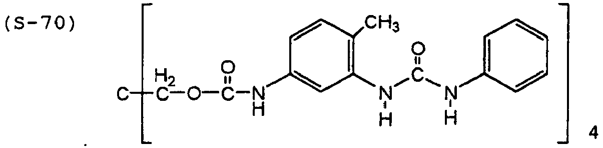

It has been known that compounds having one or more urea groups have color-developing effect, but they have not been practical because they give a low coloring density and have an insufficient shelf stability. However, surprisingly, a urea-urethane compound having at least one urea group and at least one urethane group in the molecule as defined in claim 1 is an excellent developer for a colorless or light-colored dye precursor, and a color-producing composition comprising the urea-urethane compound and the dye precursor and a recording material obtained by using the color-producing composition give a high coloring density and have an excellent shelf stability.

Although a mechanism by which such a urea-urethane compound exhibits an excellent color-developing effect is unknown, it is conjectured that the effect is due to the interaction between the urea group(s) and the urethane group(s) in the molecule.

The novel compounds of the present invention are useful in the case of recording materials which use heat as a recording energy.

The urea-urethane compound used as developer in the present invention is usually a colorless or light-colored compound that is solid at ordinary temperatures.

For producing a recording material by using the urea-urethane compound as developer, the urea-urethane compound of one kind or, if necessary, a combination of the urea-urethane compounds of two or more kinds may be used.

The colorless or light-colored dye precursor used in the present invention is a compound well known as a color former used in heat-sensitive recording materials and is not particularly limited. As the dye precursor, leuco dyes are especially preferable, and triarylmethane type leuco dyes, fluoran type leuco dyes, fluorene type leuco dyes and diphenylmethane type leuco dyes are more preferable. Typical examples of the leuco dyes are given below.

### (1) Triarylmethane type compounds

3,3-bis(p-dimethylaminophenyl)-6-dimethylaminophthalide (Crystal Violet lactone), 3,3-bis(p-dimethylaminophenyl)phthalide, 3-(p-dimethylamino-phenyl)-3-(2,2-dimethylindol-3-yl)phthalide, 3-(p-dimethylaminophenyl)-3-(2-methylindol-3-yl)phthalide, 3-(p-dimethylaminophenyl)-3-(2-phenylindol-3-yl)phthalide, 3,3-bis(1,2-dimethylindol-3-yl)-5-dimethylaminophthalide, 3,3-bis(1,2-dimethylindol-3-yl)-6-dimethylaminophthalide, 3,3-bis(9-ethylcarbazol-3-yl)-5-dimethylaminophthalide, 3,3-bis(2-phenylindol-3-yl)-5-dimethylaminophthalide, 3-p-dimethylamino-phenyl-3-(1-methylpyrrol-2-yl)-6- dimethylaminophthalide.

### (2) Diphenylmethane type compounds

4,4'-bis-dimethylaminophenylbenzhydryl benzyl ether, N-halophenylleucoauramines, N-2,4,5-trichlorophenylleucoauramine.

### (3) Xanthene type compounds

Rhodamine B anilinolactam, Rhodamine B-p-chloroanilinolactam, 3-dimethylamino-6-methyl-7-(m-trifluoromethylanilino)fluoran, 3-diethylamino-6-methyl-fluoran, 3-diethylamino-7-methyl-fluoran, 3-diethylamino-7-chloro-fluoran, 3-diethylamino-7-dibenzylaminofluoran, 3-diethylamino-6-methyl-7-chlorofluoran, 3-diethylamino-7-octylaminofluoran, 3-diethylamino-7-phenylfluoran, 3-diethylamino-6-methyl-7-anilinofluoran, 3-diethylamino-6-methyl-7-p-methylanilinofluoran, 3-diethylamino-6-chloro-7-methylfluoran, 3-diethylamino-7-(3,4-dichloroanilino)-fluoran, 3-diethylamino-7-(2-chloroanilino)fluoran, 3-diethylamino-6-methyl-7-(o,p-dimethylanilino)fluoran, 3-(N-ethyl-N-tolyl)amino-6-methyl-7-phenethylfluoran, 3-diethylamino-7-(4-nitroanilino)fluoran, 3-diethyl-amino-6-methyl-7-(m-trifluoromethylanilino)fluoran, 3-diethylamino-6-methyl-7-(o-chloroanilino)fluoran, 3-diethylamino-6-methyl-7-(p-chloroanilino)fluoran, 3-diethylamino-6-methyl-7-(o-fluoroanilino)fluoran, 3-diethylamino-6-methyl-7-(p-n-butylanilino)fluoran, 3-diethylamino-6-methyl-7-n-octylaminofluoran, 3-diethylamino-6-chloro-7-anilinofluoran, 3-diethylamino-6-ethoxyethyl-7-anilinofluoran, 3-diethylamino-benzo[a]fluoran, 3-diethylamino-benzo[c]fluoran, 3-diethylamino-6-methyl-7-benzylaminofluoran, 3-diethylamino-6-methyl-7-dibenzylaminofluoran, 3-diethylamino-7-di(p-methylbenzyl)aminofluoran, 3-diethylamino-6-methyl-7-diphenylmethylaminofluoran, 3-diethylamino-7-dinaphthylmethylaminofluoran, 10-diethylamino-4-dimethylaminobenzo[a]fluoran, 3-diethylamino-7,8-benzfluoran, 3-diethylamino-6-methyl-7-(m-trichloroanilino)fluoran, 3-diethylamino-7-(o-chloroanilino)fluoran, 3-dibutylamino-7-(o-chloro-anilino)fluoran, 3-diethylamino-6-methyl-7-(2',4'-dimethylanilino)fluoran, 3-(N,N-diethylamino)-5-methyl-7-(N,N-dibenzylamino)fluoran, 3-morpholino-7-(N-propyltrifluoromethylanilino)fluoran, 3-pyrrolidino-7-trifluoromethylanilinofluoran, 3-diethylamino-5-chloro-7-(N-benzyl-trifluoromethylanilino)fluoran, 3-pyrrolidino-7-(di-p-chlorophenyl)methylaminofluoran, 3-diethylamino-5-chloro-7-(α-phenylethylamino)fluoran, 3-(N-ethyl-N-p-toluidino)-7-(α-phenylethylamino)fluoran, 3-diethylamino-7-(o-methoxycarbonylphenylethyl)fluoran, 3-diethylamino-5-methyl-7-(a-phenylethylamino)fluoran, 3-diethylamino-7-piperidinoaminofluoran, 2-chloro-3-(N-methyltoluidino)-7-(p-N-butylanilino)fluoran, 3-(N-ethyl-N-cyclohexylamino)-5,6-benzo-7-α-naphthylamino-4'-bromofluoran, 3-diethylamino-6-methyl-7-mesitidino-4',5'-benzofluoran, 3-dibutylamino-6-methyl-fluoran, 3-dibutylamino-6-methyl-7-chlorofluoran, 3-dibutylamino-6-methyl-7-anilinofluoran, 3-dibutylamino-6-methyl-7-p-methylanilinofluoran, 3-dibutylamino-6-methyl-7-(o,p-dimethylanilino)fluoran, 3-dibutylamino-6-methyl-7-(m-trifluoromethylanilino)fluoran, 3-dibutylamino-6-methyl-7-(o-chloroanilino)fluoran, 3-dibutylamino-6-methyl-7-(p-chloroanilino)fluoran, 3-dibutylamino-6-methyl-7-(o-fluoroanilino)fluoran, 3-dibutylamino-6-methyl-7-(p-n-butylanilino)fluoran, 3-dibutylamino-6-methyl-7-n-octylaminofluoran, 3-dibutylamino-6-chloro-7-anilinofluoran, 3-dibutylamino-6-ethoxyethyl-7-anilinofluoran, 3-di-n-pentylamino-6-methyl-7-anilinofluoran, 3-di-n-pentylamino-6-methyl-7-(o,p-dimethyl-anilino)fluoran, 3-di-n-pentylamino-6-methyl-7-(m-trifluoromethylanilino)fluoran, 3-di-n-pentylamino-6-methyl-7-(o-chloroanilino)fluoran, 3-di-n-pentylamino-6-methyl-7-(p-chloroanilino)fluoran, 3-di-n-pentylamino-6-methyl-7-(o-fluoroanilino)fluoran, 3-pyrrolidino-6-methyl-7-anilinofluoran, 3-piperidino-6-methyl-7-anilinofluoran, 3-cyclohexylamino-6-chlorofluoran, 3-dimethylamino-5,7-dimethylfluoran, 3-(N-methyl-N-isoamylamino)-6-methyl-7-anilinofluoran, 3-(N-methyl-N-n-propylamino)-6-methyl-7-anilinofluoran, 3-(N-methyl-N-amylamino)-6-methyl-7-anilinofluoran, 3-(N,N-di-n-amylamino)-6-methyl-7-anilinofluoran, 3-(N-methyl-N-isopropylamino)-6-methyl-7-anilinofluoran, 3-(N-ethyl-N-n-propylamino)-6-methyl-7-anilinofluoran, 3-(N-ethyl-N-isopropylamino)-6-methyl-7-anilinofluoran, 3-(N-ethyl-N-n-butylamino)-6-methyl-7-anilinofluoran, 3-(N-ethyl-N-isobutylamino)-6-methyl-7-anilinofluoran, 3-(N-ethyl-N-n-hexylamino)-6-methyl-7-p-methylanilinofluoran, 3-(N-ethyl-N-n-hexylamino)-6-methyl-7-(o,p-dimethylanilino)fluoran, 3-(N-ethyl-N-n-hexylamino)-6-methyl-7-(m-trifluoromethylanilino)fluoran, 3-(N-ethyl-N-n-hexylamino)-6-methyl-7-(o-chloroanilino)fluoran, 3-(N-ethyl-N-isoamylamino)-6-methyl-7-anilinofluoran, 3-(N-ethyl-N-isoamylamino)-6-chloro-7-anilinofluoran, 3-(N-ethyl-N-3-methylbutylamino)-6-methyl-7-anilinofluoran, 3-(N-ethyl-N-p-toluidino)-6-methyl-7-anilinofluoran, 3-(N-ethyl-N-p-toluidino)-6-methyl-7-(p-methylanilino)fluoran, 3-(N-ethyl-N-p-toluidino)-6-methyl-'7-(o,p-dimethylanilino)fluoran, 3-(N-ethyl-N-tetrahydrofurfurylamino)-6-methyl-7-anilinofluoran, 3-(N-cyclohexyl-N-methylamino)-6-methyl-7-anilinofluoran, 3-(N-cyclohexyl-N-methylamino)-7-anilinofluoran, 3-(N-ethyl-N-3-methoxypropylamino)-6-methyl-7-anilinofluoran, 3-(N-ethyl-N-3-ethoxypropylamino)-6-methyl-7-anilinofluoran, 2-(4-oxahexyl)-3-dimethylamino-6-methyl-7-anilinofluoran, 2-(4-oxahexyl)-3-diethylamino-6-methyl-7-anilinofluoran, 2-(4-oxahexyl)-3-dipropylamino-6-methyl-7-anilinofluoran, 3,6-bis(diethylamino)-fluoran-y-(2'-nitro)anilinolactam, 3,6-bis(diethylamino)fluoran-γ-(3'-nitro)anilinolactam, 3,6-bis(diethylamino) fluoran-γ-(4'-nitro) anilinolactam, 3,6-bis(diethylamino)fluoran-y-anilinolactam.

### (4) Thiazine type compounds

benzoylleucomethylene blue, p-nitrobenzoylleucomethylene blue.

### (5) Spiro-compounds

3-methylspirodinaphthopyran, 3-ethylspirodinaphthopyran, 3,3-dichlorospirodinaphthopyran, 3-benzylspirodinaphthopyran, 3-methylnaphtho-(3-methoxybenzo)spiropyran, 3-propylspirobenzopyran.

The leuco dyes also include, for example, the following compounds that can absorb a near infrared ray: 3,6-bis(dimethylamino)fluorene-9-spiro-3'-(6'-dimethylamino-phthalide), 3-diethylamino-6-dimethylaminofluorene-9-spiro-3'-(6'-dimethylaminophthalide), 3,6-bis(diethylamino)fluorene-9-spiro-3'-(6'-dimethylaminophthalide), 3-dibutylamino-6-dimethylaminofluorene-9-spiro-3'-(6'-dimethylaminophthalide), 3-dibutylamino-6-diethylaminofluorene-9-spiro-3'-(6'-dimethylaminophthalide), 3,6-bis(dimethylamino)-fluorene-9-spiro-3'-(6'-diethylaminophthalide), 3-diethylamino-6-dimethylaminofluorene-9-spiro-3'-(6'-diethylaminophthalide), 3-dibutylamino-6-dimethylaminofluorene-9-spiro-3'-(6'-diethylaminophthalide), 3,6-bis(diethylamino)fluorene-9-spiro-3'-(6'-diethylaminophthalide), 3,6-bis(dimethylamino)fluorene-9-spiro-3'-(6'-dibutylaminophthalide), 3-dibutylamino-6-diethylaminofluorene-9-spiro-3'-(6'-diethylaminophthalide), 3-diethylamino-6-dimethylaminofluorene-9-spiro-3'-(6'-dibutylaminophthalide), 3,3-bis[2-(4-dimethylaminophenyl)-2-(4-methoxyphenyl)ethenyl]-4,5,6,7-tetrachlorophthalide.

Of the above-exemplified leuco dyes, the triarylmethane type leuco dyes, fluoran type leuco dyes, fluorene type leuco dyes and diphenylmethane type leuco dyes are preferable from the viewpoint of sensitivity and plasticizer resistance, and compounds having a structure represented by the following chemical formula (i) or (j) are more preferable: 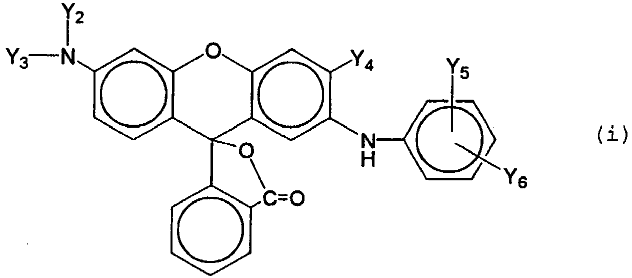 wherein both Y₂ and Y₃ are alkyl groups or alkoxyalkyl groups, Y₄ is a hydrogen atom, an alkyl group or an alkoxy group, and each of Y₅ and Y₆ is a hydrogen atom, a halogen atom, an alkyl group or an alkoxy group; or 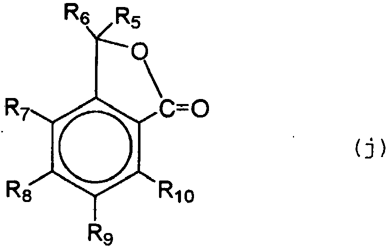 wherein each of R₅ and R₆ is a group represented by the formula (k) or the formula (1): 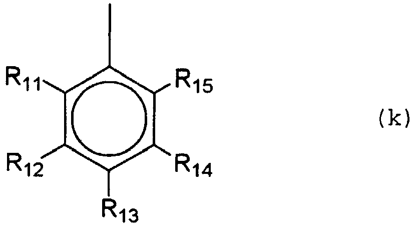 (wherein each of R₁₁ through R₁₅ is a hydrogen atom, a halogen atom, a C₁-C₈ alkyl group, a C₁-C₈ alkoxy group or -NR₁₆R₁₇ wherein each of R₁₆ and R₁₇ is a C₁-C₈ alkyl group); or 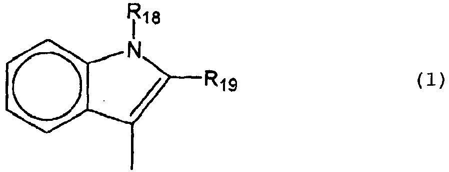 (wherein each of R₁₈ and R₁₉ is a hydrogen atom, a C₁-C₈ alkyl group or a phenyl group), and each of R₇ through R₁₀ is a hydrogen atom, a halogen atom, a C₁-C₈ alkyl group, a C₁-C₈ alkoxy group or -NR₂₀R₂₁ wherein each of R₂₀ and R₂₁ is a C₁-C₈ alkyl group.

Such colorless or light-colored dye precursors may be used in combination of two or more thereof if necessary.

The urea-urethane compound as developer is used in a proportion of preferably 5 to 1,000 parts by weight, more preferably 20 to 500 parts by weight, per 100 parts by weight of the colorless or light-colored dye precursor. As the proportion of the urea-urethane compound as developer, 5 parts by weight or more is sufficient to allow the dye precursor to develop a color. At such a proportion, the coloring density is high. When the proportion of the urea-urethane compound as developer is 1,000 parts by weight or less, the urea-urethane compound as developer hardly remains as a surplus, and this is economically advantageous and hence preferable.

As the urea-urethane compound used as developer in the present invention, any of the urea-urethane compounds of the present invention can be used. Synthesis processes of these compounds and compositions are as already described in detail.

The incorporation of an isocyanate compound into the color-producing composition of the present invention improves the shelf stability of the composition. The isocyanate compound incorporated into the color-producing composition of the present invention refers to a colorless or light-colored, aromatic or heterocyclic isocyanate compound that is solid at ordinary temperatures. For example, one or more of the following isocyanate compounds are used.

The isocyanate compound incorporated includes 2,6-dichlorophenyl isocyanate, p-chlorophenyl isocyanate, 1,3-phenylene diisocyanate, 1,4-phenylene diisocyanate, 1,3-dimethylbenzene-4,6-diisocyanate, 1,4-dimethylbenzene-2,5-diisocyanate, 1-methoxybenzene-2,4-diisocyanate, 1-methoxybenzene-2,5-diisocyanate, 1-ethoxybenzene-2,4-diisocyanate, 2,5-dimethoxybenzene-1,4-diisocyanate, 2,5-diethoxylbenzene-1,4-diisocyanate, 2,5-dibutoxybenzene-1,4-diisocyanate, azobenzene-4,4'-diisocyanate, diphenyl ether-4,4'-diisocyanate, naphthalene-1,4-diisocyanate, naphthalene-1,5-diisocyanate, naphthalene-2,6-diisocyanate, naphthalene-2,7-diisocyanate, 3,3'-dimethyl-biphenyl-4,4'-diisocyanate, 3,3'-dimethoxybiphenyl-4,4'-diisocyanate, diphenylmethane-4,4'-diisocyanate, diphenyldimethylmethane-4,4'-diisocyanate, benzophenone-3,3'-diisocyanate, fluorene-2,7-diisocyanate, anthraquinone-2,6-diisocyanate, 9-ethylcarbazole-3,6-diisocyanate, pyrene-3,8-diisocyanate, naphthalene-1,3,7-triisocyanate, biphenyl-2,4,4'-triisocyanate, 4,4',4"-triisocyanato-2,5-dimethoxytriphenylamine, 4,4',4"-triisocyanato-triphenylamine, p-dimethylaminophenyl isocyanate, tris(4-phenylisocyanato)thiophosphate. If necessary, these isocyanates may be used in the form of a so-called block isocyanate, i.e., an addition compound with a phenol, lactam or oxime, they may be used in the form of a diisocyanate dimer such as 1-methylbenzene-2,4-diisocyanate dimer, or a diiso-cyanurate trimer as an isocyanurate, and they may be used in the form of a polyisocyanate obtained as an adduct by the use of any of various polyols and the like. There may also be used water adduct isocyanates of 2,4-toluene diisocyanate, diphenylmethane diisocyanate and the like, such as 1,3-bis(3-isocyanato-4-methylphenyl)urea; polyol adducts such as trimethylolpropane adduct of toluene diisocyanate (Desmodule L, a trade name); phenol adduct isocyanates; amine adduct isocyanates; and the isocyanate compounds and isocyanate adduct compounds described in the specification of JP-A-10-76757 and the specification of JP-A-10-95171.

The isocyanate compound is used in a proportion of preferably 5 to 500 parts by weight, more preferably 20 to 200 parts by weight, per 100 parts by weight of the colorless or light-colored dye precursor. When the proportion of the isocyanate compound is 5 parts by weight or more, a sufficient improving effect on the shelf stability can be obtained and the coloring density is high. When the proportion of the isocyanate compound is 500 parts by weight or less, the isocyanate compound hardly remains as a surplus, and this is economically advantageous and hence preferable.

The incorporation of an imino compound into the color-producing composition of the present invention further improves the shelf stability.

The imino compound that can be incorporated into the color-producing composition of the present invention is a colorless or light-colored compound that has at least one imino group and is solid at ordinary temperatures. Two or more imino compounds may be incorporated in combination, depending on purposes. As the imino compound, those described in JP-A-9-142032 can be mentioned. Of the imino compounds described in the reference, imino-isoindoline derivatives are preferable, and 1,3-diimino-4,5,6,7-tetrachloroisoindoline, 3-imino-4,5,6,7-tetrachloroisoindolin-1-one and 1,3-diimino-4,5,6,7-tetrabromoisoindoline are more preferable.

The imino compound is used in a proportion of preferably 5 to 500 parts by weight, more preferably 20 to 200 parts by weight, per 100 parts by weight of the colorless or light-colored dye precursor. When the proportion of the imino compound is 5 parts by weight or more, an improving effect on the shelf stability is obtained. When the proportion of the imino compound is 500 parts by weight or less, the imino compound hardly remains as a surplus, and this is economically advantageous and hence preferable.

In addition, the incorporation of an amino compound into the color-producing composition of the present invention improves the preservability of an original recording material surface and print. The amino compound that can be incorporated is a colorless or light-colored substance having at least one primary, secondary or tertiary amino group. As such an amino compound, those described in JP-A-9-142032 can be mentioned. Of the amino compounds described in this reference, aniline derivatives having at least one amino group and represented by the following formula (VIII) are especially preferable: 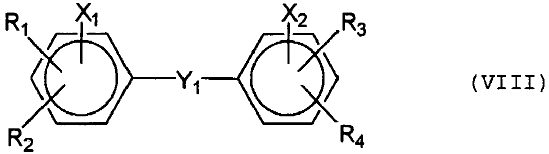 wherein R₁, R₂, R₃ and R₄ are independently a hydrogen atom, a halogen atom, an alkyl group, an alkoxy group or an amino group, X₁ and X₂ are independently an amino group or a group represented by the formula (b): 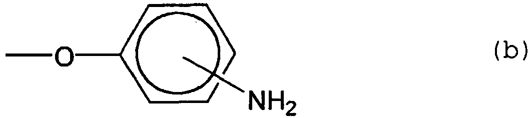 and Y₁ is any of -SO₂-, -O-, -(S)ₙ-, -(CH₂)ₙ-, -CO-, -CONH- and a group represented by any of the formulas (a) : 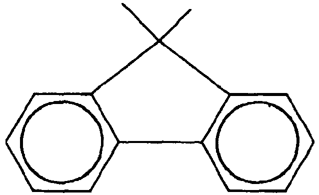 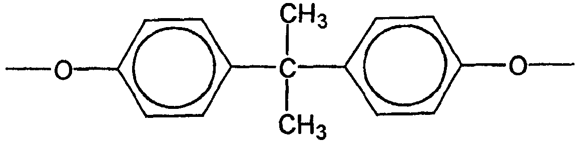 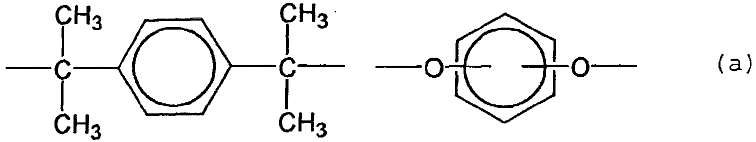 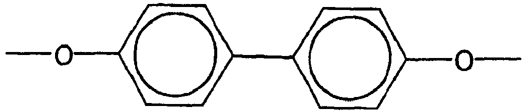 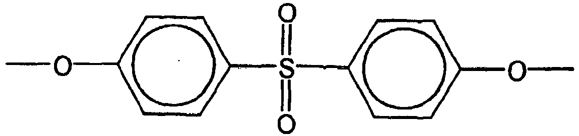 or is absent, and n is 1 or 2.

These amino compounds may be used singly or as a mixture thereof. For improving the print preservability in the plasticizer resistance, the proportion of the amino compound is preferably 1 to 500 parts by weight per 100 parts by weight of the colorless or light-colored dye precursor. When the content of the amino compound is 1 part by weight or more per part of the urea-urethane compound, the print preservability can be improved. When the content is 500 parts by weight or less, performance characteristics of the resulting composition can be sufficiently improved and such a content is advantageous from the viewpoint of cost.

The incorporation of also an acidic developer into the color-producing composition of the present invention improves the sensitivity and enables the color-producing composition to produce a brilliant color.

As the acidic developer that is used when the color-producing composition of the present invention is used in a heat-sensitive recording material, conventional electron-accepting materials are used and, in particular, phenol derivatives; aromatic carboxylic acid derivatives or their metal compounds; salicylic acid derivatives or their metal salts; N,N-diaryl-thiourea derivatives; sulfonylurea derivatives are preferable. The phenol derivatives are especially preferable. Specific examples of the phenol derivatives are 2,2-bis(4-hydroxyphenyl)propane, 2,2-bis(hydroxyphenyl)butane, 2,2-bis(hydroxyphenyl)-pentane, 2,2-bis(hydroxyphenyl)heptane, 1,1-bis(4-hydroxyphenyl)cyclohexane, butyl bis(4-hydroxyphenyl)-acetate, benzyl bis(4-hydroxyphenyl)acetate, bis(4-hydroxyphenyl) sulfone, bis(3-methyl-4-hydroxyphenyl) sulfone, 4-hydroxyphenyl-4'-methylphenyl sulfone, 3-chloro-4-hydroxyphenyl-4'-methylphenyl sulfone, 3,4-dihydroxyphenyl-4'-methylphenyl sulfone, 4-isopropyl-phenyl-4'-hydroxyphenyl sulfone, 4-isopropyloxyphenyl-4'-hydroxyphenyl sulfone, bis(2-allyl-4-hydroxyphenyl) sulfone, 4-hydroxyphenyl-4'-benzyloxyphenyl sulfone, 4-isopropylphenyl-4'-hydroxyphenyl sulfone, bis(2-methyl-3-tert-butyl-4-hydroxyphenyl) sulfide, methyl 4-hydroxybenzoate, benzyl 4-hydroxybenzoate, (4'-chlorobenzyl) 4-hydroxybenzoate, ethyl 1,2-bis(4'-hydroxybenzoate), pentyl 1,5-bis(4'-hydroxybenzoate), hexyl 1,6-bis(4'-hydroxybenzoate), dimethyl 3-hydroxy-phthalate, stearyl gallate and lauryl gallate. The salicylic acid derivatives include 4-n-octyloxysalicylic acid, 4-n-butyloxysalicylic acid, 4-n-pentyloxysalicylic acid, 3-n-dodecyloxysalicylic acid, 3-n-octanoyloxysalicylic acid, 4-n-octyloxycarbonylaminosalicylic acid and 4-n-octanoyloxycarbonylaminosalicylic acid. The sulfonylurea derivatives include, for example, compounds containing one or more arylsulfonylaminoureido groups, such as 4,4-bis(p-toluenesulfonylaminocarbonylamino)diphenylmethane, 4,4-bis(o-toluenesulfonylaminocarbonylamino)diphenylmethane, 4,4-bis(p-toluenesulfonylaminocarbonylamino)-diphenyl sulfide, 4,4-bis(p-toluenesulfonylaminocarbonylamino)diphenyl ether, N-(p-toluenesulfonyl)-N'-phenylurea. In addition, there may also be used, for example, 4,4'-[oxybis(ethyleneoxy-p-phenylenesulfonyl)]diphenol and mixtures composed mainly of this compound (e.g. D-90 (a trade name, mfd. by Nippon Soda Co., Ltd.)).

Of the above-exemplified acidic developers, 2,2-bis(4-hydroxyphenyl)propane, 4-isopropyloxyphenyl-4'-hydroxyphenyl sulfone, bis(3-allyl-4-hjrdroxyphenyl) sulfone, 2,9'-dihydroxydiphenyl sulfone and 4,4'-[oxybis(ethyleneoxy-p-phenylenesulfonyl)]diphenol are especially preferable because they improve sensitivity and make it possible to obtain a heat-sensitive recording material capable of producing a brilliant color.

In order to improve fog and the thermal response, it is also possible to add phenolic compounds such as N-stearyl-N'-(2-hydroxyphenyl)urea, N-stearyl-N'-(3-hydroxyphenyl)urea, N-stearyl-N'-(4-hydroxyphenyl)urea, p-stearoylaminophenol, o-stearoylaminophenol, p-lauroylaminophenol, p-butyrylaminophenol, m-acetylaminophenol, o-acetylaminophenol, p-acetylaminophenol, o-butylaminocarbonylphenol, o-stearylaminocarbonylphenol, p-stearylaminocarbonylphenol, 1,1,3-tris(3-tert-butyl-4-hydroxy-6-methylphenyl)butane, 1,1,3-tris(3-tert-butyl-4-hydroxy-6-ethylphenyl)butane, 1,1,3-tris(3,5-di-tert-butyl-4-hydroxyphenyl)butane, 1,1,3-tris(3-tert-butyl-4-hydroxy-6-methylphenyl)propane, 1,2,3-tris(3-tert-butyl-4-hydroxy-6-methylphenyl)butane, 1,1,3-tris(3-phenyl-4-hydroxyphenyl)butane, 1,1,3-tris(3-cyclohexyl4-hydroxy-5-methylphenyl)butane, 1,1,3-tris(3-cyclohexyl-4-hydroxy-6-methylphenyl)butane, 1,1,3-tetra(3-phenyl-4-hydroxyphenyl)propane, 1,1,3,3-tetra(3-cyclohexyl-4-hydroxy-6-methylphenyl)propane, 1,1-bis(3-tert-butyl-4-hydroxy-6-methylphenyl)butane and 1,1-bis(3-cyclohexyl-4-hydroxy-6-methylphenyl)butane.

The above-mentioned acidic developer is used in a proportion of preferably 5 to 500 parts by weight, more preferably 20 to 200 parts by weight, per 100 parts by weight of the colorless or light-colored dye precursor. When the proportion of the acidic developer is 5 parts by weight or more, the color development of the dye precursor is satisfactory and the coloring density is high.

When the proportion of the acidic developer is 500 parts by weight or less, the acidic developer hardly remains, and this is economically advantageous and hence preferable.

Also as this acidic developer, an electron-accepting material is used. The acidic developer includes, for example, inorganic compounds such as acid clay, activated clay, attapulgite, bentonite, zeolite, colloidal silica, magnesium silicate, talc, aluminum silicate; phenol, cresol, butylphenol, octylphenol, phenylphenol, chlorophenol, salicylic acid, or aldehyde condensation novolak resins derived therefrom and their metal salts; and salicylic acid derivatives such as 3-isopropylsalicylic acid, 3-phenylsalicylic acid, 3-cyclohexylsalicylic acid, 3,5-di-t-butylsalicylic acid, 3,5-di(α-methylbenzyl)-salicylic acid, 3,5-di-t-octylsalicylic acid, 3-methyl-5-benzylsalicylic acid, 3,5-di(α,α-dimethylbenzyl)-salicylic acid, 3-phenyl-5-(α,α-dimethylbenzyl)-salicylic, and metal salts thereof.

The incorporation of also a fluorescent dye into the color-producing composition of the present invention improves the whiteness. As the fluorescent dye to be incorporated into the color-producing composition of the present invention, various well-known ones can be used, and there are mentioned stilbene derivatives, coumarin derivatives, pyrazoline derivatives, bisstyrylbiphenyl derivatives, naphthalimide derivatives and bisbenzoxazolyl derivatives. Although the fluorescent dye is not limited to them, diaminostilbenedisulfonic acid derivatives are especially preferable.

As to the amount of the fluorescent dye used, the fluorescent dye is made present in an amount of preferably 0.01 to 3 wt%, more preferably 0.1 to 2 wt%, based on the total weight (in terms of solids) of the color-producing composition. When the amount of the fluorescent dye used is more than 3 wt%, the color-producing composition is colored in some cases. When the amount is less than 0.01 wt%, the effect of the fluorescent dye on the whiteness is lessened.

Next, the color-producing composition of the present invention may contain shelf-stability-imparting agents. The shelf-stability-imparting agents usable in the present invention are additives such as image-stabilizing agents, light stabilizers, antioxidants.

By using these shelf-stability-imparting agents in combination with a urea-urethane compound developer (i.e. a developer comprising a urea-urethane compound) and a colorless and light-colored dye precursor, the light resistance of the color-producing composition can be improved and a recording material excellent in light resistance can be obtained.

The image-stabilizing agents as preferable examples of the shelf-stability-imparting agents used in the present invention include, for example, 1,1,3-tris(2-methyl-4-hydroxy-5-tert-butylphenyl)butane, 1,1,3-tris(2-methyl-4-hydroxy-5-cyclohexylphenyl)-butane, hindered phenol compounds [e.g. 4,4'-butylidenebis(2-tert-butyl-5-methylphenol), 4,4'-thiobis(2-tert-butyl-5-methylphenol), 2,2'-thiobis(6-tert-butyl-4-methylphenol) and 2,2'-methylenebis(6-tert-butyl-4-methylphenol)], 4-benzyloxy-4'-(2-methylglycidyloxy)diphenyl sulfone, 4,4'-diglycidyloxy-diphenyl sulfone, 1,4-diglycidyloxybenzene, sodium 2,2'-methylenebis(4,6-di-tert-butylphenyl)phosphate, 2-propanol derivatives and salicylic acid derivatives. Usually, these image-stabilizing agents are used in a proportion of preferably 5 to 1,000 parts by weight, more preferably 10 to 500 parts by weight, per 100 parts by weight of the colorless or light-colored dye precursor. When the proportion of the image-stabilizing agents is 5 parts by weight or more, the light resistance is good and the coloring density is high. When the proportion of the image-stabilizing agents is more than 1,000 parts by weight, their effect on the light resistance is not heightened and moreover, such a proportion is economically disadvantageous.

The light stabilizers as preferable examples of the shelf-stability-imparting agents used in the present invention include, for example, 2-(2'-hydroxy-5'-methylphenyl)benzotriazole, 2-(3',5'-di-t-butyl-2'-hydroxyphenyl)benzotriazole, 2-(5'-t-butyl-2'-hydroxyphenyl)benzotriazole, 2-[2'-hydroxy-5'-(1,1,3,3-tetramethylbutyl)phenyl]benzotriazole, 2-(3',5'-di-t-butyl-2'-hydroxyphenyl)-5-chlorobenzotriazole, 2-(3'-t-butyl-2'-hydroxy-5'-methylphenyl)-5-chlorobenzo-triazole, 2-(2'-hydroxy-4'-octoxyphenyl)benzotriazole, 2-(3',5'-di-t-pentyl-2'-hydroxyphenyl)benzotriazole, 2-(3'-t-butyl-2'-hydroxy-5'-octyloxycarbonylethylphenyl)-5-chlorobenzotriazole; 4-hydroxy-, 4-methoxy-, 4-octoxy-, 4-decyloxy-, 4-dodecyloxy-, 4-benzyloxy-, 4,2',4'-trihydroxy-, 2'-hydroxy-4,4'-dimethoxy- or 4-(2-ethylhexyloxy)-2-hydroxybenzophenone derivatives; 4-t-butylphenyl salicylate, phenyl salicylate, octylphenyl salicylate, dibenzoylresorcinol, bis(4-t-butylbenzoyl)resorcinol, 2,4-di-t-butylphenyl 3,5-di-t-butyl-4-hydroxybenzoate, hexadecyl 3,5-di-t-butyl-4-hydroxybenzoate; ethyl α-cyano-β,β-diphenylacrylate, isooctyl α-cyano-β,β-diphenylacrylate, methyl α-carbomethoxy-cinnamate, methyl α-cyano-β-methyl-p-methoxycinnamate and the like; bis(2,2,6,6-tetramethyl-4-piperidyl) sebacate, bis(2,2,6,6-tetramethyl-4-piperidyl) succinate, bis(1,2,2,6,6-pentamethyl-4-piperidyl) sebacate, bis(1-octyloxy-2,2,6,6-tetramethyl-4-piperidyl) sebacate, bis(1,2,2,6,6-pentamethyl-4-piperidyl) adipate; 4,4'-di-octyloxy-oxanilide, 2,2'-diethoxyoxyoxanilide, 2,2'-di-octyloxy-5,5'-di-t-butyloxanilide, 2,2'-di-dodecyloxy-5,5'-di-t-butyloxanilide, 2-ethoxy-2'-ethyloxanilide, N,N'-bis(3-dimethylaminopropyl)oxanilide, 2-ethoxy-5-t-butyl-2'-ethoxyoxanilide; and 2,4,6-tris(2-hydroxy-4-octyloxyphenyl)-1,3,5-triazine, 2-(2-hydroxy-4-octyloxyphenyl)-4,6-bis(2,4-dimethylphenyl)-1,3,5-triazine, 2-(2,4-dihydroxyphenyl)-4,6-bis(2,4-dimethylphenyl)-1,3,5-triazine, 2,4-bis(2-hydroxy-4-propyloxyphenyl-6-(2,4-dimethylphenyl)-1,3,5-triazine, 2-(2-hydroxy-4-dodecyloxyphenyl)-4,6-bis(2,4-dimethylphenyl)-1,3,5-triazine. Usually, these light stabilizers are used in a proportion of preferably 5 to 1,000 parts by weight, more preferably 10 to 500 parts by weight, per 100 parts by weight of the colorless or light-colored dye precursor. When the proportion of the light stabilizers is 5 parts by weight or more, the light resistance is good and the coloring density is high. When the proportion of the light stabilizers is more than 1,000 parts by weight, their effect on the light resistance is not heightened and moreover, such a proportion is economically disadvantageous.

The antioxidants as preferable examples of the shelf-stability-imparting agents used in the present invention include, for example, 2,6-di-t-butyl-4-methylphenol, 2-t-4,6-dimethylphenol, 2,6-di-t-butyl-4-ethylphenol, 2,6-di-t-butyl-4-n-butylphenol, 2,6-di-t-butyl-4-isobutylphenol, 2,6-dicyclopentyl-4-methylphenol, 2-(α-methylcyclohexyl)-4,6-dimethylphenol, 2,6-dioctadecyl-4-methylphenol, 2,4,6-tricyclohexylphenol, 2,6-dinonyl-4-methylphenol, 2,6-di-t-butyl-4-methoxy-methylphenol, 2,4-dimethyl-6-(1'-methyl-undeca-1'-yl)-phenol,, 2,4-dimethyl-6-(1'-methyl-heptadeca-1'-yl)-phenol, 2,4-dimethyl-6-(1'-methyl-trideca-1'-yl)-phenol, and mixtures thereof; 2,4-di-octylthiomethyl-6-t-butylphenol, 2,4-di-octylthiomethyl-6-methylphenol, 2,4-di-octylthiomethyl-6-ethylphenol, 2,6-di-dodecyl-thiomethyl-4-nonylphenol, and mixtures thereof; 2,6-dit-butyl-4-methoxyphenol, 2,5-di-t-butylhydroquinone, 2,5-di-t-amylhydroquinone, 2,6-diphenyl-4-octadecyloxyphenol, 2,6-di-t-butylhydroquinone, 2,5-di-t-butyl-4-hydroxyanisole, 3,5-di-t-butyl-4-hydroxyanisole, 3,5-di-t-butyl-4-hydroxyphenyl stearate, bis(3,5-di-t-butyl-4-hydroxyphenyl) adipate, and mixtures thereof; 2,4-bis-octylmercapto-6-(3,5-di-t-butyl-4-hydroxy-anilino)-1,3,5-triazine, 2-octylmercapto-4,6-bis(3,5-di-t-butyl-4-hydroxyanilino)-1,3,5-triazine, 2-octylmercapto-4,6-bis(3,5-di-t-butyl-4-hydroxyphenoxy)-1,2,3-triazine, 1,3,5-tris(3,5-di-t-butyl-4-hydroxy-benzyl)-isocyanurate, 1,3,5-tris(4-t-butyl-3-hydroxy-2,6-dimethylbenzyl)isocyanurate, 2,4,6-tris(3,5-di-t-butyl-4-hydroxyphenylethyl)-1,3,5-triazine, 1,3,5-tris(3,5-di-t-butyl-4-hydroxyphenylpropionyl)-hexahydro-1,3,5-triazine, 1,3,5-tris(3,5-dicyclohexyl-4-hydroxybenzyl)-isocyanurate; 2,2'-methylenebis(6-t-butyl-4-methylphenol), 2,2'-methylene-bis(6-t-butyl-4-ethylphenol), 2,2'-ethylidenebis(4,6-di-t-butylphenol), 2,2'-ethylidenebis(6-t-butyl-4-isobutylphenol), 4,4'-methylenebis(2,6-di-t-butyl-phenol), 4,4'-methylenebis(6-t-butyl-2-methylphenol), 1,1-bis(5-t-butyl-4-hydroxy-2-methylphenyl)butane, ethylene glycol bis[3,3'-bis(3'-t-butyl-4'-hydroxy-phenyl) butyrate]; 1,3,5-tris(3,5-di-t-butyl-4-hydroxybenzyl)-2,4,6-trimethylbenzene, 1,4-bis(3,5-di-t-butyl-4-hydroxybenzyl)-2,3,5,6-tetramethylbenzene, 2,4,6-tris(3,5-di-t-butyl-4-hydroxybenzyl)-phenol. Usually, these antioxidants are used in a proportion of preferably 5 to 1,000 parts by weight, more preferably 10 to 500 parts by weight, per 100 parts by weight of the colorless or light-colored dye precursor. When the proportion of the antioxidants is 5 parts by weight or more, the light resistance is good and the coloring density is high. When the proportion of the antioxidants is more than 1,000 parts by weight, their effect on the light resistance is not heightened and moreover, such a proportion is economically disadvantageous.

The color-producing composition of the present invention can be made into a heat-sensitive recording material by forming a color-producing layer of the composition on some substrate by a method such as coating. The structure of the recording material is varied depending on the kind of the recording material.

When the color-producing composition is used in a heat-sensitive recording material, a heat-sensitive recording layer capable of producing a color on heating is formed on a substrate. Specifically, the above-mentioned urea-urethane compound, the above-mentioned colorless or light-colored dye precursor such as a leuco dye, and the heat-meltable material described hereinafter should be applied on a substrate, each in the form of a dispersion together with other necessary components to form a heat-sensitive recording layer. The dispersion is prepared by finely grinding one or more compounds as each of the components described above, with a sand grinder or the like in an aqueous solution containing a compound having dispersing capability, such as a water-soluble polymer or a surfactant. The particle size of each of the dispersions thus obtained is preferably adjusted to 0.1 to 10 µm, in particular, to about 1 µm. Specific examples of the compound having dispersing capability which can be used in the present invention are water-soluble polymers such as poly(vinyl alcohol)s, carboxylic acid-modified poly(vinyl alcohol)s, sulfonic acid-modified poly(vinyl alcohol)s, methyl cellulose, hydroxypropylmethyl cellulose, hydroxypropyl cellulose; anionic surfactants such as condensed naphthalenesulfonates, polyoxyethylene alkyl ether sulfuric acid ester salts (e.g. sodium polyoxyethylene lauryl ether sulfates, sodium polyoxyethylene alkyl ether sulfates and sodium polyoxyethylene alkyl phenyl ether sulfates), dialkylsulfo-succinic acid ester sodium, alkylphosphates (e.g. diethanolamine alkylphosphates and potassium alkylphosphates), specialty carboxylic acid-based polymers; nonionic surfactants such as polyoxyethylene alkyl ethers, polyoxyethylene alky phenyl ethers, polyoxyethylene sorbitan fatty acid esters, fatty acid monoglycerides, polyethylene glycol fatty acid esters; and cationic surfactants such as dicyanamidopolyamines, tertiary amine salts, quaternary ammonium salts. Of these, the polyvinyl alcohols, carboxylic acid-modified polyvinyl alcohols, sulfonic acid-modified polyvinyl alcohols and methyl cellulose are especially preferable. The above-exemplified compounds may be used singly or as a mixture thereof.

When the urea-urethane compound developer according to the present invention is used in a heat-sensitive recording material, the adjustment of the average particle size of the urea-urethane compound developer to not more than 5 µm and not less than 0.05 µm makes it possible to obtain a heat-sensitive recording material which has a sufficient color development sensitivity, gives a very stable printed developed color image, and has a good plasticizer resistance. The average particle size is more preferably not more than 3 µm and not less than 0.1 µm. When the average particle size is less than 0.05 µm, the preservability of the original recording material surface against plasticizers is deteriorated. On the other hand, when the average particle size is more than 5 µm, the sensitivity of the heat-sensitive recording material is decreased.

Particularly when the urea-urethane compound is subjected to wet grinding in an aqueous medium, the temperature of the aqueous medium is preferably 60°C or lower. At the time of the grinding, the urea-urethane compound developer comes into contact with water, so that hydrolysis of its urethane group(s) proceeds depending on conditions. Therefore, the sensitivity of a heat-sensitive recording material obtained by the use of the urea-urethane compound developer tends to be decreased. Particularly when the medium temperature at the grinding is higher than 60°C, the sensitivity is remarkably decreased. The medium temperature at the grinding is more preferably 40°C or lower.

In addition, when the urea-urethane compound developer is ground, it is preferably ground in a neutral pH range of 5 to 10. When the pH at the grinding is lower than 5, an inorganic pigment and the like are decomposed in the production of a heat-sensitive coating liquid, so that the sensitivity tends to be decreased. On the other hand, when the pH is higher than 10, the urea-urethane compound developer is hydrolyzed, so that the sensitivity is decreased in some cases. Specific examples of dispersing agent usable for preparing a dispersion of the urea-urethane compound developer by grinding in the present invention are water-soluble polymers such as poly(vinyl alcohol)s, carboxylic acid-modified poly(vinyl alcohol)s, sulfonic acid-modified poly(vinyl alcohol)s, methyl cellulose, hydroxypropylmethyl cellulose, hydroxypropyl cellulose; anionic surfactants such as condensed naphthalenesulfonates, polyoxyethylene alkyl ether sulfuric acid ester salts (e.g. sodium polyoxyethylene lauryl ether sulfates, sodium polyoxyethylene alkyl ether sulfates and sodium polyoxyethylene alkyl phenyl ether sulfates), dialkylsulfo-succinic acid ester sodium, alkylphosphates (e.g. diethanolamine alkylphosphates and potassium alkylphosphates), specialty carboxylic acid-based polymers; nonionic surfactants such as polyoxyethylene alkyl phenyl ethers, polyoxyethylene sorbitan fatty acid esters, fatty acid monoglycerides, polyethylene glycol fatty acid esters; and cationic surfactants such as dicyanamidopolyamines, tertiary amine salts, quaternary ammonium salts. Of these, the water-soluble polymers and the anionic surfactants are especially preferable because they make it possible to obtain a heat-sensitive recording material that has a high sensitivity irrespective of conditions of dispersing the urea-urethane compound developer and has an improved preservability of the original recording material surface against plasticizers irrespective of the average particle size of the urea-urethane compound developer. The poly(vinyl alcohol)s, modified poly(vinyl alcohol)s, methyl cellulose, hydroxypropylmethyl cellulose, condensed sodium naphthalenesulfonate, polycarboxylic acid ammonium salts, water-soluble low-molecular weight copolymers and sodium 2-ethylhexylsulfosuccinate are more preferable. Of these, methyl cellulose, hydroxypropylmethyl cellulose, condensed sodium naphthalenesulfonate, and water-soluble low-molecular weight copolymers are still more preferable, and hydroxypropylmethyl cellulose is the most preferable. The above-exemplified dispersing agents may be used singly or as a mixture thereof.

As a dispersing agent usable for preparing a dispersion of the colorless or light-colored dye precursor by grinding in the present invention, the same compounds as those used as the dispersing agent for dispersing the urea-urethane compound developer can be used. Of such compounds, the water-soluble polymers, the anionic surfactants and mixed dispersing agents of these two kinds of the compounds are especially preferable for improving a heat-sensitive recording material in sensitivity and in preservability of the original recording material surface against plasticizers. Mixed dispersing agents consisting of methyl cellulose or hydroxypropylmethyl cellulose as a water-soluble polymer and a polyoxyethylene alkyl ether sulfate or sodium 2-ethylhexylsulfosuccinate as an anionic surfactant are more preferable. A mixed dispersing agent of hydroxypropylmethyl cellulose and sodium 2-ethylhexylsulfosuccinate is the most preferable.

The pH of a coating liquid containing the urea-urethane compound and the colorless or light-colored dye precursor is preferably 5 to 12.

The heat-sensitive recording layer may contain, besides the components described above, pigments such as diatomaceous dearth, talc, kaolin, calcined kaolin, calcium carbonate, magnesium carbonate, titanium oxide, zinc oxide, silicon oxide, aluminum hydroxide, urea-formaldehyde resin. In addition, the heat-sensitive recording layer may, if necessary, contain metal salts of higher fatty acids, such as zinc stearate, calcium stearate; and waxes such as paraffin, oxidized paraffin, polyethylenes, oxidized polyethylenes, stearamide, cator wax, for the purpose of, for example, preventing the wear of a head and sticking. If necessary, the heat-sensitive recording layer may also contain dispersing agents such as sodium dioctylsulfosuccinate; ultraviolet absorbers of benzophenone type, benzotriazole type; surfactants; fluorescent dyes.

As a binder usable for forming the heat-sensitive recording layer, there can be mentioned, for example, water-soluble binders such as starches, hydroxyethyl cellulose, methyl cellulose, carboxymethyl cellulose, gelatin, casein, poly(vinyl alcohol)s, modified poly(vinyl alcohol)s, sodium poly(acrylate)s, acrylamide-acrylic ester copolymers, acrylamide-acrylic ester-methacrylic acid terpolymers, alkali salts of styrene-maleic anhydride copolymers, alkali salts of ethylene-maleic anhydride copolymers; and latex type water-insoluble binders of styrene-butadiene copolymers, acrylonitrile-butadiene copolymers, methyl acrylate-butadiene copolymers.

As the substrate for the heat-sensitive recording layer, paper is mainly used, though any of various woven fabrics, nonwoven fabrics, synthetic resin films, laminated papers, synthetic papers, metal foils, and composite sheets obtained by combining two or more of them may be used besides paper, depending on their purpose. The basis weight of the substrate is preferably 40 g/m² to 200 g/m². The substrate is preferably excellent in surface smoothness and flatness because a heat-sensitive recording material obtained by the use of the substrate is desired to have as high a flatness as possible. Therefore, the substrate is preferably subjected to surface treatment by applying heat and pressure by means of any of a machine calender, soft calender and supercalender.

The surface pH of the substrate is preferably 3 to 9, more preferably 5 to 9, most preferably 6 to 8. When the surface pH of the substrate is lower than 3, fog tends to occur. When the surface pH of the substrate is higher than 12, the urea-urethane compound developer is decomposed, so that the coloring density is decreased in some cases.

The heat-sensitive recording layer may be composed of either a single layer or two or more layers. The heat-sensitive recording layer may have, for example, a multilayer structure formed by incorporating each color-producing component into one layer. A protective layer composed of a single layer or two or more layers may be formed on the heat-sensitive recording layer, and an intermediate layer composed of a single layer or two or more layers may also be formed between the substrate and the heat-sensitive recording layer. The heat-sensitive recording layer can be obtained by mixing aqueous dispersions prepared by fine grinding of each color-producing component or any other component, with a binder, applying the resulting mixture on the substrate, and drying the mixture. The coating amount of this coating liquid is preferably 1 to 15 g/m² when the coating liquid is in a dried state.

When the color-producing composition of the present invention is used in a heat-sensitive recording material, a heat-meltable material may be incorporated into the color-producing composition in order to improve the sensitivity. The heat-meltable material is preferably one which has a melting point of 60°C to 180°C, in particular, one which has a melting point of 80°C to 140°C. The heat-meltable material includes, for example, benzyl p-benzyloxybenzoate, stearamide or its emulsified product, palmitamide, N-methylol-stearamide, β-naphthyl benzyl ether, N-stearylurea, N,N'-distearylurea, phenyl β-naphthoate, phenyl 1-hydroxy-2-naphthoate, β-naphthol (p-methylbenzyl) ether, 1,4-dimethoxynaphthalene, 1-methoxy-4-benzyloxynaphthalene, N-stearoylurea, p-benzylbiphenyl, 1,2-di(m-methylphenoxy)ethane, 1-phenoxy-2-(4-chloro-phenoxy)ethane, 1,4-butanediol phenyl ether, dimethyl terephthalate, m-terphenyl, dibenzyl oxalate and (p-chlorobenzyl) oxalate.

In addition, 4,4'-dimethoxybenzophenone, 4,4'-dichlorobenzophenone, 4,4'-difluorobenzophenone, diphenyl sulfone, 4,4'-dichlorodiphenyl sulfone, 4,4'-difluorodiphenyl sulfone, 4,4'-dichlorodiphenyl disulfide, diphenylamine, 2-methyl-4-methoxydiphenylamine, N,N'-diphenyl-p-phenylenediamine, 1-(N-phenylamino) naphthalene, benzil and 1,3-diphenyl-1,3-propanedione are preferable as the heat-meltable material because they are very effective in improving the sensitivity.

As the heat-meltable material, there may also be used benzyl 4-hydroxybenzoate, 4-(benzyloxy)phenol, 2,4-dihydroxybenzophenone, 2,2',4,4'-tetrahydroxybenzophenone, 4,4'-dihydroxybenzophenone, 2,2-bis(4-hydroxy-phenyl)propane, 4,4'-dihydroxydiphenyl sulfone, bis(3-methyl-4-hydroxyphenyl) sulfone, bis(3,5-dimethyl-4-hydroxyphenyl) sulfone, 3,4-dihydroxyphenyl-4'-methylphenyl sulfone, bis(2-methyl-3-tert-butyl-4-hydroxyphenyl) sulfide, 4,4'-dihydroxydiphenyl ether, 4,4'-thiodiphenol, 4,4'-dihydroxydiphenylmethane, 3,3'-dihydroxydiphenylamine, bis(4-hydroxy-3-methylphenyl) sulfide, 4-hydroxy-4'-isopropoxydiphenyl sulfone, 4,4'-thiobisbenzenethiol, salicylanilide, 4,4'-diamino-3,3'-diethyldiphenylmethane, 4,4'-diaminobenzanilide, 3,3'-dichloro-4,4'-diaminodiphenylmethane, 3,3'-dimethyl-4,4'-diaminodiphenylmethane, 4,4'-thiodianiline, 2,2'-dithiodianiline, 4,4'-dithiodianiline, 4,4'-diamino-diphenyl ether, 3,3'-diaminodiphenyl ether, 3,4'-diaminodiphenyl ether, 4,4'-diaminodiphenylmethane, 3,4'-diaminodiphenylmethane, bis(3-amino-4-chlorophenyl) sulfone, bis(3,4-diaminophenyl) sulfone, 4,4'-diaminodiphenyl sulfone, 3,3'-diaminodiphenyl sulfone, 3,4'-diaminodiphenyl sulfone, 3,3'-diaminodiphenyl-methane, 4,4'-diaminodiphenylamine, 3,3'-diaminobenzo-phenone, 4,4'-diaminobenzophenone, acetoacetic o-chloroanilide, acetoacetic anilide, acetoacetic o-toluidide, acetoacetic p-toluidide, acetoacetic o-anisidide, acetoacetic m-xylidide and p-acetotoluidide.

Of these, diphenyl sulfone, di-p-methylbenzyl oxalate, benzil, β-naphthyl benzyl ether, p-benzylbiphenyl, 1,2-di(m-methylphenoxy)ethane, 1,2-diphenoxy-methylbenzene, m-terphenyl and stearamide are preferably used.

In addition, employment of a heat-meltable material of the following structural formula (XVIII), among the above-exemplified heat-meltable materials, in a heat-sensitive recording material is markedly effective in increasing the sensitivity of the recording material and improving the plasticizer resistance of a print portion and the heat resistance of the original recording material surface. Heat-meltable materials represented by the structural formula (XIX) shown below are especially preferable. 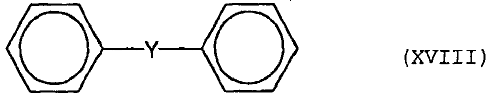 wherein Y is any of -SO₂-, -(S)ₙ-, -O-, -CO-, -CH₂-, -CH(C₆H₅)-, -C(CH₃)₂-, -COCO-, -CO₃-, -COCH₂CO-, -COOCH₂-, -CONH-, -OCH₂- and -NH-, n is 1 or 2, and hydrogen atom(s) of each benzene ring may be replaced by a halogen atom, a hydroxyl group, a nitro group, a nitroso group, a nitrile group, an isocyanate group, an isothiocyanate group, a mercapto group, a sulfamoyl group, a sulfone group, an amino group, an aromatic compound residue, an aliphatic compound residue or a heterocyclic compound residue. 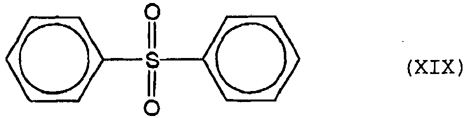 wherein hydrogen atom(s) of each benzene ring may be replaced by a halogen atom, a hydroxyl group, a nitro group, a nitroso group, a nitrile group, an isocyanate group, an isothiocyanate group, a mercapto group, a sulfamoyl group, a sulfone group, an amino group, an aromatic compound residue, an aliphatic compound residue or a heterocyclic compound residue.

The above-exemplified heat-meltable materials may be used singly or as a mixture thereof. For attaining a sufficient thermal response, the heat-meltable material is used in a proportion of preferably 10 to 300 parts by weight, more preferably 20 to 250 parts by weight, per 100 parts by weight of the colorless or light-colored dye precursor.

As a dispersing agent usable for preparing a dispersion of the heat-meltable material by grinding in the present invention, the same compounds as those used as the dispersing agent for dispersing the urea-urethane compound developer can be used. Of such compounds, the water-soluble polymers and the anionic surfactants are especially preferable for improving a heat-sensitive recording material in preservability of the original recording material surface against plasticizers. The poly(vinyl alcohol)s, modified poly(vinyl alcohol)s, methyl cellulose, hydroxypropylmethyl cellulose, condensed sodium naphthalenesulfonate, polycarboxylic acid ammonium salts, water-soluble low-molecular weight copolymers and sodium 2-ethylhexylsulfosuccinate are more preferable. Of these, the modified poly(vinyl alcohol)s, methyl cellulose, hydroxypropylmethyl cellulose, condensed sodium naphthalenesulfonate, polycarboxylic acid ammonium salts are still more preferable, and hydroxypropylmethyl cellulose is the most preferable. The above-exemplified dispersing agents may be used singly or as a mixture thereof.

When the urea-urethane compound developer is ground, fine grinding of the developer together with the above-mentioned heat-meltable material (co-grinding) further improves the sensitivity and plasticizer resistance of a heat-sensitive recording material as compared with fine grinding of each compound followed by mixing of the ground compounds. The reason why these effects are obtained is not completely clear.

Furthermore, the moisture resistance of the non-print portion (the original surface) of a heat-sensitive recording material can be improved by using at least one dispersing agent for the urea-urethane compound developer selected from methyl cellulose, hydroxypropylmethyl cellulose, condensed sodium naphthalenesulfonate and water-soluble low-molecular weight copolymers, and at least one dispersing agent for the heat-meltable material selected from modified poly(vinyl alcohol)s, methyl cellulose, hydroxypropylmethyl cellulose, condensed sodium naphthalenesulfonate and polycarboxylic acid ammonium salts.

The color-producing composition of the present invention can be used in various heat-sensitive recording materials and is especially suitable for heat-sensitive magnetic recording materials, labels for heat-sensitive recording, multicolor heat-sensitive recording materials, and heat-sensitive recording materials for laser marking.

When the color-producing composition of the present invention is used in a heat-sensitive magnetic recording material, the recording material is preferably in the following form: a heat-sensitive recording layer containing the urea-urethane compound developer is formed on one side of a substrate and a magnetic recording layer is formed on the other side.

The magnetic recording layer of the heat-sensitive magnetic recording material is formed by coating a substrate with a coating material prepared by uniformly dispersing ferromagnetic powder such as barium ferrite, strontium ferrite, Co-γ-Fe₂O₂ or γ-Fe₂O₂ in an aqueous binder such as an aqueous emulsion resin, and drying the coated substrate. In this case, various additives such as antistatic agents (e.g. carbon graphite), lubricants (e.g. wax), color pigments for hue adjustment, coating-film flexibilizers [e.g. poly(ethylene oxide)s] may be added depending on their purpose.

The heat-sensitive magnetic recording material of the present invention is suitable as heat-sensitive magnetic recording materials used as rail road tickets, tickets, prepaid cards.

When the color-producing composition of the present invention is used in a label for heat-sensitive recording, the label is preferably in the following form: a heat-sensitive recording layer containing the urea-urethane compound developer is formed on one side of a substrate and an adhesive layer is formed on the other side.

The adhesive layer of this heat-sensitive recording material is composed mainly of a pressure-sensitive adhesive. The pressure-sensitive adhesive includes, for example, synthetic-rubber-based emulsion type adhesives, acrylic emulsion type adhesives, natural-rubber-based solvent type adhesives, acrylic solvent type adhesives and silicon-based solvent type adhesives. Of these, the acrylic emulsion type adhesives are especially preferable.

When a reverse-side layer (a back coating layer) is, if necessary, formed between the adhesive layer and the substrate in the label for heat-sensitive recording produced by a process according to the present invention, taking-out of curl, prevention of electrostatic charge, and adjustment of coefficient of friction are possible in the label for heat-sensitive recording. As the components of a coating liquid for the reverse-side layer and a coating method for the coating liquid, the same components, method as in the formation of the heat-sensitive recording layer may be employed. The dry spread of the coating liquid ranges preferably from 0.2 to 10.0 g/m².

The order of coating in the production of the label for heat-sensitive recording is not particularly limited. For example, either of the following orders may be employed: the heat-sensitive recording layer is formed on one side of the substrate and then the back coating layer is formed on the other side, after which the adhesive layer is formed on the back coating layer; or the back coating layer is formed on one side of the substrate and then the heat-sensitive recording layer is formed on the other side, after which the adhesive layer is formed on the back coating layer.

As to a method for forming the adhesive layer on the back coating layer, a liquid for forming the adhesive layer may be directly applied on the back coating layer and dried, or a material obtained by previously applying a liquid for forming the adhesive layer, on release paper, followed by drying may be attached to the back coating layer side of a heat-sensitive recording material having no adhesive layer formed thereon.

In addition, the thermal response can be improved by forming an intermediate layer composed of a single layer or two or more layers, between the heat-sensitive recording layer and the substrate. The intermediate layer is composed mainly of an organic or inorganic pigment, hollow particles and an aqueous binder such as a water-soluble polymer or a latex. As the organic or inorganic pigment and the aqueous binder, the same organic or inorganic pigment and aqueous binder as used in the heat-sensitive recording layer can be used. A method for forming the intermediate layer is not particularly limited. As this method, the same method as the method for forming the heat-sensitive recording layer can be adopted. The dry spread for forming the intermediate layer ranges preferably from 2.0 to 15.0 g/m².

When the color-producing composition of the present invention is used in a multicolor heat-sensitive recording material, this recording material is preferably in the following form: at least two heat-sensitive recording layers are formed on one side of a substrate and at least one of said heat-sensitive recording layers contains the urea-urethane compound developer.

As the substrate used, there can be used synthetic paper produced by kneading a polyolefin resin and a white inorganic pigment with heating, extruding the kneaded product through a die, stretching the extruded product in a lengthwise direction, laminating one or two films made of a polyolefin resin and a white inorganic pigment on each side of the stretched product, and stretching the resulting assembly in a crosswise direction to make the same semitransparent or opaque; films obtained by kneading one of or a mixture of two or more of thermoplastic resins such as polyethylenes, polypropylenes, ethylene-vinyl acetate copolymer resins, poly(vinyl chloride)s, polystyrenes, polyesters with heating, extruding the kneaded product through a die, and then stretching the kneaded product biaxially; opaque films obtained by mixing a white inorganic pigment with any of the above-exemplified resins, followed by biaxial stretching; and substrates produced from pulp fiber, such as woodfree paper, medium-duty paper, machine glazed paper, regenerated paper, coated paper. The substrates made of pulp fiber are preferably coated with a heat-sensitive layer after previous formation of a layer for coating in order to improve the uniformity of image.

The heat-sensitive color-producing layer according to the present invention comprises as its main constituents an adhesive and a color-producing composition capable of causing color development reaction owing to the contact of materials with each other made by heating. Specific examples of the color-producing composition are combinations of a colorless or light-colored dye precursor and the above-mentioned urea-urethane developer capable of allowing said dye precursor to develop a color, on heating, and combinations of a diazo compound and a coupler capable of developing a color by its reaction with a diazo compound. If necessary, crosslinking agents, pigments and heat-meltable materials may be incorporated into the color-producing composition. Usually, the coating amount of the heat-sensitive color-producing layer is preferably 3 to 15 g/m² from the viewpoint of color development sensitivity and coloring density.

As a color-developable dye, the colorless or light-colored dye precursor already described is used which can develop a color when reacted with the urea-urethane compound developer with heating.

On the other hand, in a heat-sensitive recording layer comprising as its main constituents a diazo compound and a coupler capable of developing a color by its reaction with said diazo compound, these compounds are a well-known light-decomposable diazo compound and a coupler capable of forming a pigment by its reaction with said diazo compound. If necessary, a basic material may be added in order to accelerate the reaction of the diazo compound with the coupler. The coupler and the basic material are preferably used as a mixture thereof in proportions of 10 to 1,000 parts by weight and 10 to 2,000 parts by weight, respectively, per 100 parts by weight of the diazo compound.

The term "light-decomposable diazo compound" used herein means a diazo type photosensitive material capable of forming a pigment by its reaction with a coupling component on heating, such as a diazonium salt, diazosulfonate compound, diazoamino compound or quinonediazide compound. The diazonium salt refers to a compound represented by the general formula:

Ar-N₂⁺·X⁻

wherein Ar is an aromatic portion, N₂⁺ is a diazonium group, and X⁻ is a counter anion. Such compounds have various maximum absorption wavelengths, depending on the positions and kinds of substituents of the Ar portion.

Specific examples of the diazonium compound used in the present invention are 4-dimethylamino-benzenediazonium, 4-diethylaminobenzenediazonium, 4-dipropylaminobenzenediazonium, 4-methylbenzylamino-benzenediazonium, 4-dibenzylaminobenzenediazonium, 4-ethylhydroxyethylaminobenzenediazonium, 4-diethylamino-2-methoxybenzenediazonium, 4-dimethyl-3-methylbenzene-diazonium, 4-benzoylamino-2,5-diethoxybenzenediazonium, 4-morpholinobenzenediazonium, 4-morpholino-2,5-diethoxybenzenediazonium, 4-morpholino-2,5-dibutoxybenzenediazonium, 4-anilinobenzenediazonium, 4-toluylmercapto-2,5-diethoxybenzenediazonium, 4-(N,N-dioctylcarbamoyl)benzenediazonium, 2-octadecyloxybenzenediazonium, 4-(4-tert-octylphenoxy)benzenediazonium, 4-(2,4-di-tert-amylphenoxy)benzenediazonium, 2-(4-tert-octylphenoxy)benzenediazonium, 5-chloro-2-(4-tert-octylphenoxy)benzenediazonium, 2,5-bis-octadecyloxybenzenediazonium, 2,4-bis-octadecyloxybenzenediazonium, 4-(N-octyllauroylamino)benzenediazonium. Specific examples of the counter anion of the diazonium salt used in the present invention are C1·1/2ZₙCl₂⁻, BF₄⁻, PF₆⁻, B(ph)₄⁻, CₙF₂ₙ₊₁COO⁻ (n is 3 to 9), CₘF₂ₘ₊₁SO₃⁻ (m is 2 to 8) and (CₖF₂ₖ₊₁SO₂)₂CH⁻ (k is 1 to 18).

The diazosulfonate compound used in the present invention is a compound represented by the general formula:

Ar-N₂-SO₃Na

wherein Ar is an aromatic portion. Specific examples of the diazosulfonate compound used in the present invention are sodium benzenediazosulfonates having one or more substituents including 2-methoxy, 2-phenoxy, 2-methoxy-4-phenoxy, 2,4-dimethoxy, 2-methyl-4-methoxy, 2,4-dimethyl, 2,4,6-trimethyl, 2,4,6-trimethoxy, 2,4-dimethoxy-5-chloro, 2-methoxy-5-nitro, 2-methoxy-5-acetamido, 2-methoxy-5-N,N-diethylsulfonamido, 2-methoxy-5-N-phenylcarbamyl, 3-methyl, 4-methyl, 4-methoxy, 4-ethoxy, 4-phenyl, 4-phenoxy, 4-acetamido; and sodium benzenediazosulfonates having one or more substituents including 4-(N-ethyl-N-benzylamino), 4-(N,N-dimethylamino), 4-(N,N-diethylamino), 4-(N,N-diethylamino)-3-chloro, 4-(N-ethylamino)-3-methyl, 4-(N,N-diethylamino)-2-methyl, 4-(N-ethyl-N-β-hydroxy-ethylamino), 4-pyrrolidino-3-chloro, 4-pyrrolidino-3,5-dichloro, 4-morpholino, 4-morpholino-3-chloro, 4-morpholino-2-methoxy, 4-morpholino-2,5-diethoxy, 4-morpholino-2,5-dibutoxy, 4-(4'-tolylmercapto)-2,5-dibutoxy, 4-(4'-tolylmercapto)-2,5-diethoxy, 4-(4'-methoxybenzoylamino)-2,5-dibutoxy, 4-diphenylamino. When any of these diazosulfonate compounds is used, the diazosulfonate compound is preferably activated by light irradiation before printing.

The diazoamino compound usable in the present invention is a compound obtained by coupling a diazo group with dicyandiamide, sarcosine, methyltaurine, N-ethylanthranilic acid-5-sulfonic acid, monoethanolamine, diethanolamine or guanidine.

The quinonediazide used in the present invention is considered as an internal-salt type diazonium salt from the viewpoint of structure and is, for example, o-quinonediazide or o-naphthoquinonediazide. The quinonediazide includes salts, esters and amide compounds of 1,2-quinonediazide-4-sulfonic acid, 1,2-naphthoquinonediazide-5-sulfonic acid, 1,2-naphthoquinonediazide-4-sulfonic acid. Specific examples of the quinonediazide used in the present invention are sodium 1,2-quinonediazide-4-sulfonate, sodium 1,2-naphthoquinonediazide-5-sulfonate, sodium 1,2-naphthoquinonediazide-4-sulfonate, p-cumylphenyl 1,2-naphthoquinonediazide-5-sulfonate, p-cumylphenyl 1,2-naphthoquinonediazide-4-sulfonate, methyl 1,2-naphthoquinonediazide-5-sulfonate, ethyl 1,2-naphthoquinonediazide-5-sulfonate, 1,2-naphthoquinone-diazide-5-sulfonic acid dimethylamide, esters of 1,2-naphthoquinonediazide-5-sulfonic acid and a novolak resin. In addition, these light-decomposable diazo compounds may be used singly or in combination.

The coupler used in the present invention is one that reacts with the diazo compound to form a pigment. For example, typical couplers capable of forming a yellow pigment are compounds which have a methylene group activated by a carbonyl group adjacent thereto and are represented by the general formula RCOCH₂CO-R' wherein R is an alkyl group or an allyl group, and R' is an aromatic amine. Magenta couplers are, for example, 1) cyanoacetyl derivatives of cyclic compounds, or 2) heterocyclic compounds having active methylene or any other coupling portion on the heterocyclic ring. The magenta couplers include, for example, pyrazolone compounds and indazolone compounds. Cyan couplers include, for example, phenols and naphthols.

Specific examples of the coupler used in the present invention are 4-(p-toluenesulfonylamino)-ω-benzoylacetanilide, α-benzoyl-o-methoxyacetanilide, 2-cyanoacetyl-coumarone, 1-(2,4,6-trichlorophenyl)-3-p-nitroamino-2-pyrazolon-5-one, resorcin, phloroglucin, 2,3-dihydroxynaphthalene, 2,6-dibromo-1,5-dihydroxy-naphthalene, N-(o-acetamidophenethyl)-1-hydroxy-2-napthoamide. In addition, these couplers may be used singly or in combination.

For smoother progress of the coupling reaction of the diazo compound with the coupler under a basic atmosphere, a basic material is preferably incorporated into the heat-sensitive color-producing layer. As the basic material, a slightly water-soluble or water-insoluble basic material or a material capable of producing an alkali on heating is used. The basic material includes, for example, nitrogen-containing compounds such as inorganic and organic ammonium salts, organic amines, amides, urea and thiourea and their derivatives, thiazoles, pyrroles, pyrimidines, piperazines, guanidines, imidazoles, imidazolines, triazoles, morpholines, piperidines, amidines, formamidines, pyridines.

Specific examples of these compounds are tricyclohexylamine, tribenzylamine, octadodecylbenzyl-amine, stearylamine, allylurea, thiourea, methyl-thiourea, allylthiourea, ethylenethiourea, 2-benzylimidazole, 4-phenylimidazole, 2-phenyl-4-methylimidazole, 2-undecylimidazoline, 2,4,5-trifuryl-2-imidazoline, 1,2-diphenyl-4,4-dimethyl-2-imidazoline, 2-phenyl-2-imidazoline, 1,2,3-triphenylguanidine, 1,2-dicyclohexylguanidine, 1,2,3-tricyclohexylguanidine, guanidine trichloroacetate, N,N'-dibenzylpiperazine, 4,4'-dithiomorpholine, morpholinium trichloroacetate, 2-aminobenzothiazole, 2-benzoylhydrazinobenzothiazole. These basic materials may be used singly or in combination.

In the present invention, the storage stability can be improved by adding a weakly acidic material such as citric acid, tartaric acid, oxalic acid, boric acid, phosphoric acid or pyrophosphoric acid to the heat-sensitive color-producing layer formed of a combination of the diazo compound and the coupler.

Needless to say, the color-producing components used in the present invention can be used in a solid dispersion state attained by dispersing the components in an aqueous solution of a water-soluble polymer, followed by coating and drying, as in a conventional method adopted in heat-sensitive recording materials. It is also possible to improve the green stability by encapsulating a color former to form microcapsules and preventing the contact of the color former with a developer at ordinary temperatures by utilizing the isolating effect of the capsule walls, as described in JP-A-59-190886, JP-A-60-49991, JP-A-61-169281. The microcapsules are characterized in that they enable the color former and the developer to come into contact with each other only during heating at a certain temperature or higher. The temperature at the starting of the contact of the color former with the developer can be controlled by properly choosing a material for the capsule wall, a core material for the capsule, additives.

As a material for the walls of the microcapsules in the present invention, there are mentioned conventional materials for microcapsule wall, such as polyurethanes, polyureas, polyesters, polycarbonates, urea-formaldehyde resins, melamine resins, polystyrenes, styrene-methacrylate copolymers, gelatin, poly(vinylpyrrolidone)s, poly(vinyl alcohol)s. These polymers may be used singly or in combination.

In the present invention, as the adhesive contained in the heat-sensitive color-producing layer, either water-soluble resins or water-dispersible resins may be used. However, when any of these resins is mixed with dispersions of the above-mentioned color-developable dye and developer, respectively, the resulting mixture should be free from coloration, aggregation and a high viscosity. In addition, a coating film formed as heat-sensitive recording layer should be tough and should not have desensitizing effect. The content of the adhesive in the heat-sensitive color-producing layer is preferably 8 to 20% based on the amount (in terms of solids) of the heat-sensitive color-producing layer. A content of less than 8% is disadvantageous in that the strength of the coating film is low. A content of more than 20% involves a problem of sensitivity decrease. In order to improve the water resistance of the heat-sensitive color-producing layer, a crosslinking agent for curing the resin can be used.

In the multicolor heat-sensitive recording material of the present invention, forming an intermediate layer between heat-sensitive recording layers is effective in improving the thermal partitionment. The intermediate layer comprises as its main constituent the same resin as the water-soluble or water-dispersible resin used as the adhesive in the heat-sensitive recording layers, and may further comprises pigments, crosslinking agents. The coating amount of the intermediate layer is preferably 1.0 to 5.0 g/m². When the coating amount is less than 1.0 g/m², no sufficient preventive effect on diffusion between the recording layers can be obtained, resulting in a deteriorated quality of image. When the coating amount is more than 5.0 g/m², the sensitivity is disadvantageously decreased.

As an especially preferable form of the multicolor heat-sensitive recording material of the present invention, there can be mentioned a multicolor heat-sensitive recording material in which two heat-sensitive recording layers which have different color development temperatures, respectively, and undergo color development in different color tones, respectively, are laminated on one side of a substrate, and of these recording layers, the upper heat-sensitive recording layer contains either an agent used both as developer and tone reducer, or a reversible developer, and the lower heat-sensitive recording layer contains the urea-urethane compound developer.

Of these, the agent used both as developer and tone reducer in the upper heat-sensitive recording layer is an amphoteric compound having an acidic group having a color-developing function and a basic group having an achromatizing function, which performs the color-developing function on heating at a low temperature and performs the achromatizing function on heating at a high temperature. A typical example of the acidic group is phenolic hydroxyl group or carboxyl group. A typical example of the basic group is amino group. Although the amphoteric compound may have the basic group as a functional group, it is preferably has the basic group as a portion of a salt compound, as in a complex of a phenolcarboxylic acid compound and an amine compound. Specific examples of such an agent used both as developer and tone reducer are as follows. The phenolcarboxylic acid compound that constitutes the agent used both as developer and tone reducer includes 2-hydroxybenzoic acid, 3-hydroxybenzoic acid, 4-hydroxybenzoic acid, 3,4-dihydroxybenzoic acid, 3,5-dihydroxybenzoic acid, 2,3-dihydroxybenzoic acid, 2,4-dihydroxybenzoic acid, 2,5-dihydroxybenzoic acid, 2,6-dihydroxybenzoic acid, gallic acid, bis(4-hydroxy-phenyl)acetic acid, 3,3-bis(4-hydroxyphenyl)propionic acid.

The amine compound that forms the salt or complex salt together with the phenolcarboxylic acid compound includes octylamine, nonylamine, decylamine, laurylamine, tetradecylamine, heptadecylamine, stearylamine, behenylamine, 3-methoxypropylamine, hexamethylenediamine.

The reversible developer is, for example, a phenolic compound or a phosphonic acid compound, which has an aliphatic hydrocarbon group of 8 or more carbon atoms. Specific examples of such a reversible developer are those mentioned below. The reversible developer is not limited to those mentioned below, and any reversible developer may be used so long as it performs a color-developing function on heating at a low temperature and performs an achromatizing function on heating at a high temperature.

The reversible developer includes 4-(octadecylthio)phenol, 4-(dococylthio)phenol, 4-(octadecyloxy)phenol, 4-(dococyloxy)phenol, N-octadecyl-4-hydroxybenzamide, 4'-hydroxydocosane-anilide, N-(4-hydroxyphenyl)-N'-n-octadecylurea, docosylphosphonic acid. When recording is conducted with a thermal printer by using the multicolor heat-sensitive recording material of the present invention, printing by heating at a low temperature causes color development only in the low-temperature color-producing layer, and printing by heating at a high temperature causes achromatization in the low-temperature color-producing layer in a print portion and causes color development only in the high-temperature color-producing layer.

Employment of the urea-urethane compound makes it possible to obtain an article for laser marking which is sufficient in coloring density and gives such a very stable printed developed color image that the image is hardly discolored or faded even by fats and oil, chemicals, fingerprints. Therefore, the employment is especially advantageous from the viewpoint of long-term preservation of records.

The heat-sensitive recording layer of the article for laser marking of the present invention preferably contains a recording sensitivity improving agent.

As the recording sensitivity improving agent usable therein, compounds capable of absorbing laser beams used for irradiation are used. Specific examples thereof are various inorganic compounds such as aluminum hydroxide, wollastonite, bentonite, micas (e.g. muscovite and phlogopite), calcium silicate, talc, kaolin, clay, and silicate minerals (e.g. foyaite, hornblende and albite). Aluminum hydroxide, muscovite, wollastonite and kaolin are especially preferable. These inorganic compounds may be used singly or as a mixture thereof.

The proportions of the colorless or light-colored dye precursor and recording sensitivity improving agent used in the heat-sensitive recording layer in the present invention are not particularly limited and may be properly chosen depending on the kinds of the dye precursor and recording sensitivity improving agent used. Usually, the recording sensitivity improving agent can be used in a proportion of 10 to 5,000 parts by weight, preferably 100 to 2,000 parts by weight, per 100 parts by weight of the color former.

The contents of the dye precursor, the urea-urethane compound developer and the recording sensitivity improving agent in the heat-sensitive recording layer can be adjusted as follows: based on the total weight (in terms of solids) of said layer, the content of the dye precursor ranges from 5 to 30 wt%, preferably from 10 to 25 wt%, the content of the urea-urethane compound developer ranges from 10 to 60 wt%, preferably from 20 to 50 wt%, and the content of the recording sensitivity improving agent ranges from 5 to 40 wt%, preferably from 10 to 30 wt%.

The incorporation of also an acidic developer into the heat-sensitive recording layer of the article for laser marking of the present invention improves the sensitivity and enables the article for laser marking to produce a brilliant color. As the acidic developer, the above-exemplified conventional electron-accepting materials are used.

For further improving the sensitivity of the article for laser marking of the present invention, a heat-meltable material can be incorporated into the heat-sensitive recording layer. The heat-meltable material is preferably one which has a melting point of 60°C to 180°C, in particular, 80°C to 140°C.

The color-producing marking composition can be obtained by using the above-mentioned colorless or light-colored dye precursor, urea-urethane compound developer, recording sensitivity improving agent and aqueous binder and water as essential constituents, and mixing various assistants therewith if necessary.

The water used in the color-producing marking composition has a pH in a range of 5 to 12, preferably 6 to 9. When the pH is lower than 5, fog is caused. When the pH is higher than 12, there are likely to be undesirable influences such as loss of the color-developing capability of the urea-urethane compound developer. The water may be used in admixture with a water-soluble organic solvent such as methanol or ethanol.

In addition to the essential constituents described above, various assistants may, if necessary, be incorporated into the color-producing marking composition used in the present invention, in order to, for example, facilitate the application of the composition on a substrate. The various assistants include, for example, dispersing agents (e.g. sodium dioctyl-sulfosuccinate, sodium dodecylbenzenesulfonate, lauryl alcohol sulfuric acid ester sodium salt, and fatty acid metal salts), opacifying agents (e.g. titanium oxide), defoaming agents, viscosity modifiers, fluorescent dyes, and coloring agents.

A substrate on which the color-producing marking composition used in the present invention is printed (applied) is not particularly limited so long as it requires marking. As the substrate, there are mentioned, for example, the predetermined portions of food containers, packaging materials and electronic parts, and articles (e.g. substrates for label) to be attached thereto. As the substrates for label, papers (e.g. paper and synthetic paper), synthetic resin films, plastics, metallized paper and synthetic paper, metallized films, metals, wood are properly used depending on their purpose.

The color-producing marking composition is prepared, for example, as follows. The binder is dissolved or dispersed in water or a solvent composed mainly of water. Among the color former, the developer and the recording sensitivity improving agent, components that should be dispersed in water or a solvent composed mainly of water are treated together or individually in water or an aqueous solvent, which contains a dispersing agent such as a poly(vinyl alcohol), by using a dispersing machine such as a ball mill, attritor or sand grinder, whereby one or more dispersions are prepared. The average particle size of each component after the dispersing operation is usually about 2 µ or less, preferably about 1 µ or less. Then, the binder and the dispersion(s) are mixed to obtain the color-producing marking composition of the present invention. The solid content of said color-producing marking composition is 20 to 70 wt%, preferably about 30 to about 65 wt%.

The color-producing marking composition may be applied directly on the substrate, or it may be applied on the substrate that has been previously subjected to surface treatment or undercoating. The application can be carried out by using a suitable coater such as a roll coater, gravure coater, micro-gravure coater, knife coater or spray coater. The thickness of the coating film (the heat-sensitive recording layer) obtained by the application and drying can be usually adjusted to 1 to 4 µ. When the thickness is less than 1 µ, color development by laser irradiation is not sufficient and moreover, the coating film tends to be peeled off. On the other hand, when the thickness is more than 4 µ, the drying characteristics and the label attachability tend to be deteriorated. The drying is varied depending on coating conditions such as the speed of line and may be conducted either at room temperature, or by heating under conditions which do not cause color development in the heat-sensitive recording layer.

The protective layer of the article for laser marking is formed by applying a transparent clear coating liquid on the heat-sensitive recording layer. The clear coating liquid is an aqueous composition consisting of or comprising an aqueous binder and water.

As the aqueous binder used in the clear coating liquid for the protective layer in the present invention, there are mentioned those obtained by using as a base a per se known water-soluble or water-dispersible resin used in a coating material or ink. Such a resin has a hydrophilic group (e.g. carboxyl group or amino group) optionally introduced thereinto for impartment of the water-solubility or water-dispersibility. As said resin for the aqueous binder, a resin having a glass transition temperature in a range of 20 - 80°C, preferably 35 - 70°C is used. When the glass transition temperature is lower than 20°C, the scuff resistance, chemical resistance and water resistance of the protective layer are deteriorated. On the other hand, when the glass transition temperature is higher than 80°C, the protective layer is brittle, is poor in flexibility, and is easily cracked. Therefore, both of such glass transition temperatures are not desirable. If necessary, leveling agents, slip-properties-imparting agents and defoaming agents may be incorporated into said clear coating liquid in addition to the components described above.

As the aqueous binder used in the clear coating liquid, an acrylic resin can be obtained by using an alkyl (number of carbon atoms: 1 to 24) ester of acrylic acid or methacrylic acid as a main component in combination with any of, for example, unsaturated carboxylic acids such as acrylic acid, methacrylic acid, maleic acid; hydroxy-containing unsaturated monomers such as hydroxyethyl acrylate, hydroxypropyl methacrylate; amino-containing unsaturated monomers such as acrylamide, methacrylamide; and other unsaturated monomers such as styrene, acrylonitrile, vinyl acetate, vinyl chloride, and copolymerizing the alkyl ester with such a comonomer. A polyester resin can be obtained by the ester reaction of a polybasic acid (including acid anhydrides) having two or more carboxyl groups in the molecule with a polyhydric alcohol having two or more hydroxyl groups in the molecule. The glass transition point of such an aqueous binder can be adjusted to any temperature by properly choosing the kinds, combination and proportions of components that constitute said binder.

Said clear coating liquid is prepared by dissolving or dispersing the above-mentioned aqueous binder in suitable water, and if necessary, is incorporated with leveling agents, slip-properties-imparting agents and defoaming agents. The solid content of said clear coating liquid ranges preferably from 20 to 70 wt%, in particular, from 30 to 60 wt%.

Said clear coating liquid can be printed (applied) on the surface of a dried coating film formed as the heat-sensitive recording layer. A method for the printing (application) is not particularly limited. The application can be carried out by means of a roll coater, gravure coater, micro-gravure coater or spray coater.

The thickness of the coating film thus formed can be usually adjusted to 3 to 10 µ. When the thickness is less than 3 µ, the protection of the heat-sensitive recording layer afforded by said clear coating is not sufficient and the chemical resistance and rub resistance are deteriorated. On the other hand, when the thickness is more than 10 µ, the drying characteristics and physical performance of the coating film tend to be deteriorated. Drying of said clear coating liquid is varied depending on coating conditions such as the speed of line and may be conducted either at room temperature, or by heating under conditions which do not cause color development in the heat-sensitive recording layer.

When the thus formed heat-sensitive recording layer of the article for marking is irradiated with laser beams, the irradiated portion is heated, so that the urea-urethane compound developer and the dye precursor react with each other to develop a color, resulting in marking. Although the amount of energy of laser beams used for the irradiation is not particularly limited, it is preferably 1.4 J (joule)/cm² or less when the possibility of fracture of the coating film is taken into consideration. On the other hand, although the lower limit of the amount of energy required for color development is unknown because there is no apparatus capable of producing low energy, sufficient color development takes place even at an amount of energy of 0.4 J/cm². Therefore, the suitable amount of energy for color development by the irradiation ranges from 0.4 to 1.4 J/cm², in particular, from 0.45 to 1.2 J/cm². As a laser used for the irradiation, a pulsed laser or a scanning laser is suitable. As to the kind of the laser, any of, for example, gas lasers, excimer lasers and semiconductor lasers may be used. Specific examples of the laser are carbon dioxide lasers, mixed gas lasers, YAG lasers, ruby lasers.

As a method for irradiating a portion of a desirable form with laser beams, there are mentioned a method of irradiating the coating film with laser beams through a metal mask to irradiate the coating film with laser beams in conformity with the form of the opening of the metal mask; and a method of inputting a desirable form to a computer and irradiating the coating film with laser beams in accordance with the desirable form in a manner of so-called drawing with a single stroke. When the heat-sensitive recording layer is irradiated with laser beams, the irradiated portion is increased in temperature to undergo color development caused by melting and mixing of the color-producing components in the heat-sensitive recording layer, resulting in the appearance of letters or a figure, which has a clear desirable form. This color change by the irradiation can be carried out in a moment because the energy density of laser beams is high.

A label as the article for laser marking of the present invention can be produced by forming a heat-sensitive recording layer and a protective layer by the above-mentioned methods on a substrate for label properly selected depending on its purpose from papers (e.g. paper and synthetic paper), synthetic resin films, plastics, metallized paper and synthetic paper, metallized films, metals. Although the label thus obtained can be used in all the fields of conventional labels, it is preferably used in the fields of, in particular, food, medicine, toiletries, publications, and electric and electronic parts.

A packaging material as the article for laser marking is produced by forming a heat-sensitive recording layer and a protective layer by the above-mentioned methods on any of various conventional packaging materials such as boxes, packing paper and packages, which are obtained by the use of a substrate properly selected depending on its purpose from papers (e.g. paper and synthetic paper), synthetic resin films, plastics, metallized paper and synthetic paper, metallized films, metals, glass, wood. Although said packaging material can be used in all the fields of conventional packaging materials, it is preferably used in the fields of, in particular, food, medicine, toiletries, publications, and electric and electronic parts.

A container as the article for laser marking of the present invention is produced by forming a heat-sensitive recording layer and a protective layer on a substrate such as glass, plastic or metal by the above-mentioned methods. This food container can be used in all the fields of conventional food containers, such as the fields of bottles for liquors and refreshing drinks, retort-food containers, instant-food containers, cosmetics containers, medicine container, toiletry products.

As a binder, conventional various binders can be used. The binder includes, for example, water-soluble binders such as starches, hydroxyethyl cellulose, methyl cellulose, carboxymethyl cellulose, gelatin, casein, poly(vinyl alcohol)s, modified poly(vinyl alcohol)s, sodium poly(acrylate)s, acrylamide-acrylic ester copolymers, acrylamide-acrylic ester-methacrylic acid terpolymers, alkali salts of styrene-maleic anhydride copolymers, alkali salts of ethylene-maleic anhydride copolymers; and latex type water-insoluble binders of styrene-butadiene copolymers, acrylonitrile-butadiene copolymers, methyl acrylate-butadiene copolymers.

In the recording material , the recording layer may contain a hindered phenol compound or an ultraviolet absorber. The hindered phenol compound or ultraviolet absorber includes, for example, 1,1,3-tris(3'-cyclohexyl-4'-hydroxyphenyl)butane, 1,1,3-tris(2-methyl-4-hydroxy-5-tert-butylpheny)butane, 4,4'-thiobis(3-methyl-6-tert-butylphenol), 1,3,5-trimethyl- 2,4,6-tris(3,5-di-tert-butyl-4-hydroxybenzyl)benzene, 2,2'-dihydroxy-4,4'-dimethoxybenzophenone, p-octylphenyl salicylate, 2-(2'-hydroxy-5'-methylpheny)benzotriazole, ethyl-2-cyano-3,3'-diphenyl acrylate and tetra(2,2,6,6-tetramethyl-4-piperidyl)-1,2,3,4-butanetetracarbonate.

A method for forming the color-producing layer is not particularly limited. The color-producing layer is formed, for example, by applying a coating liquid for the color-producing layer on a substrate by a suitable coating method such as free-fall curtain coating, air-knife coating, Bariber blade coating, Pure blade coating or short-dwell coating, and drying the thus treated substrate. The coating amount of the coating liquid for the color-producing layer is not particularly limited and is usually controlled in a range of 1 to 15 g/m², preferably about 3 to about 10 g/m², in terms of dry weight.

The thermal response can be improved by forming an intermediate layer between the heat-sensitive recording layer and the substrate. In the case of conventional heat-sensitive recording materials, a technique for improving the color development sensitivity, for example, by co-using a heat-meltable material in a color-producing layer has been employed. The improvement of the sensitivity by such a method is disadvantageous in that fog tends to be caused by heat or friction. It facilitates the occurrence of fog particularly in heat-sensitive recording materials obtained by using the urea-urethane compound developer excellent in color development sensitivity. In the case of conventional heat-sensitive recording materials without print preservability, even if fog is caused, it disappears like a print, so that, in particular, fog in the case of using the recording material after long-term storage is seldom taken into account. However, in the case of heat-sensitive recording materials obtained by using the urea-urethane compound developer especially excellent in long-term print preservability, the following problem characteristic of them is caused in some cases: when fog is once caused, it is preserved and hence it is accumulated each time the recording material is stored and then used, so that the recording material surface becomes dirty when the recording material is used after long-term storage. In such a case, by forming the intermediate layer, a practical color development sensitivity can be attained without using a heat-meltable material or by using only a small amount of a heat-meltable material, and a heat-sensitive recording material can be obtained which is so excellent in resistance to fog caused by heat or friction that the fog is hardly accumulated even if the recording material is used after long-term storage.

The intermediate layer is composed mainly of an organic or inorganic pigment, hollow particles and an aqueous binder such as a water-soluble polymer or a latex. As the organic or inorganic pigment and the aqueous binder, the same organic or inorganic pigment and aqueous binder as used in the heat-sensitive recording layer can be used. A method for forming the intermediate layer is not particularly limited. As this method, the same method as the method for forming the heat-sensitive recording layer can be adopted. The dry spread for forming the intermediate layer ranges preferably from 2.0 to 15.0 g/m². In this case, the surface pH of the intermediate layer formed on the substrate is preferably 3 to 9, more preferably 5 to 9, most preferably 6 to 8.

In addition, in the present invention, the rub resistance of the resulting recording paper can be improved by forming a protective layer composed mainly of a water-soluble polymer, on the heat-sensitive recording layer if necessary. When the urea-urethane compound developer of the present invention is used which is excellent in color development sensitivity and resistance of print to long-term storage, a trace of rubbing tends to be left because of the high sensitivity. On the other hand, the trace of rubbing once left is preserved for an indefinite time and hence traces of rubbing are accumulated each time the recording material is stored and then used. Therefore, the paper surface becomes dirty in some cases when the recording paper is used after long-term storage. In such a case, by forming the protective layer, the accumulation of traces of rubbing can be made difficult even if the recording paper is used after long-term storage. Specific examples of the water-soluble polymer contained in the protective layer are the water-soluble polymer binders mentioned in relation to the above-mentioned heat-sensitive recording layer. The water-soluble polymer can be used together with a conventional waterproofing agent capable of waterproofing the water-soluble polymer. Specific examples of the waterproofing agent are formaldehyde, glyoxal, chrome alum, melamine, melamine-formaldehyde resins, polyamide resins, polyamide-epichlorohydrin resins.

Furthermore, pigments, metal soaps, waxes, crosslinking agents are incorporated into the protective layer for the purpose of, for example, improving the matching with a thermal head during printing and improving the water resistance of the protective layer.

The pigments include zinc oxide, calcium carbonate, barium sulfate, titanium oxide, lithopone, talc, pagodite, kaolin, aluminum hydroxide, silica, amorphous silica. The amount of the pigments added is 0.5 to 4 times, preferably 0.8 to 3.5 times, the total weight of the polymers. When the amount is below the lower limit of the above range, the pigments are not effective in improving the matching with a thermal head. When the amount is above the upper limit, the sensitivity of the heat-sensitive recording material is remarkably decreased, so that the commercial value of the recording material is impaired.

The metal soaps include, for example, emulsions of higher fatty acid metal salts such as zinc stearate, calcium stearate, aluminum stearate. The metal soaps are added in a proportion of 0.5 to 20 wt%, preferably 1 to 10 wt%, based on the total weight of the protective layer. The waxes include, for example, emulsions of paraffin wax, microcrystalline wax, carnauba wax, methylolstearoamide, polyethylene wax. The waxes are added in a proportion of 1 to 20 wt%, preferably 1 to 10 wt%, based on the total weight of the protective layer.

In forming the protective layer on the heat-sensitive recording layer, a surfactant is added to a coating liquid for forming the protective layer, in order to obtain a uniform coating layer. The surfactant includes alkali metal salts of sulfosuccinic acids, fluorine-containing surfactants. Specific examples of the surfactant are sodium salts or ammonium salts of di-(2-ethylhexyl)sulfosuccinic acid, di-(n-hexyl)sulfosuccinic acid. In general, any surfactant is effective so long as it is anionic. Conventional auxiliary additives such as fillers, heat-meltable materials (lubricants), surfactants, fluorescent dyes may also be incorporated into the protective layer. Specific examples of the fillers, heat-meltable materials and fluorescent dyes are those mentioned in relation to the above-mentioned heat-sensitive recording layer. The dry spread of the protective layer is preferably about 0.5 to about 10 g/m², in particular, about 1 to about 5 g/m².

When a reverse-side layer (a back coating layer) is, if necessary, formed on the side reverse to the recording layer of the recording material produced by the process of the present invention, curling of the resulting recording paper can be made difficult. Particularly in the case of the urea-urethane compound developer of the present invention, the formation of the reverse-side layer is effective for the following reason: said developer is superior to other developers in dispersibility, gives a dispersion with a small particle size easily, and has a high sensitivity, but when a coating liquid prepared by mixing said developer with other necessary components is applied on a substrate, the cohesive force of a binder is increased at the time of drying because of the small particle size of the developer, so that the recording layer is eaily shrunk, resulting in easy curling. As the components of the coating liquid for the reverse-side layer and a coating method for the coating liquid, the same components and method as in the case of the protective recording layer may be employed. The dry spread of the coating liquid ranges preferably from 0.2 to 10.0 g/m².

The present invention is explained in further detail with the following examples.

The analyses of materials and the evaluation of physical properties were carried out by the following methods.

### <IR spectrum>

Measured by diffuse reflectance spectroscopy by the use of FTIR-8100M manufactured by Shimadzu Corp.

### <Mass spectrum>

Measured by using JMS-HX100 manufactured by JEOL LTD., nitrobenzyl alcohol as a matrix, and xenon as a primary gas.

### <Color development sensitivity of thermal paper>

Coloring density at an applied voltage of 24 V and a pulse width of 1.5 msec was measured with an optical densitometer by using a printing tester manufactured by Ohkura Denki K.K., and a thermal head KJT-256-8MG manufactured by Kyocera Co., Ltd.

### <Plasticizer resistance>

A heat-sensitive recording material was held between vinyl chloride wrap films or in a vinyl chloride file, and a load of 300 g/cm² was applied thereto from above. After standing at 40°C for 24 hours, the coloring density of the printed portion and the non-printed portion (the original recording material surface) was visually estimated. When there was only a slight decrease in print density, the print preservability was rated as good.

### <Heat resistance>

A heat-sensitive recording material was allowed to stand at 60°C and 25% RH for 24 hours and the degree of fading of print was visually estimated. When the degree of fading is low, the print preservability was rated as good.

In addition, a heat-sensitive recording material was allowed to stand at 80°C and 25% RH for 24 hours and the degree of fading of print was visually estimated. When the degree of fading was low, the print preservability was rated as good. The coloring density of the original recording material surface was also visually estimated. When the color development was slight, the preservability of original recording material surface was rated as good.

### Example 1

To 27.8 g of 2,4-toluene diisocyanate was added 111 g of toluene as a solvent, followed by adding dropwise thereto a solution of 7.4 g of aniline in 37 g of toluene at room temperature over a period of 1 hour, and the reaction was carried out for another 1 hour. The white solid precipitated was recovered by filtration, washed with hexane and then dried overnight in a vacuum to obtain 20 g of white crystals. Subsequently, 5 g of the thus obtained compound was added to 50 mL of methanol, and the reaction was carried out at 60°C for 30 minutes, after which the excess methanol was removed by the use of an evaporator, and toluene was added to the residue to effect crystallization. The resulting white crystals were recovered by filtration, washed with hexane and then dried overnight in a vacuum to obtain 5.4 g of white crystals. The melting point of these white crystals was 196°C.

Analytical measurement of these white crystals was as follows.

### Result of IR measurement:

Characteristic peaks appeared at 1060 cm⁻¹, 1250 cm⁻¹, 1600 cm⁻¹, 1650 cm⁻¹, 1670 cm⁻¹, 1700 cm⁻¹ and 3300 cm⁻¹.

The structural formula of the major component of this compound is presumed to be the structural formula of the above-mentioned compound (S-1).

Next, a dispersion was prepared by dispersing 2 g of this compound by grinding it together with 8 g of a 2.5 wt% aqueous solution of a poly(vinyl alcohol) (Gohseran L-3266, a trade name, mfd. by The Nippon Synthetic Chemical Industry Co., Ltd.) in a paint shaker for 6 hours. The temperature of the dispersion immediately after the dispersing operation was 25°C. The diameter of dispersed particles of the compound was about 0.6 µm.

Another dispersion was prepared by dispersing 70 g of 3-dibutylamino-6-methyl-7-anilinofluoran by grinding it together with 130 g of a 8 wt% poly(vinyl alcohol) aqueous solution in a sand grinder (mfd. by AIMEX CO., LTD.; vessel capacity 400 ml) at a number of revolution of 2,000 rpm for 3 hours.

Further another dispersion was prepared by dispersing 70 g of diphenyl sulfone by grinding it together with 130 g of a 5.4 wt% poly(vinyl alcohol) aqueous solution in a sand grinder (mfd. by AIMEX CO., LTD.; vessel capacity 400 ml) at a number of revolution of 2,000 rpm for 3 hours.

Still another dispersion was prepared by mixing 10 g of calcium carbonate with 30 g of water and stirring the mixture by the use of a stirrer.

A coating liquid was obtained by stirring and mixing the above-mentioned dispersions and other components in the following proportions (dry basis proportions); the dispersion of the above-mentioned compound in terms of dry solids: 30 parts by weight, the 3-dibutylamino-6-methyl-7-anilinofluoran dispersion in terms of dry solids: 15 parts by weight, the diphenyl sulfone dispersion in terms of dry solids: 30 parts by weight, the calcium carbonate dispersion in terms of dry solids: 20 parts by weight, a zinc stearate dispersion (solid content: 16 wt%) in terms of dry solids: 10 parts by weight, and a 15 wt% poly(vinyl alcohol) in terms of dry solids: 7 parts by weight.

The coating liquid was applied on base paper with a basis weight of 50 g/m² by the use of a bar coater of rod number 10. After drying, supercalendering was conducted to obtain a heat-sensitive recording material. The coating amount of the coating liquid was 4 g/m² in terms of dry weight.

The result of evaluating the sensitivity of the heat-sensitive recording material obtained was so good that the optical density was 1.2. The result of estimating the degree of a thermal color change of the original recording material surface (the heat resistance) was so good that the color change was slight. The thermal fading of the print portion was desirably slight. These evaluation results are summarized in Table 1.

### Example 2

To 17 g of 2,4-toluene diisocyanate was added 40 g of methyl ethyl ketone as a solvent, followed by adding dropwise thereto 3.8 g of methanol, and the reaction was carried out with stirring at 60°C for 5 hours. Then, 9.9 g of 4,4'-diaminodiphenyl sulfone was added thereto, and the reaction was carried out with stirring at 60°C for 4 hours. After completion of the reaction, the reaction solution was cooled to room temperature and poured into 800 g of acetonitrile, and the crystals precipitated were recovered by filtration, washed with hexane and then dried overnight in a vacuum to obtain 15 g of a compound as white crystals.

The melting point of the white crystals was 169°C, and their analytical measurements were as follows.

### Result of IR measurement:

Characteristic peaks appeared at 1220 cm⁻¹, 1550 cm⁻¹, 1590 cm⁻¹, 1660 cm⁻¹, 1740 cm⁻¹ and 3300 cm⁻¹. Result of mass spectrum measurement:
[M+H]⁺ was detected at m/z 661.

The structural formula of the major component of this compound is presumed to be the structural formula of the above-mentioned compound (S-13).

Then, a heat-sensitive recording material was produced in the same manner as in Example 1 except for using the compound obtained above, in place of the urea-urethane compound synthesized in Example 1, and was evaluated. The results obtained are summarized in Table 1.

### Example 3

In 100 mL of ethyl acetate was dissolved 3.46 g of aniline, and the resulting solution was stirred at room temperature. A solution of 10 g of trimethylolpropane adduct of toluene diisocyanate (Coronate L, a trade name, mfd. by Nippon Polyurethane Industry Co., Ltd.; a 75% ethyl acetate solution) in 50 mL of ethyl acetate was added dropwise thereto over a period of 1 hour, and the reaction was carried out for another 30 minutes. The crystals formed were recovered by filtration and dried overnight in a vacuum to obtain 5.1 g of a compound as white crystals. The melting point of the white crystals was 161°C, and their analytical measurement was as follows.

### Result of IR measurement:

Characteristic peaks appeared at 1070 cm⁻¹, 1220 cm⁻¹, 1550 cm⁻¹, 1600 cm⁻¹, 1700 cm⁻¹ and 3300 cm⁻¹.

The structural formula of the major component of this compound is presumed to be the structural formula of the above-mentioned compound (S-33).

Then, a heat-sensitive recording material was produced in the same manner as in Example 1 except for using the compound obtained above, in place of the urea-urethane compound synthesized in Example 1, and was evaluated. The results obtained are summarized in Table 1.

### Example 9

To 27.8 g of 2,4-toluene diisocyanate was added 100 g of toluene as a solvent, followed by adding dropwise thereto a solution of 7.4 g of aniline in 37 g of toluene at room temperature over a period of 1 hour, and the reaction was carried out for another 1 hour. The white solid precipitated was recovered by filtration, washed with hexane and then dried overnight in a vacuum to obtain 20 g of white crystals. Subsequently, 5 g of the thus obtained compound was added to 50 mL of methanol, and the reaction was carried out at 60°C for 30 minutes, after which the excess methanol was removed by the use of an evaporator, and the residue was dried overnight in a vacuum to obtain 5.4 g of a urea-urethane composition as white crystals. The melting point of the white crystals was 196°C. In IR measurement of the crystals, characteristic peaks due to a urea-urethane compound appeared at 1670 cm⁻¹ and 1700 cm⁻¹. The content of the urea-urethane main constituent in the urea-urethane composition was 92% as measured by liquid chromatography.

Then, a heat-sensitive recording material was produced in the same manner as in Example 1 except for using the above-mentioned composition in place of the urea-urethane compound synthesized in Example 1, and was evaluated. The results obtained are summarized in Table 1.

### Example 10

To 17 g of 2,4-toluene diisocyanate was added 40 g of methyl ethyl ketone as a solvent, followed by adding dropwise thereto 3.8 g of methanol, and the reaction was carried out with stirring at 60°C for 5 hours. Then, 9.9 g of 4,4'-diaminodiphenyl sulfone was added thereto, and the reaction was carried out with stirring at 60°C for 4 hours. After completion of the reaction, the methyl ethyl ketone as solvent was removed by the use of an evaporator, and the residue was dried overnight in a vacuum to obtain 16 g of a urea-urethane composition as white crystals. The melting point of the white crystals was 169°C. In IR analysis on the crystals, characteristic peaks due to a urea-urethane compound appeared at 1660 cm⁻¹ and 1740 cm⁻¹. The content of the urea-urethane main constituent in the urea-urethane composition was 52% as measured by liquid chromatography.

Then, a heat-sensitive recording material was produced in the same manner as in Example 1 except for using the above-mentioned composition in place of the urea-urethane compound synthesized in Example 1, and was evaluated. The results obtained are summarized in Table 1.

### Comparative Example 1

A heat-sensitive recording material was produced in the same manner as in Example 1 except for using 2,2-bis(4-hydroxyphenyl)propane in place of the urea-urethane compound synthesized in Example 1, and was evaluated. The results obtained are summarized in Table 1.

### Comparative Example 2

To 17.4 g of 2,4-toluene diisocyanate was added 5 mL of methyl ethyl ketone as a solvent, followed by adding dropwise thereto a solution of 3.2 g of methanol in 5 mL of methyl ethyl ketone, and the reaction was carried out with stirring at room temperature for 2 hours. Then, a solution of 7.3 g of n-butylamine in 100 mL of methyl ethyl ketone was added dropwise thereto with stirring at room temperature, and the resulting mixture was stirred for 1 hour. The crystals precipitated were recovered by filtration, washed with hexane and then dried overnight in a vacuum to obtain 27 g of a compound as white crystals. The melting point of the white crystals was 156°C, and their analytical measurement was as follows.

### Result of IR measurement:

Characteristic peaks appeared at 1240 cm⁻¹, 1550 cm⁻¹, 1640 cm⁻¹, 1720 cm⁻¹ and 3300 cm⁻¹.

The presumed structural formula of the major component of this compound is the formula (R-1) shown hereinafter.

Then, a heat-sensitive recording material was produced in the same manner as in Example 1 except for using the compound obtained above, in place of the urea-urethane compound synthesized in Example 1, and was evaluated. The results obtained are summarized in Table 1.

### Comparative Example 3

To 10.0 g of 2,4-toluene diisocyanate was added 100 g of toluene. While stirring the resulting mixture at 25°C, a solution of 15.5 g of stearylamine in 100 mL of toluene was added thereto, and the reaction was continued at 25°C for 22 hours. After completion of the reaction, the white solid precipitated was recovered by filtration, washed with toluene and then dried overnight in a vacuum to obtain 20.4 g of white crystals. Thereafter, 5 g of the thus obtained compound was added to 50 mL of methyl ethyl ketone. While stirring the resulting mixture at 80°C, a solution of 8.6 g of p-hydroxybenzylcarboxylic acid in 20 ml of methyl ethyl ketone and then 5 mg of dibutyltin laurate as catalyst were added thereto, and the reaction was continued at 80°C for 12 hours. After completion of the reaction, the reaction mixture was cooled to room temperature, and the crystals precipitated were recovered by filtration, washed with methyl ethyl ketone and then dried overnight in a vacuum to obtain 5.6 g of white crystals.

Analytical measurements of these white crystals were as follows.

### Result of IR measurement:

Characteristic peaks appeared at 1220 cm⁻¹, 1520 cm⁻¹, 1630 cm⁻¹, 1710 cm⁻¹, 2900 cm⁻¹ and 3300 cm⁻¹. Result of mass spectrum measurement:
[M+H]⁺ was detected at m/z 596.

The presumed structural formula of the major component of this compound is the formula (R-2) shown hereinafter.

A heat-sensitive recording material was produced in the same manner as in Example 1 except for using the compound obtained above, in place of the urea-urethane compound synthesized in Example 1, and was evaluated. The results obtained are summarized in Table 1.

### Comparative Example 4

To 100 mL of dioxane was added 3.0 g of p-aminophenol. While stirring the resulting mixture at 50°C, a solution of 5.4 g of toluene sulfonylisocyanate in 30 mL of dioxane was added dropwise thereto over a period of 1 hour, and the reaction was continued at 50°C for 5 hours. After completion of the reaction, the reaction solution was concentrated and then poured into hexane to effect crystallization, and the solid precipitated was recovered by filtration, washed with hexane and then dried overnight in a vacuum to obtain 4.9 g of brown crystals. Thereafter, 2 g of the thus obtained compound was added to 50 mL of dioxane. While stirring the resulting mixture at 80°C, a solution of 3.8 g of octadecyl isocyanate in 10 mL of dioxane and then 2 mg of dibutyltin laurate as catalyst were added thereto, and the reaction was continued at 80°C for 20 hours. After completion of the reaction, the reaction mixture was cooled to room temperature, and the crystals precipitated were recovered by filtration, washed with dioxane and then dried overnight in a vacuum to obtain 2.7 g of slightly pink crystals.

Analytical measurement of these slightly pink crystals was as follows.

### Result of IR measurement:

Characteristic peaks appeared at 1230 cm⁻¹, 1470 cm⁻¹, 1510 cm⁻¹, 1570 cm⁻¹, 1620 cm⁻¹, 1700 cm⁻¹, 2900 cm⁻¹ and 3300 cm⁻¹.

The presumed structural formula of the major component of this compound is the formula (R-3) shown below.

A heat-sensitive recording material was produced in the same manner as in Example 1 except for using the compound obtained above, in place of the urea-urethane compound synthesized in Example 1, and was evaluated. The results obtained are summarized in Table 1.

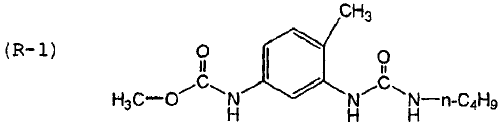

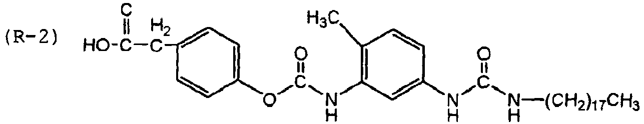

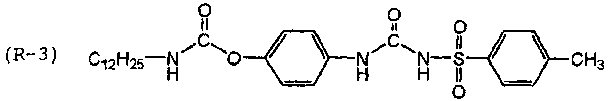

### Comparative Example 5

To 10 g of 2,4-toluene diisocyanate was added 50 g of toluene as a solvent, followed by adding thereto 30 g of aniline, and the reaction was carried out at 25°C for 3 hours. After completion of the reaction, the white solid precipitated was recovered by filtration, washed with hexane and then dried overnight in a vacuum to obtain 17 g of a compound as white crystals.

The presumed structural formula of the major component of this compound is the structural formula of the compound (C-1) shown hereinafter.

Next, 2 g of this compound was ground together with 8 g of a 2.5 wt% poly(vinyl alcohol) aqueous solution in a paint shaker for 45 minutes to be dispersed, whereby a dispersion was obtained.

Then, a heat-sensitive recording material was produced in the same manner as in Example 1 except for using this dispersion of said compound in place of the dispersion of the compound obtained in Example 1, and was evaluated. The results obtained are summarized in Table 1.

### Comparative Example 6

To 10 g of 2,4-toluene diisocyanate was added 30 g of toluene as a solvent, followed by adding thereto 30 g of phenol, and the reaction was carried out at 100°C for 3 hours. After completion of the reaction, the toluene was removed by concentration and hexane was added to the residue, and the white solid precipitated was recovered by filtration, washed with hexane and then dried overnight in a vacuum to obtain 15 g of a compound as white crystals.

The presumed structural formula of the major component of this compound is the structural formula of the compound (C-2) shown below.

Next, 2 g of the obtained compound was ground together with 8 g of a 2.5 wt% poly(vinyl alcohol) aqueous solution in a paint shaker for 45 minutes to be dispersed, whereby a dispersion was obtained.

Then, a heat-sensitive recording material was produced in the same manner as in Example 1 except for using this dispersion of said compound in place of the dispersion of the compound obtained in Example 1, and was evaluated. The results obtained are summarized in Table 1.

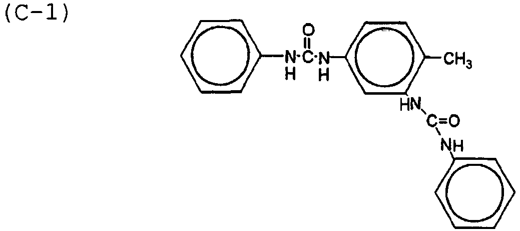

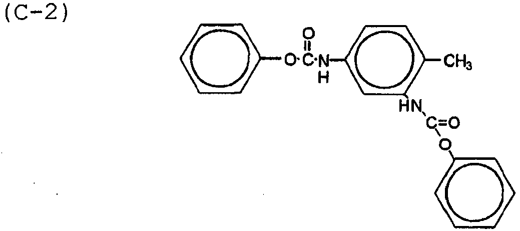

### Comparative Example 7

A heat-sensitive recording material was produced in the same manner as in Example 1 except for using 1,3-diphenylurea in place of the urea-urethane compound synthesized in Example 1, and was evaluated. The results obtained are summarized in Table 1.

**Table 1**

| | Color development sensitivity of thermal paper | Plasticizer resistance (print preservability) | Heat resistance | | Total evaluation |
|---|---|---|---|---|---|
| | | | Print preservability at 60°C | Preservability of original recording material surface at 80°C | |
| Example 1 | 1.2 | Δ | ⊚ | ○ | ○ |
| Example 2 | 1.2 | ⊚ | ⊚ | ○ | ⊚ |
| Example 3 | 1.1 | ○ | ⊚ | ○ | ○~⊚ |
| Example 9 | 1.2 | Δ | ⊚ | ○ | ○ |
| Example 10 | 1.2 | ⊚ | ⊚ | ○ | ⊚ |
| Comparative Example 1 | 1.3 | × | Δ~○ | × | × |
| Comparative Example 2 | 0.3 | × | × | Δ | × |
| Comparative Example 3 | 0.3 | × | Δ | ○ | × |
| Comparative Example 4 | 0.4 | × | Δ | ○ | × |
| Comparative Example 5 | 0.5 | × | × | ○ | × |
| Comparative Example 6 | 0.4 | × | × | ○ | × |
| Comparative Example 7 | 0.6 | × | × | Δ | × |

1. Sensitivity becomes higher with an increase of optical density (OD value).
2. Plasticizer resistance (print preservability)
   ⊚ ~ Substantially no fading.
   ○ ~ A slight color tone change without blur and the like.
   Δ ~ Marked fading.
   × ~ Complete loss of the color of print.
3. Heat resistance (print preservability at 60°C)
   ⊚ ~ Substantially no fading.
   ○ ~ A slight color tone change without blur and the like.
   Δ ~ Marked fading.
   × ~ Complete loss of the color of print.
4. Heat resistance (the preservability of an original recording material surface at 80°C)
   ⊚ ~ Substantially no fog was caused.
   ○ ~ Reading of a print portion was possible though there was a slight color tone change.
   Δ ~ Reading of a print portion was difficult owing to fog.
   × ~ Reading of a print portion was impossible owing to serious fog.

### Example 87

A dispersion was prepared by dispersing 2 g of the compound obtained in Example 2, by grinding it together with 8 g of a 2.5 wt% aqueous solution of methyl cellulose (Metlose SM-15, mfd. by Shin-Etsu Chemical Co., Ltd.) in a paint shaker for 6 hours.

Another dispersion was prepared by dispersing 70 g of 3-dibutylamino-6-methyl-7-anilinofluoran by grinding it together with 130 g of a 5.4 wt% poly(vinyl alcohol) aqueous solution in a sand grinder (mfd. by AIMEX CO., LTD.; vessel capacity 400 ml) at a number of revolution of 2,000 rpm for 3 hours.

Further another dispersion was prepared by dispersing 70 g of diphenyl sulfone by grinding it together with 130 g of a 5.4 wt% aqueous solution of a poly(vinyl alcohol) (Gohsenol KL-05, mfd. by The Nippon Synthetic Chemical Industry Co., Ltd.) in a sand grinder (mfd. by AIMEX CO., LTD.; vessel capacity 400 ml) at a number of revolution of 2,000 rpm for 3 hours.

Still another dispersion was prepared by mixing 10 g of calcium carbonate with 30 g of water and stirring the mixture by the use of a stirrer.

A coating liquid was obtained by stirring and mixing the above-mentioned dispersions and other components in the following proportions (dry basis proportions); the dispersion of the above-mentioned compound in terms of dry solids: 30 parts by weight, the 3-dibutylamino-6-methyl-7-anilinofluoran dispersion in terms of dry solids: 15 parts by weight, the diphenyl sulfone dispersion in terms of dry solids: 30 parts by weight, the calcium carbonate dispersion in terms of dry solids: 20 parts by weight, a zinc stearate dispersion (solid content: 16 wt%) in terms of dry solids: 10 parts by weight, and a 15 wt% poly(vinyl alcohol) in terms of dry solids: 7 parts by weight.

Then, the coating liquid was applied on the surface of woodfree paper with a basis weight of 50 g/m² in an amount of 5 g/m² in terms of dry weight and dried, followed by supercalendering, to produce a heat-sensitive recording material, and the recording material was evaluated. The results obtained are summarized in Table 4.

### Examples 99 to 101

Heat-sensitive recording materials were produced in the same manner as in Example 87 except for using methyl cellulose (Metlose SM-15, mfd. by Shin-Etsu Chemical Co., Ltd.) (Example 99), hydroxypropylmethyl cellulose (Metlose 60SH-03, mfd. by Shin-Etsu Chemical Co., Ltd.) (Example 100) or a modified poly(vinyl alcohol) (Gohseran L-3266, mfd. by The Nippon Synthetic Chemical Industry Co., Ltd.) (Example 101) in place of the poly(vinyl alcohol) (Gohsenol KL-05, mfd. by The Nippon Synthetic Chemical Industry Co., Ltd.) used as a dispersing agent for diphenyl sulfone in Example 87, and the heat-sensitive recording materials were evaluated. The results obtained are summarized in Table 4.

### Example 102

A heat-sensitive recording material was produced in the same manner as in Example 99 except for using a modified poly(vinyl alcohol) (Gohseran L-3266, mfd. by The Nippon Synthetic Chemical Industry Co., Ltd.) in place of the methyl cellulose (Metlose SM-15, mfd. by Shin-Etsu Chemical Co., Ltd.) used as a dispersing agent for the urea-urethane compound in Example 99, and the recording material was evaluated. The results obtained are summarized in Table 4.

### Comparative Example 10

A heat-sensitive recording sheet was produced in the same manner as in Example 87 except for using 2,2-bis(4-hydroxyphenyl)propane in place of the urea-urethane compound used in Example 87, and was evaluated. The results obtained are summarized in Table 4.

**Table 4**

| | Sensitivity of thermal paper | Plasticizer resistance (print preservability) | Plasticizer resistance (preservability of original recording material surface) | Total evaluation |
|---|---|---|---|---|
| Example 87 | 1.3 | ○~⊚ | ⊚ | ○~⊚ |
| Example 99 | 1.3 | ⊚ | ⊚ | ⊚ |
| Example 100 | 1.3 | ⊚ | ⊚ | ⊚ |
| Example 101 | 1.4 | ⊚ | ⊚ | ⊚ |
| Example 102 | 1.4 | ⊚ | ⊚ | ⊚ |
| Comparative Example 10 | 1.3 | × | ○ | × |

1. Sensitivity becomes higher with an increase of optical density (OD value).
2. Plasticizer resistance (print preservability)
   ⊚ ~ Substantially no fading.
   ○ ~ A slight color tone change without blur and the like.
   Δ ~ Marked fading.
   × ~ Complete loss of the color of print.
3. Plasticizer resistance (the preservability of an original recording material surface)
   ⊚ ~ The original recording material surface is hardly colored.
   ○ ~ The original recording material surface is very slightly colored though there is a subtle color tone change.
   Δ ~ The original recording material surface is markedly colored.
   × ~ The original recording material surface is so seriously colored that reading of print is difficult.

### INDUSTRIAL APPLICABILITY

Employment of a specific urea-urethane compound makes it possible to provide at a low price a color-producing composition and a heat-sensitive recording material which are excellent in image preservability and color development sensitivity.

## Claims

1. A urea-urethane compound which is represented by any one of the following formulae (S-1) to (S-25), (S-28) to (S-60) and (S-63) to (S-70):

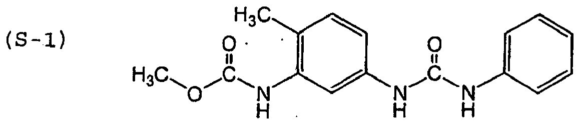

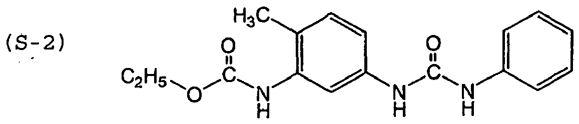

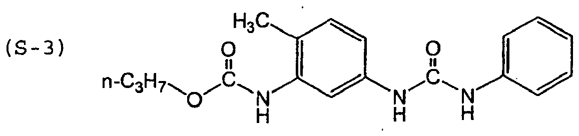

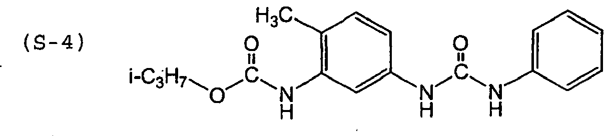

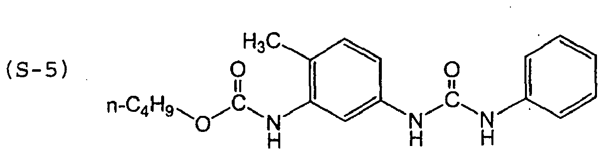

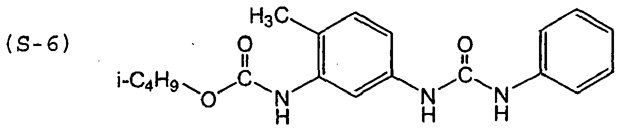

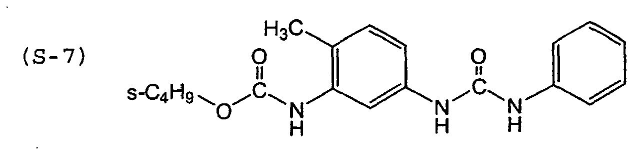

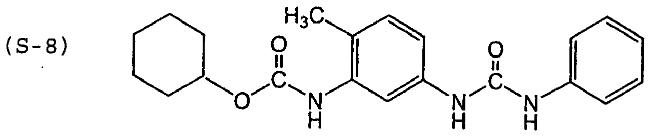

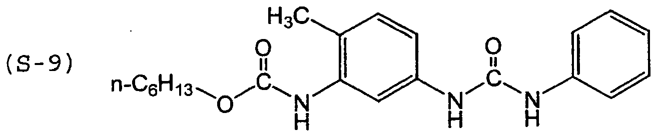

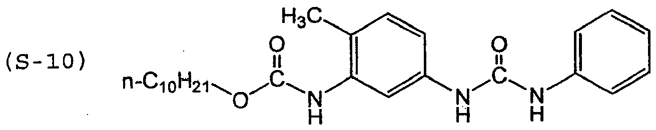

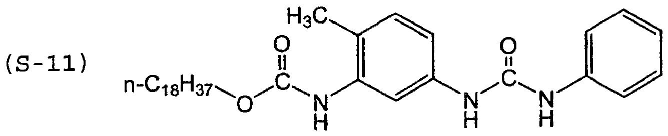

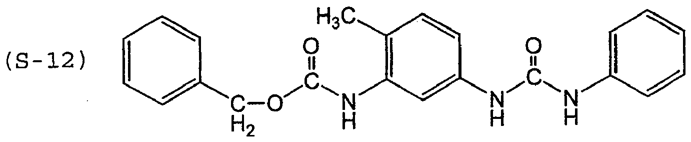

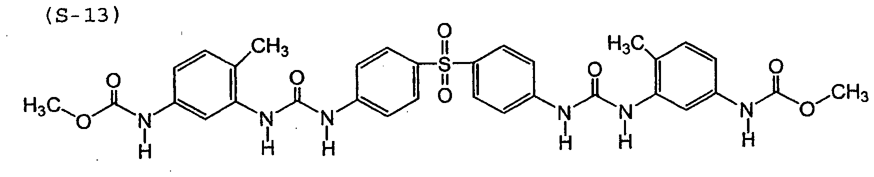

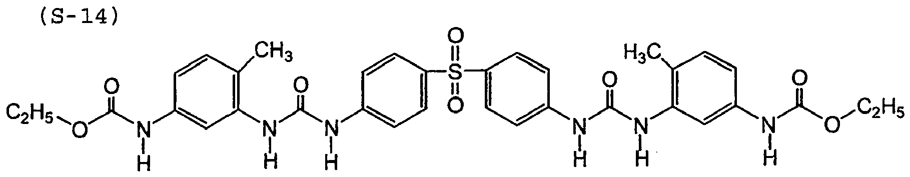

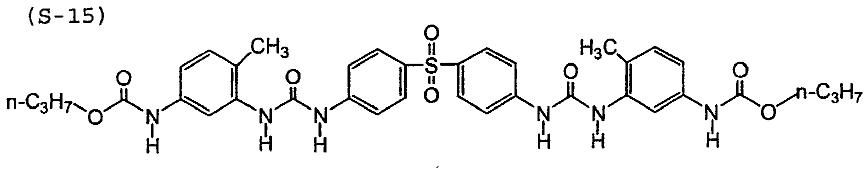

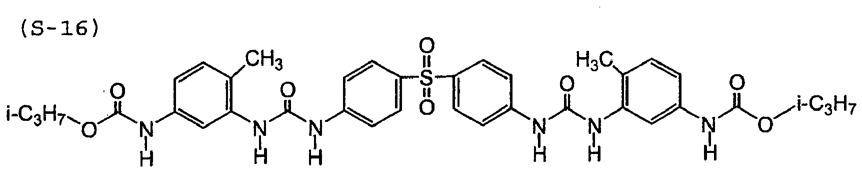

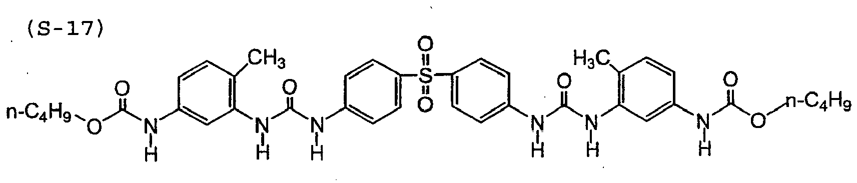

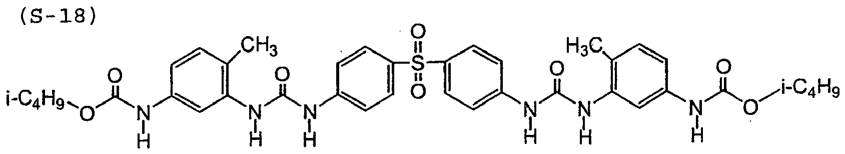

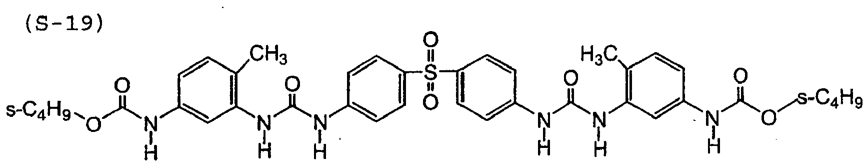

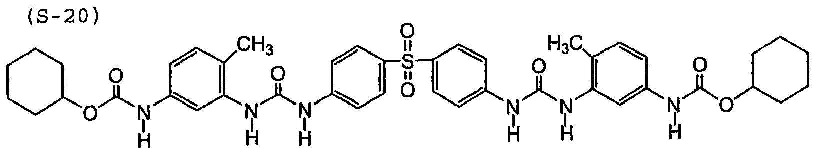

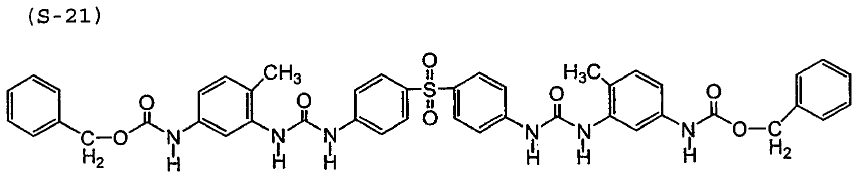

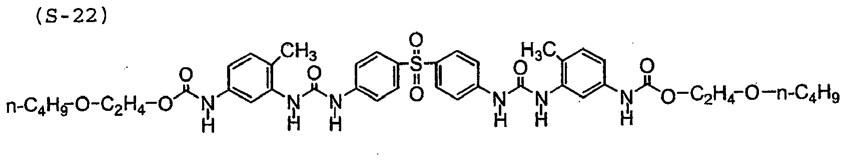

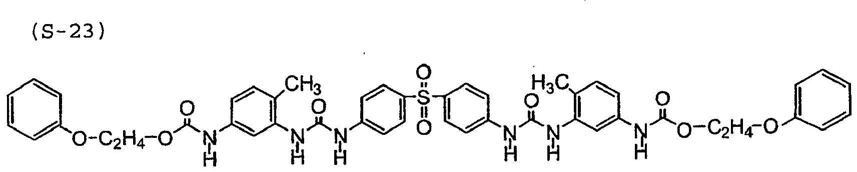

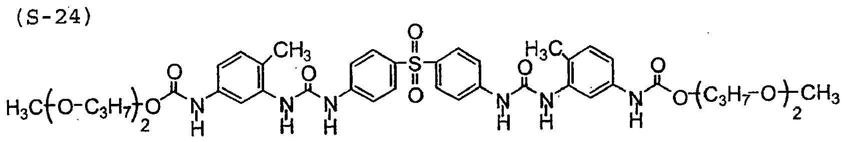

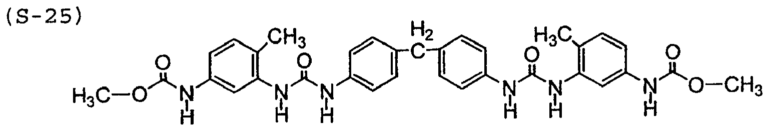

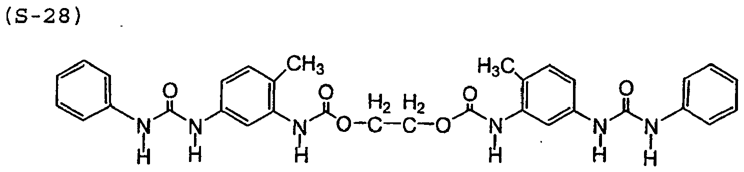

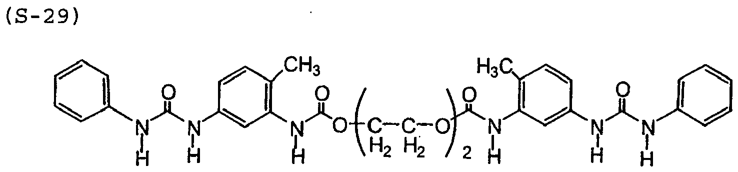

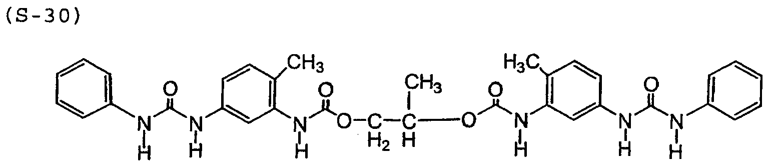

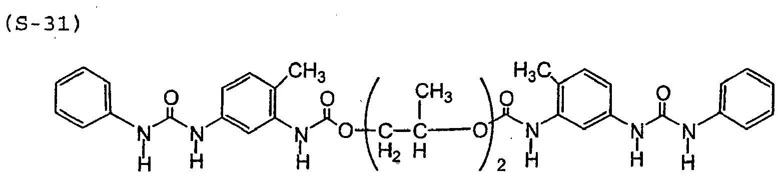

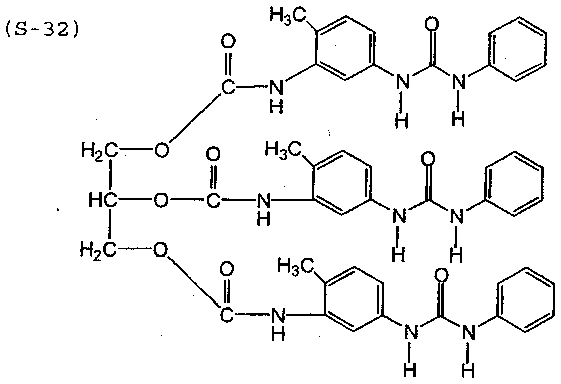

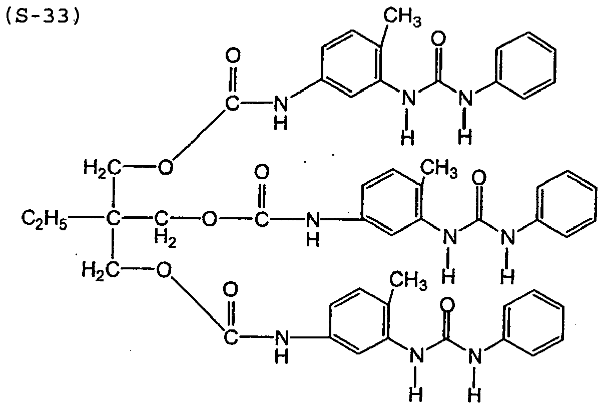

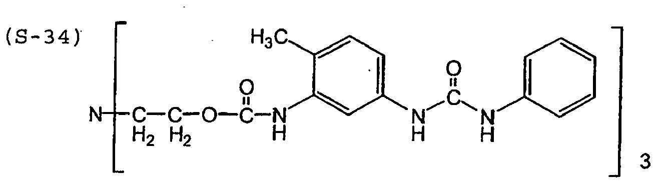

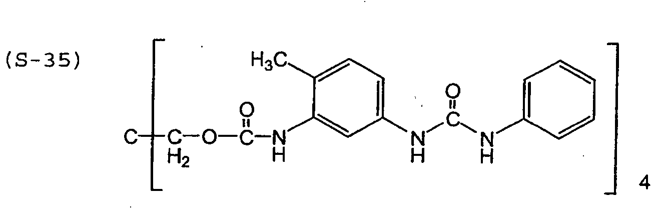

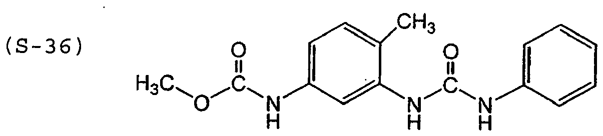

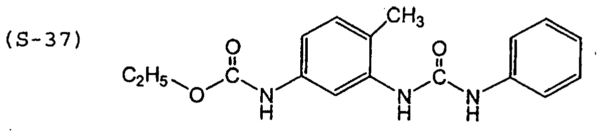

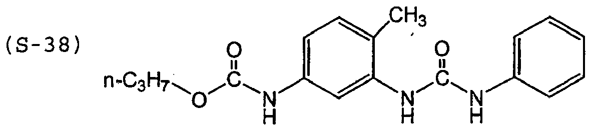

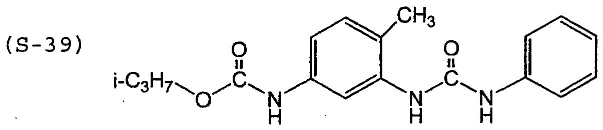

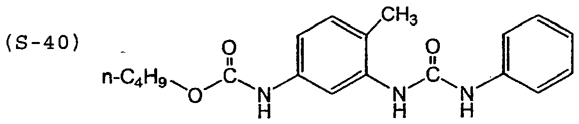

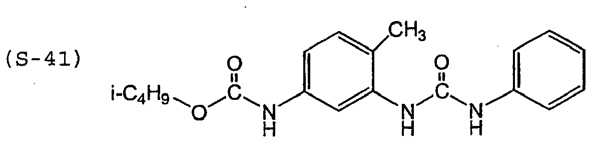

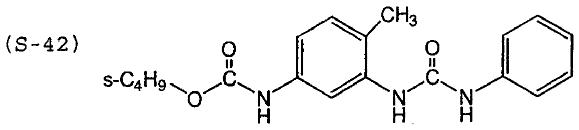

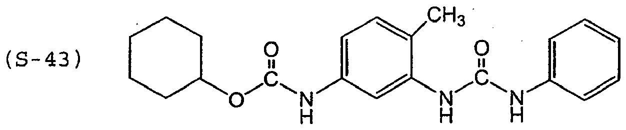

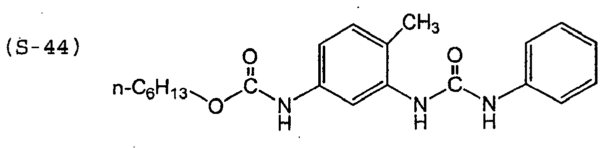

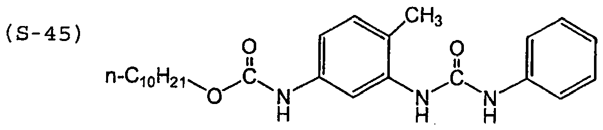

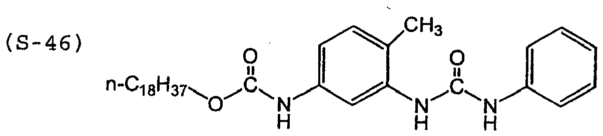

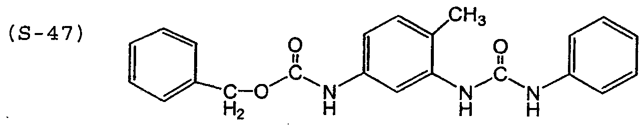

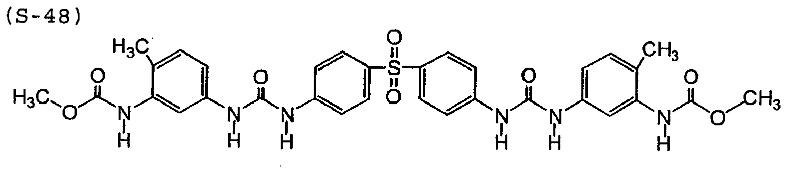

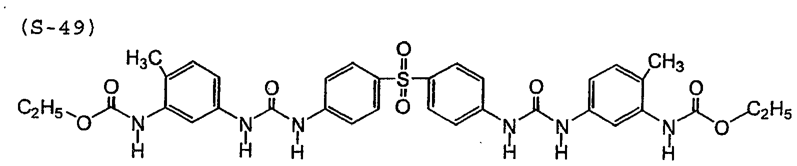

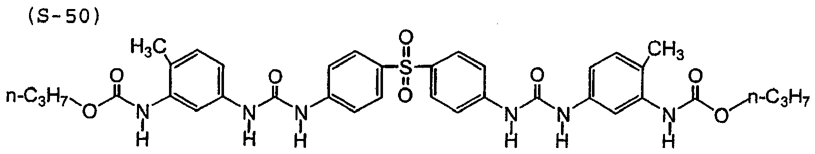

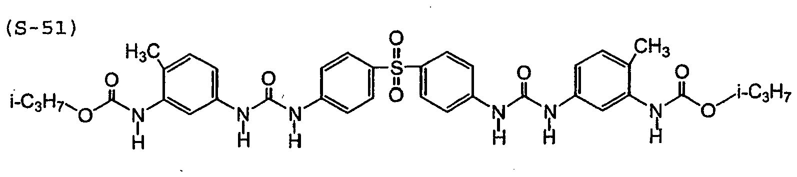

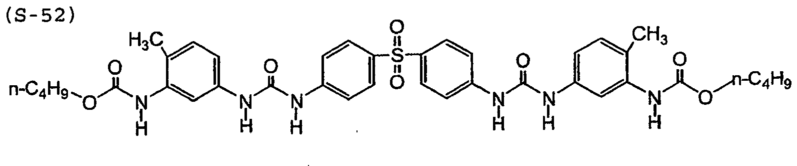

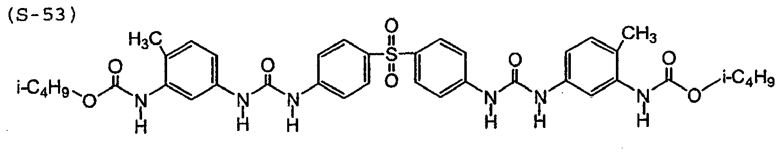

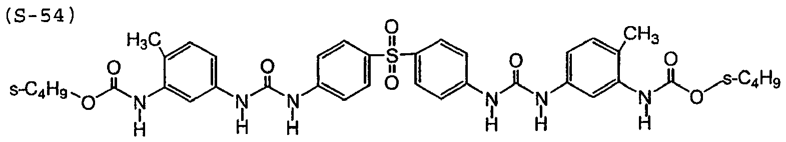

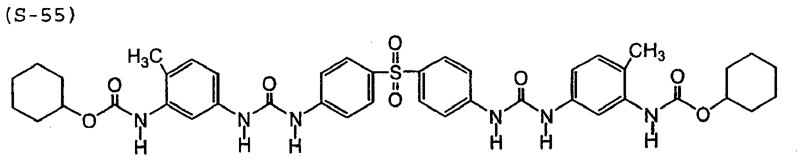

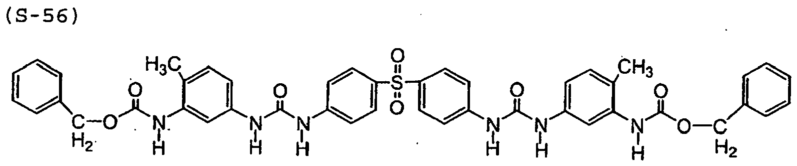

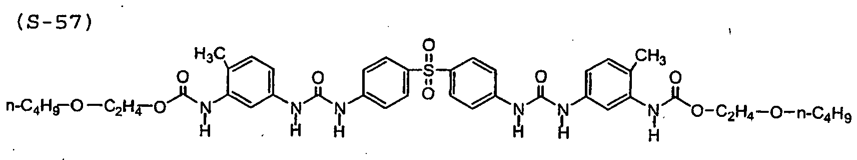

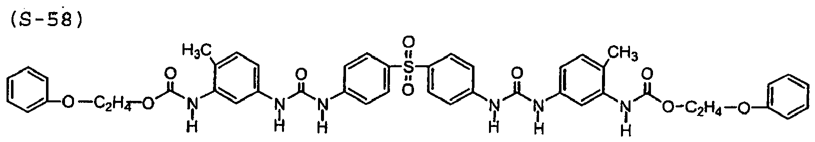

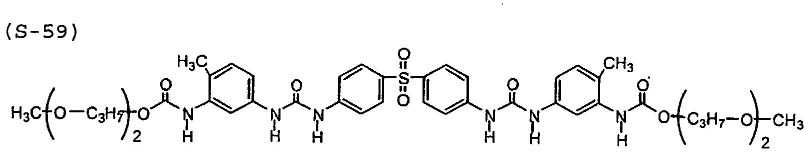

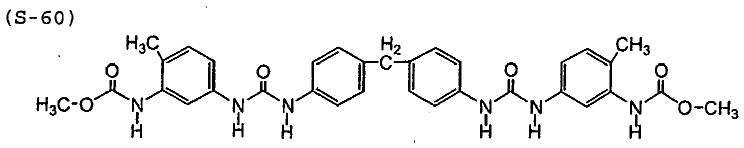

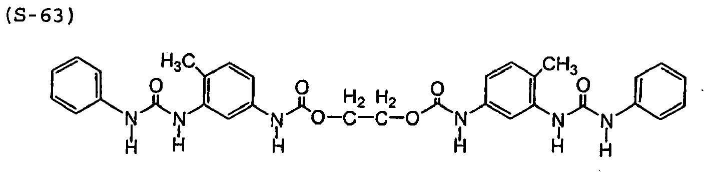

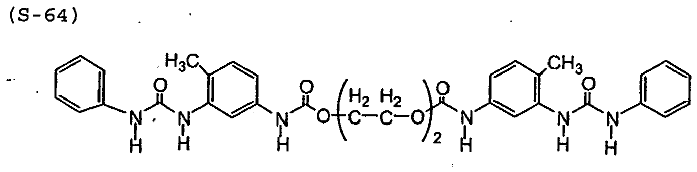

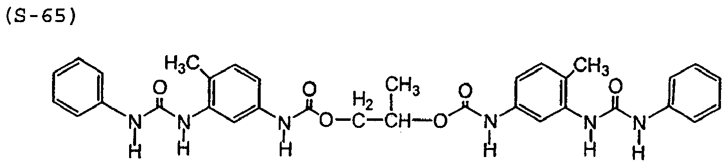

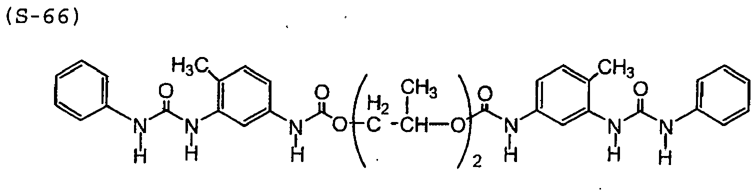

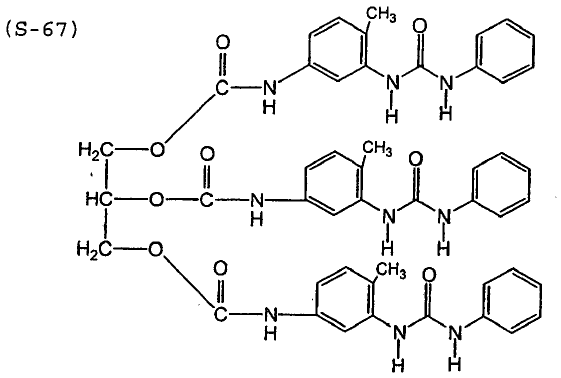

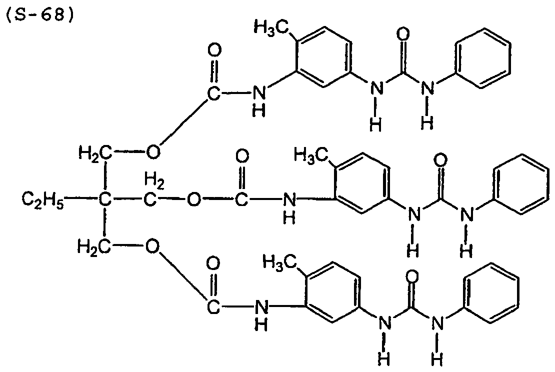

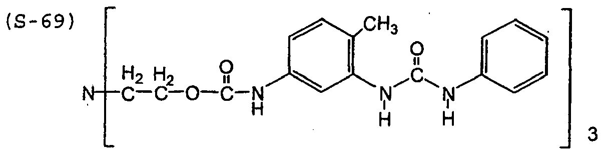

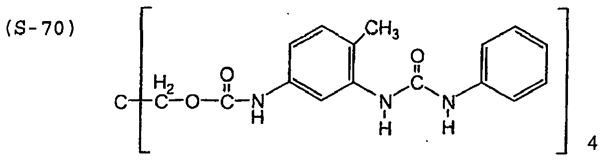

2. A color-producing composition comprising a developer comprising a urea-urethane compound according to claim 1, and a colorless or light-colored dye precursor.

3. A color-producing composition according to claim 2, wherein the developer further comprises an acidic developer.

4. Use of the color-producing composition according to claim 2 or 3 for the production of a heat-sensitive recording material.

## Patentansprüche

1. Urea-Urethanverbindung, welche durch eine der folgenden Formeln (S-1) bis (S-25), (S-28) bis (S-60) und (S-63) bis (S-70) dargestellt ist:

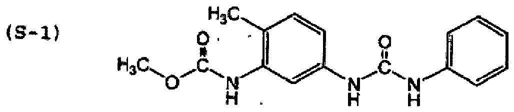

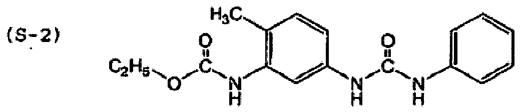

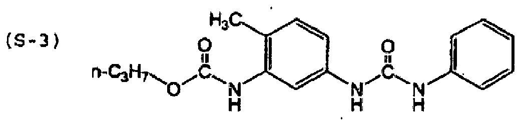

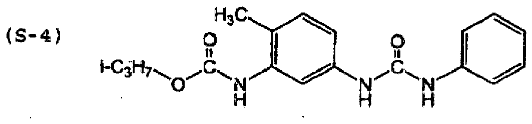

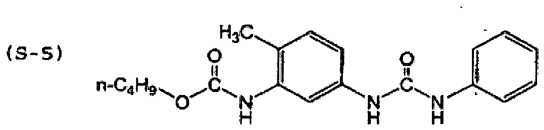

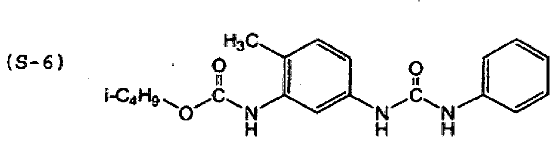

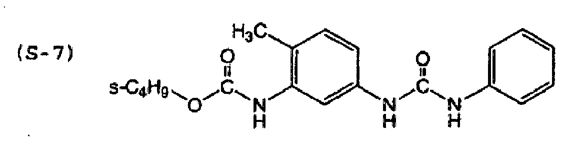

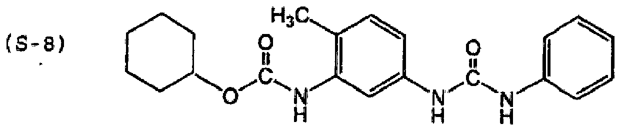

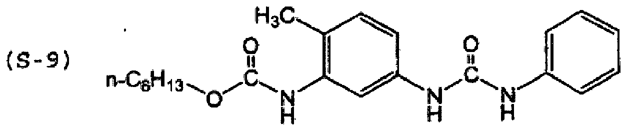

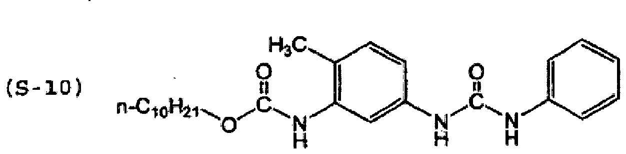

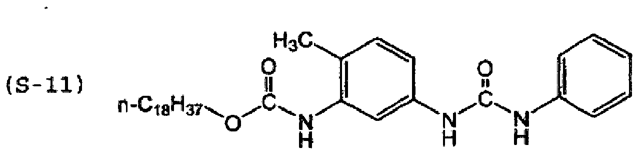

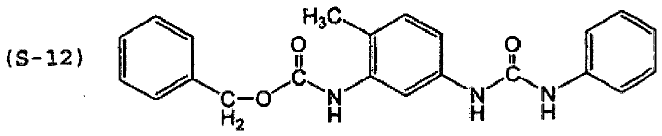

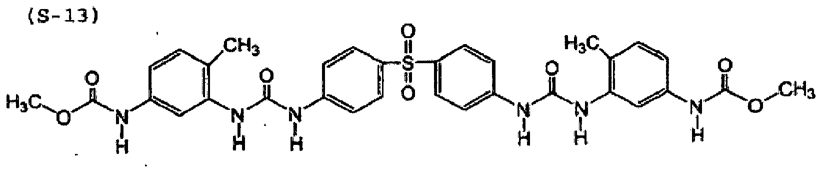

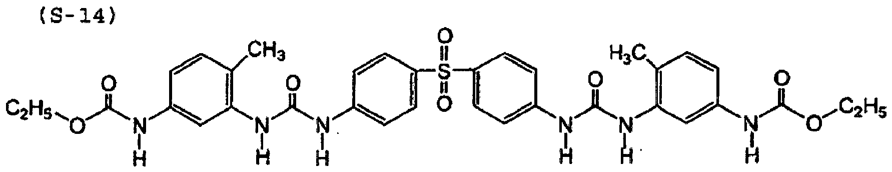

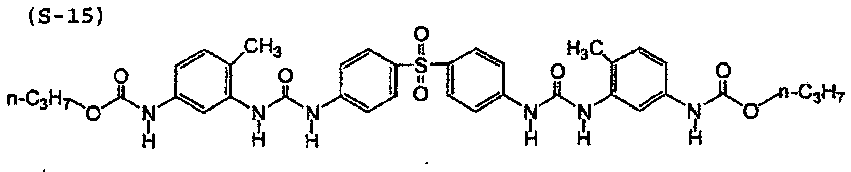

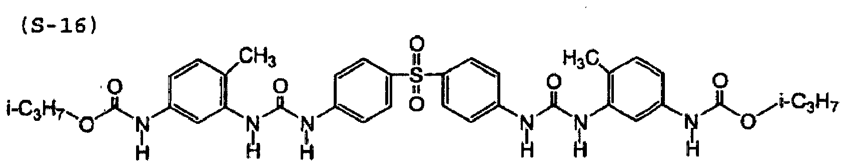

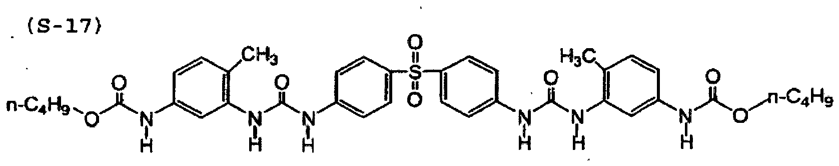

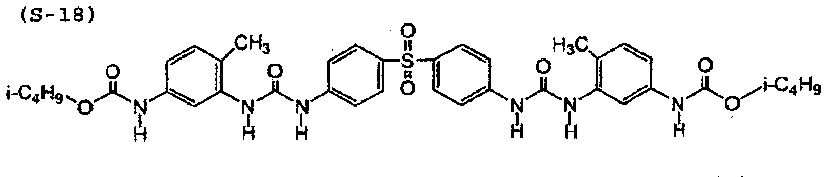

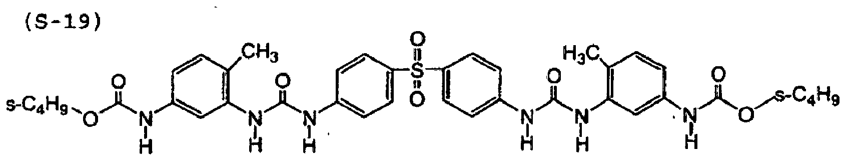

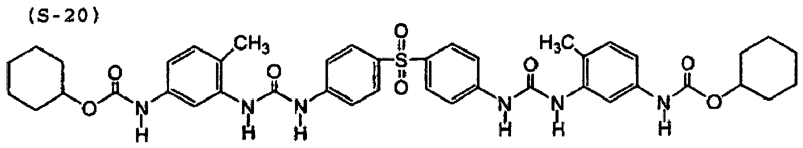

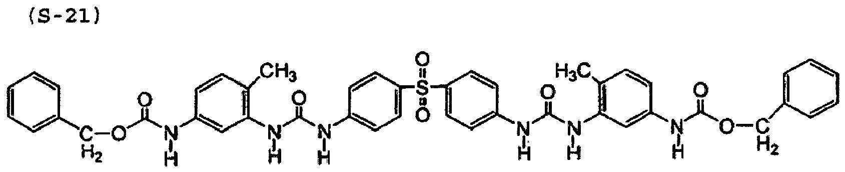

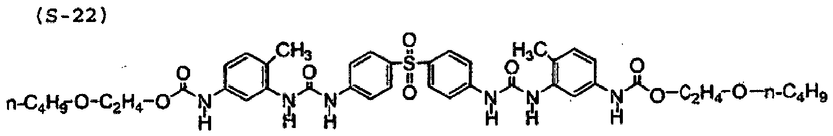

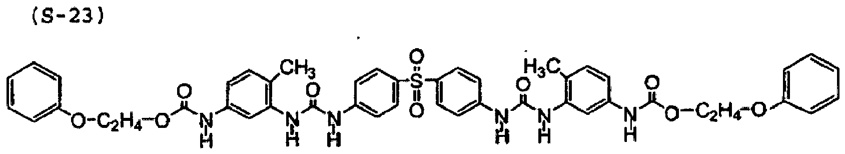

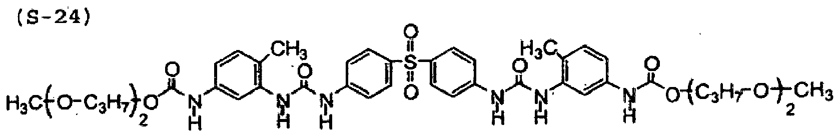

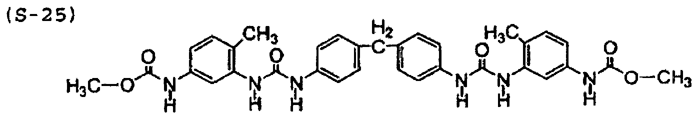

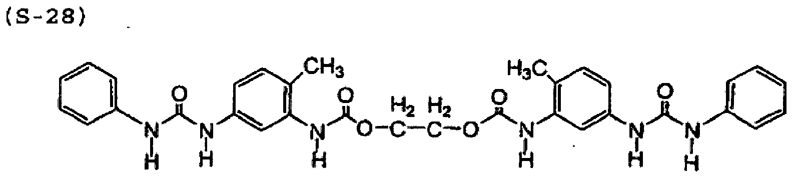

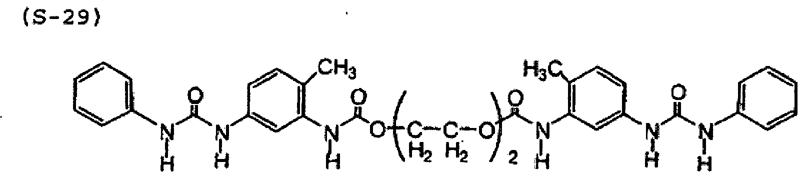

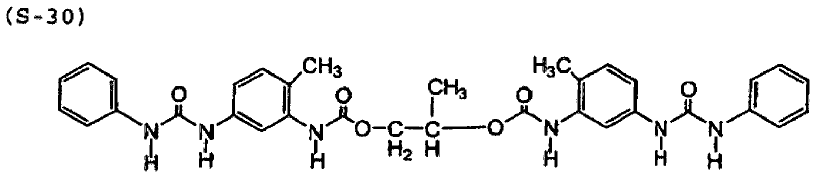

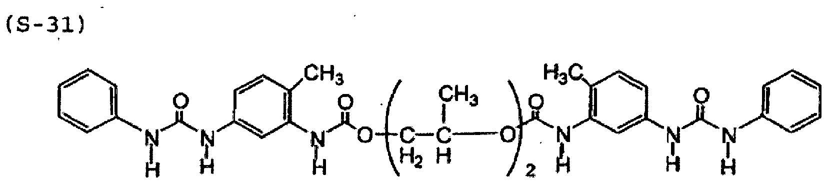

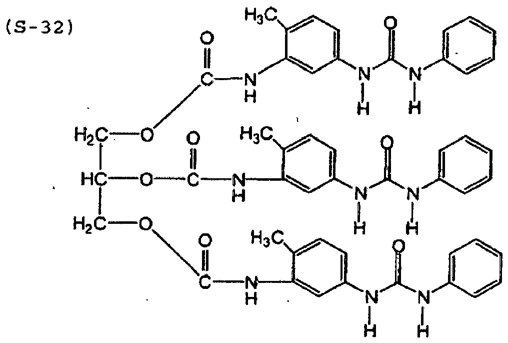

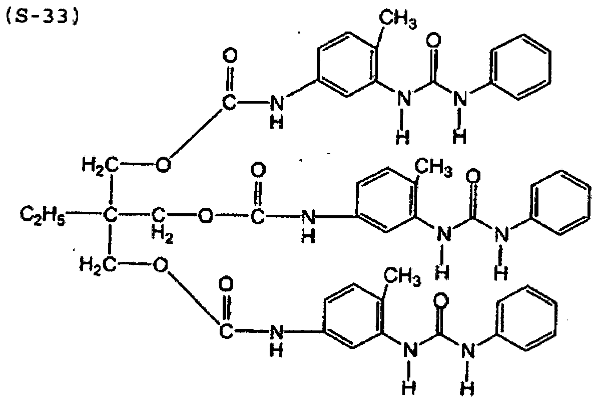

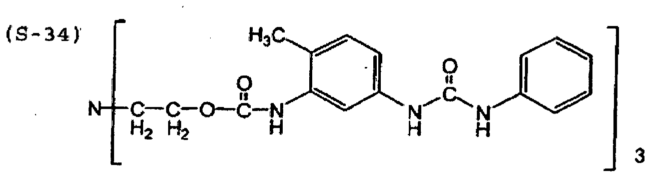

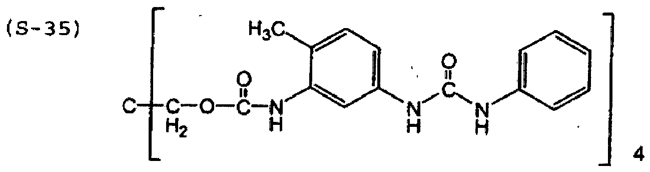

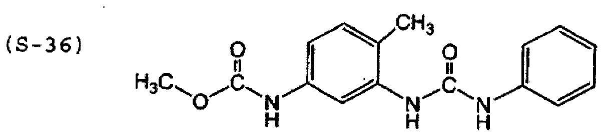

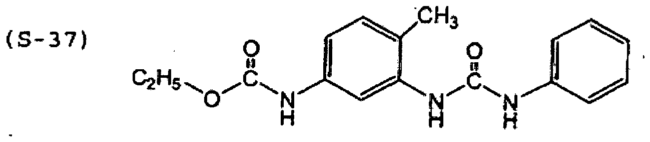

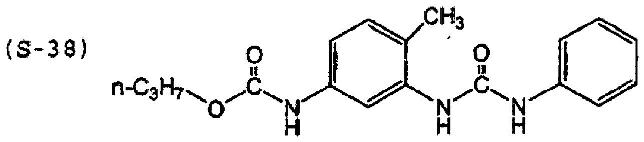

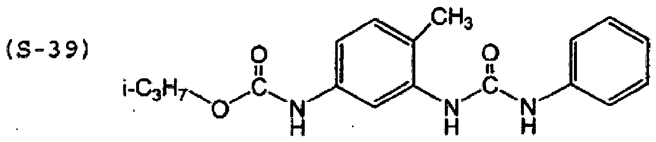

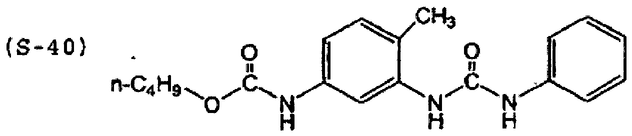

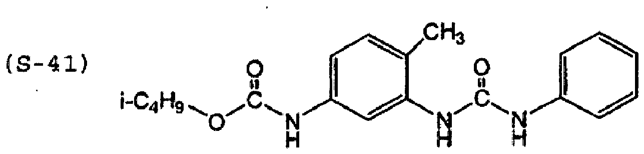

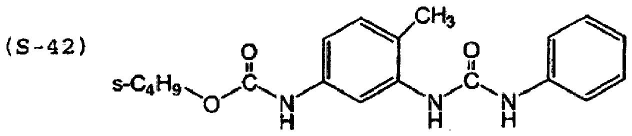

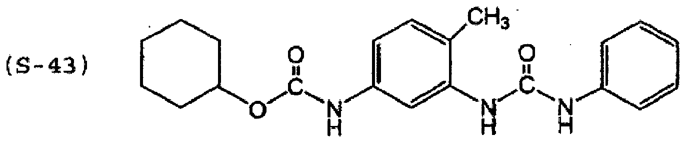

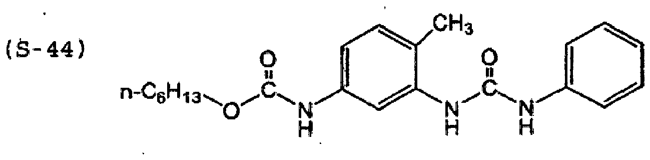

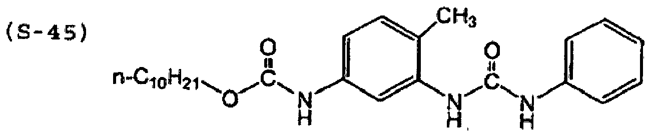

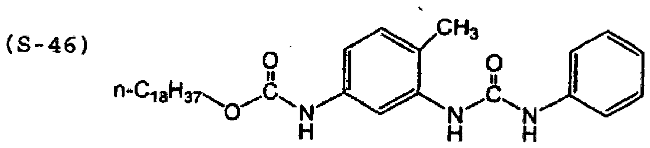

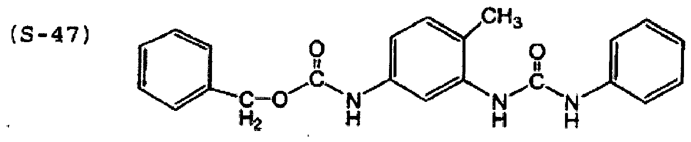

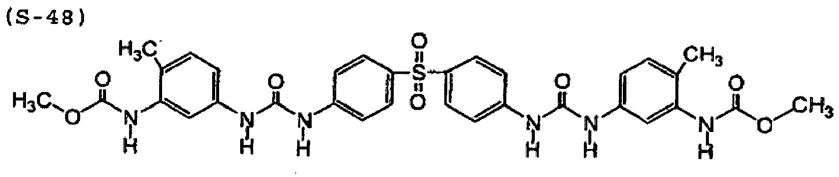

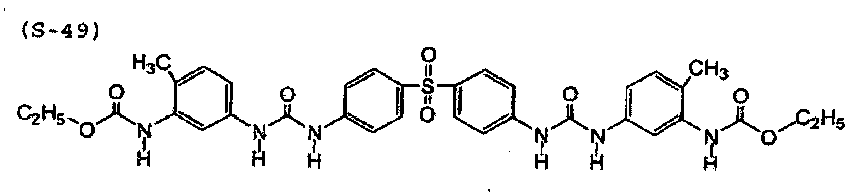

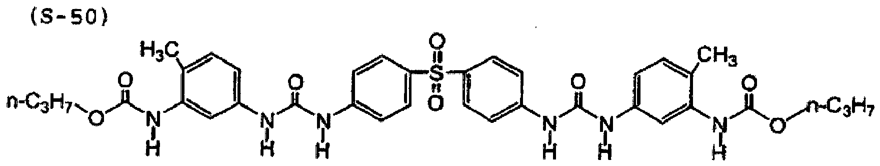

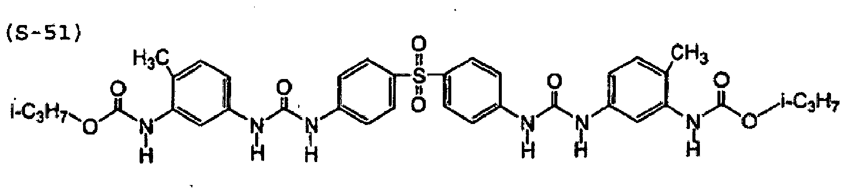

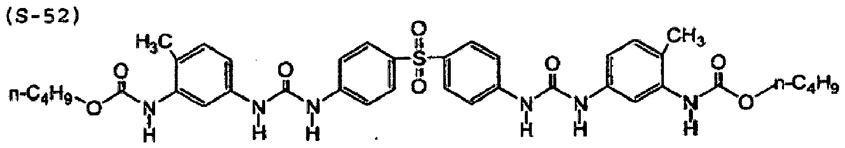

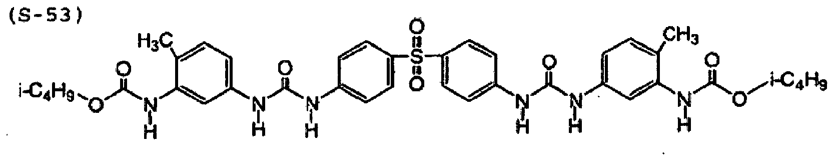

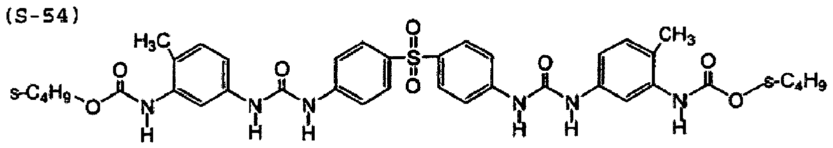

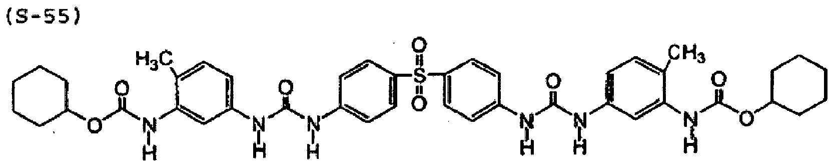

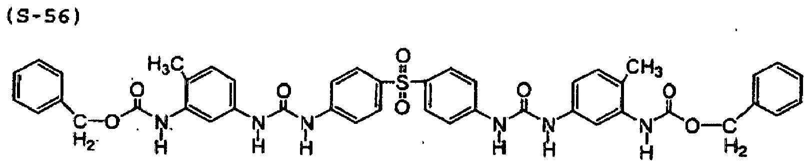

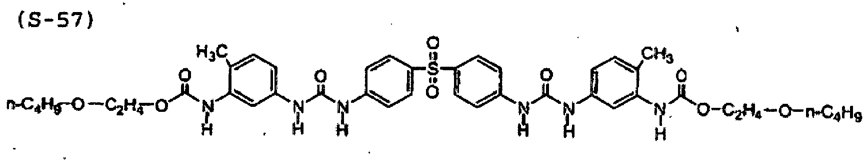

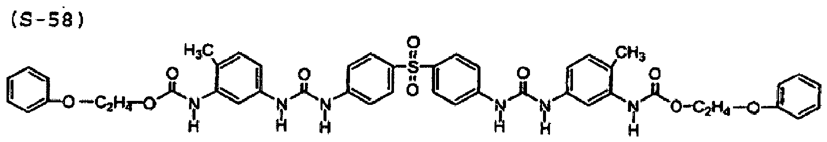

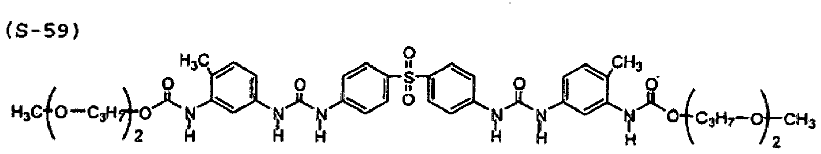

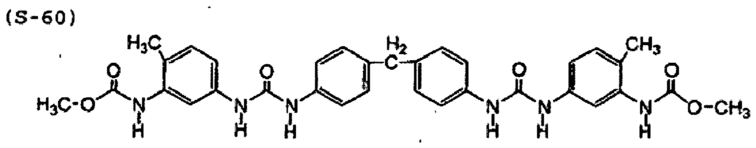

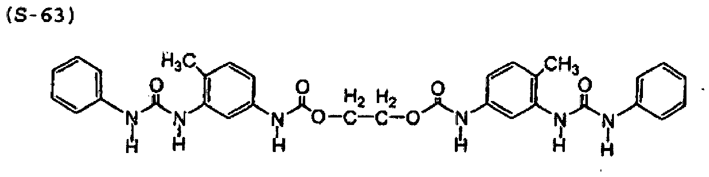

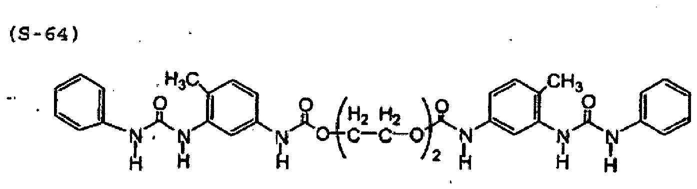

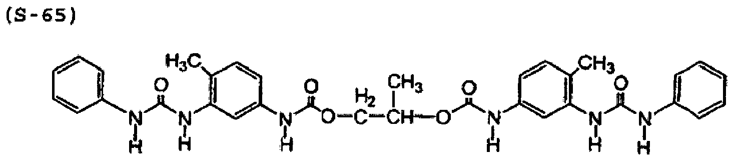

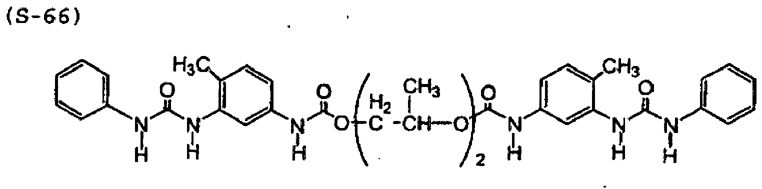

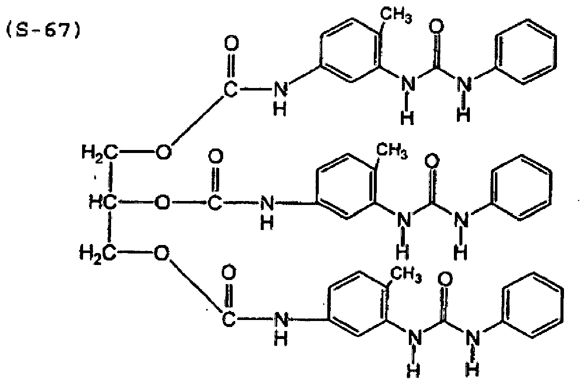

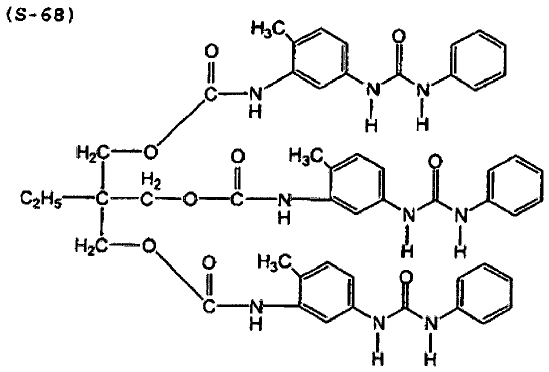

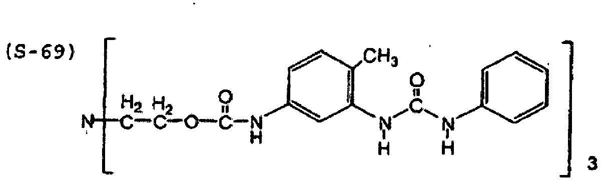

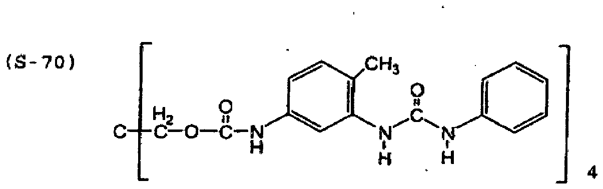

2. Farbproduzierende Zusammensetzung , umfassend einen Entwickler, welcher eine Urea-Urethanverbindung nach Anspruch 1 und einen farblosen oder schwach gefärbten Farbstoffvorläufer umfasst.

3. Farbproduzierende Zusammensetzung nach Anspruch 2, wobei der Entwickler weiterhin einen sauren Entwickler umfasst.

4. Verwendung der farbproduzierenden Zusammensetzung nach Anspruch 2 oder 3 zur Herstellung eines wärmeempfindlichen Aufzeichnungsmaterials.

## Revendications

1. Composé d'urée-uréthane représenté par l'une quelconque des formules (S-1) à (S-25), (S-28) à (S-60) et (S-63) à (S-70) suivantes :

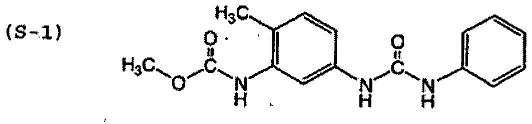

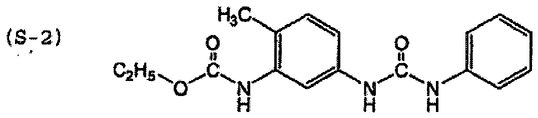

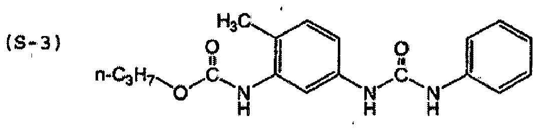

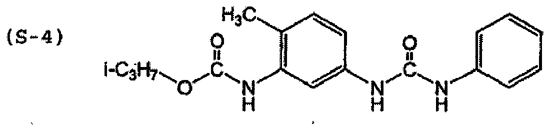

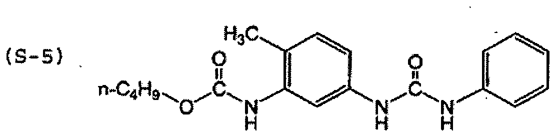

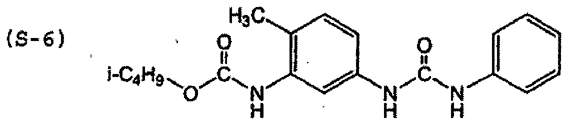

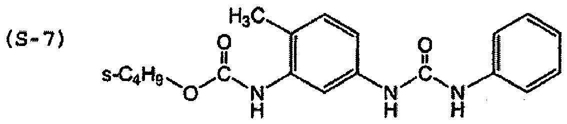

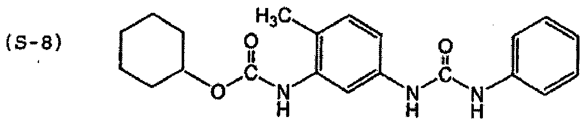

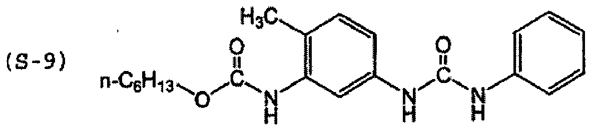

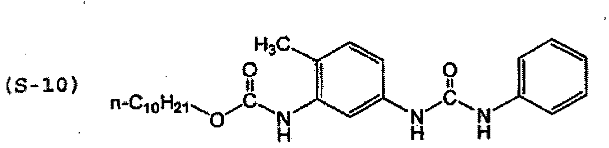

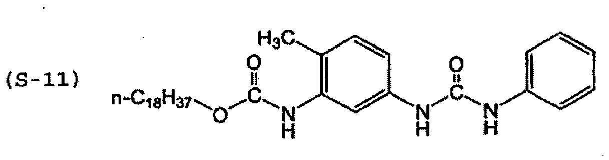

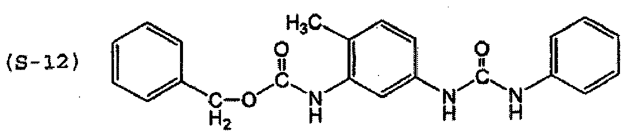

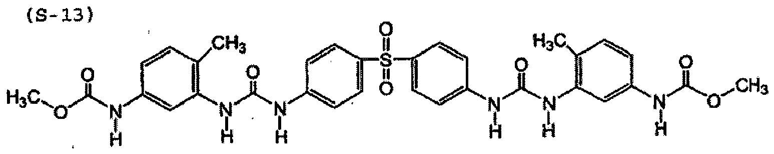

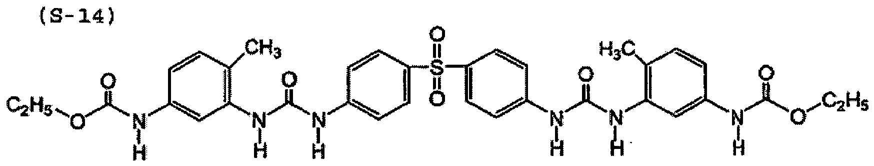

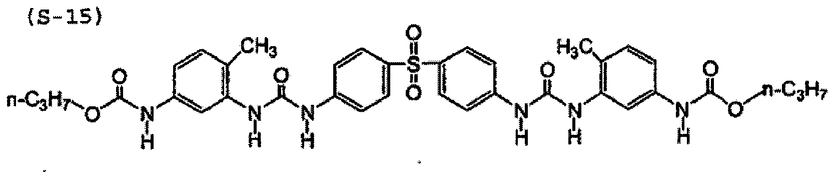

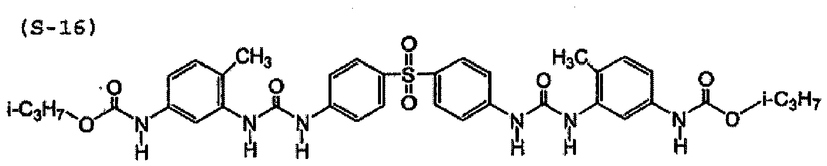

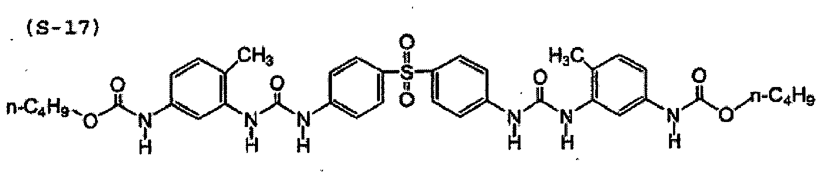

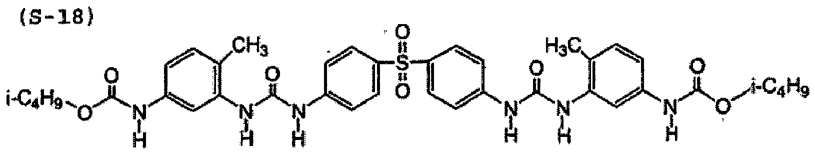

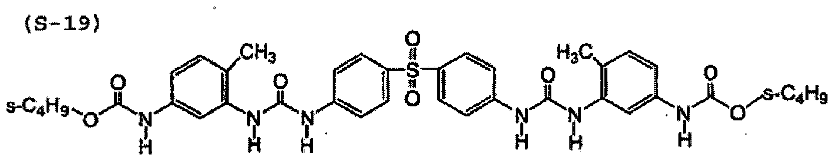

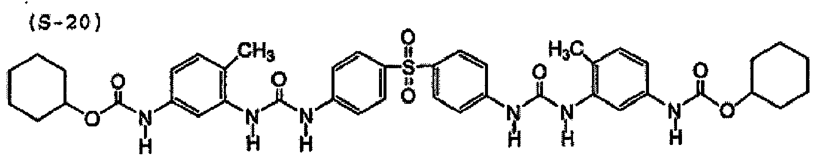

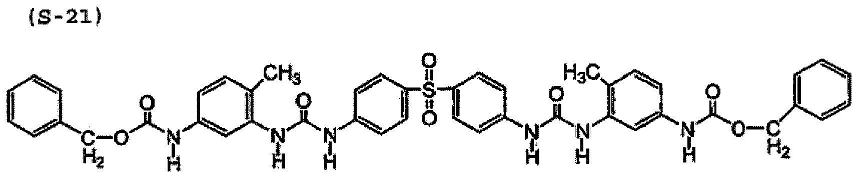

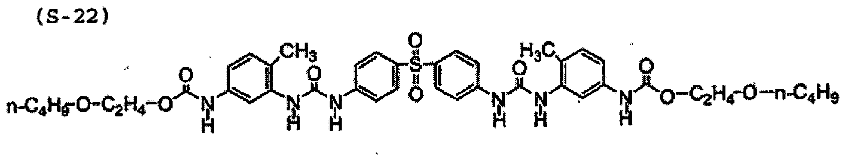

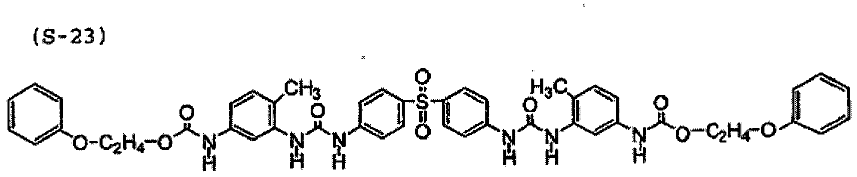

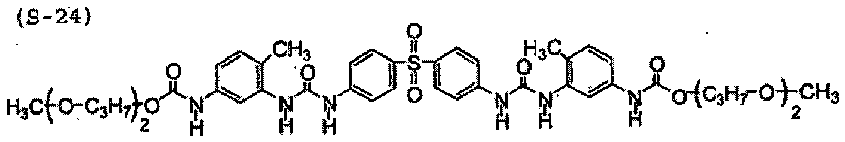

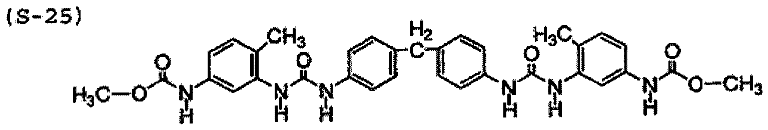

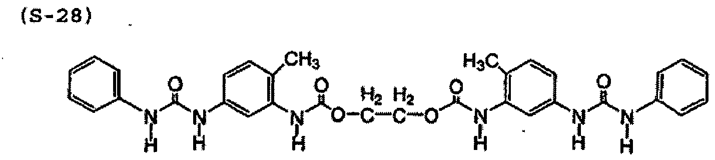

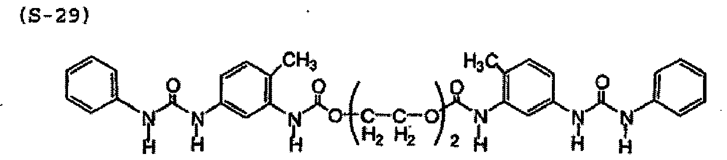

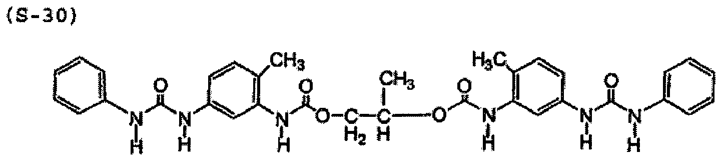

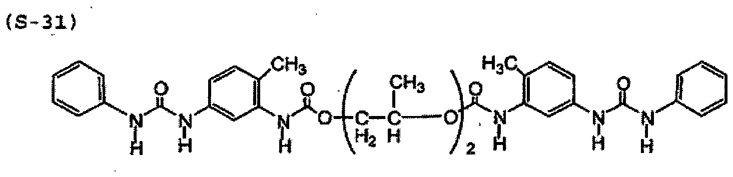

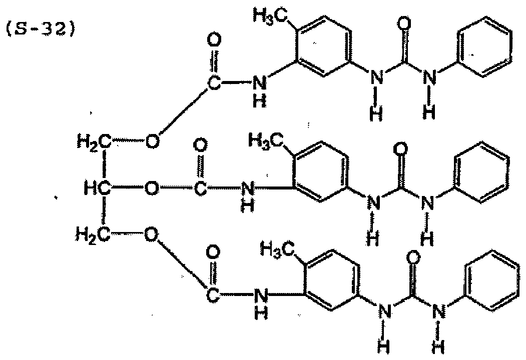

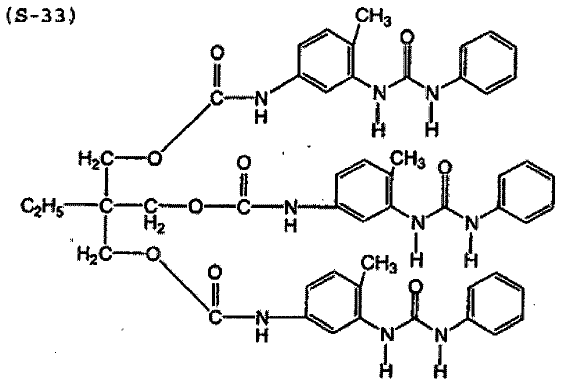

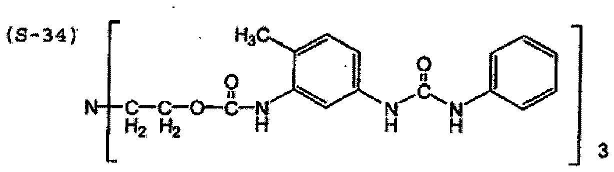

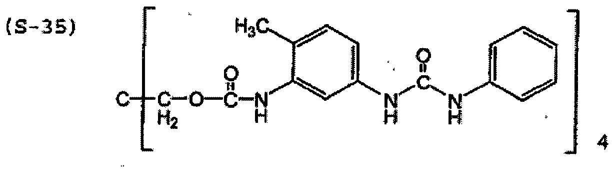

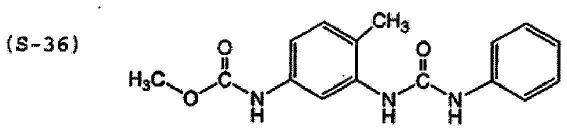

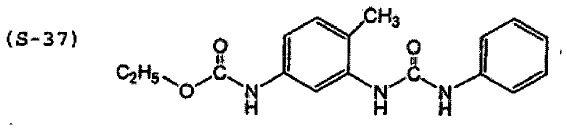

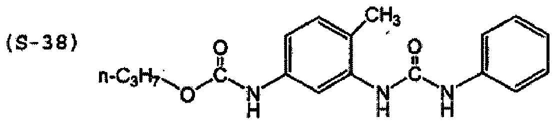

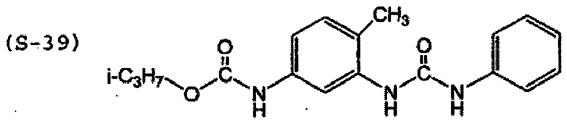

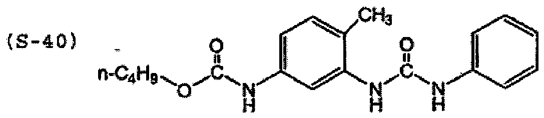

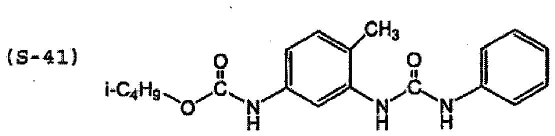

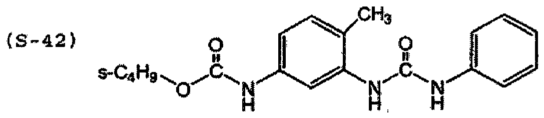

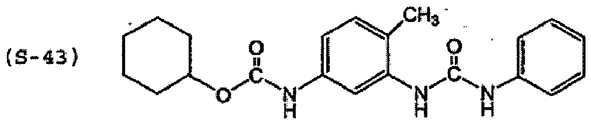

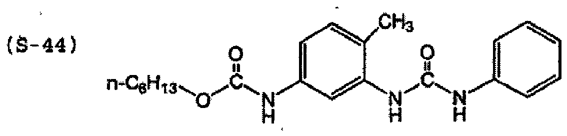

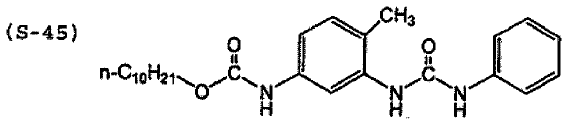

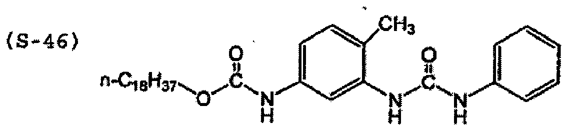

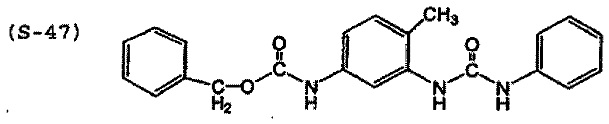

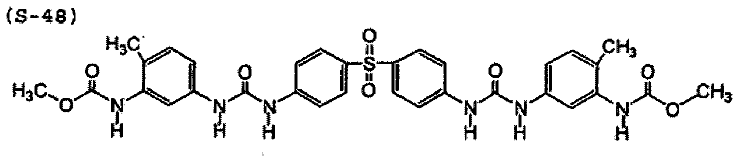

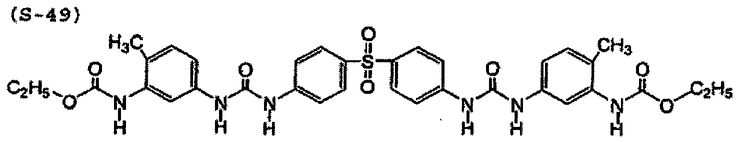

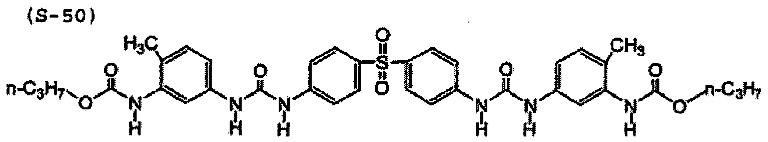

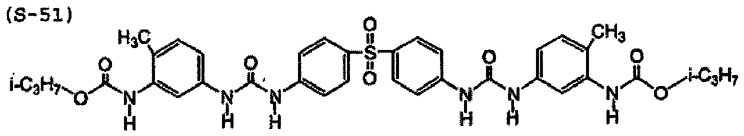

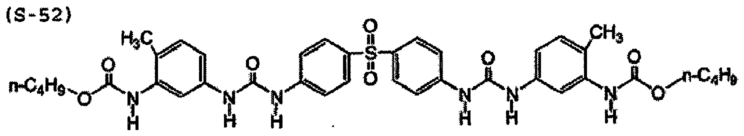

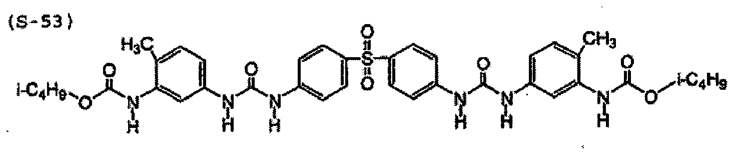

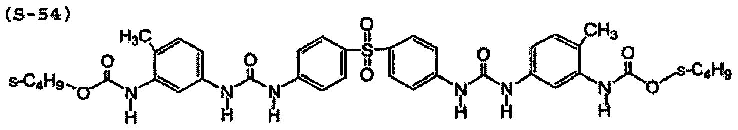

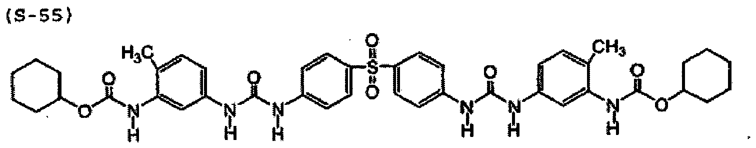

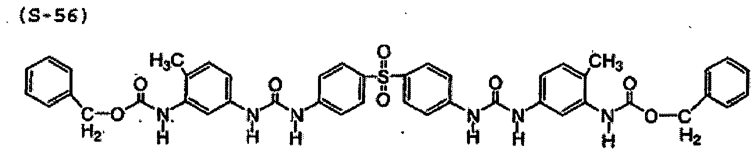

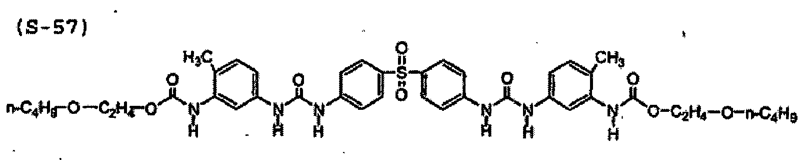

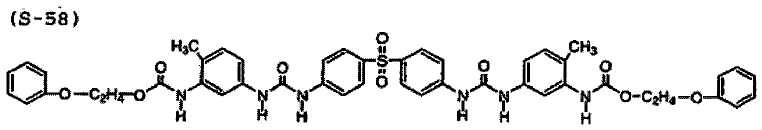

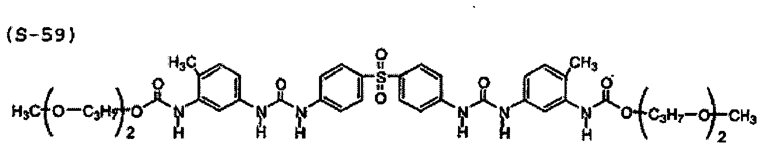

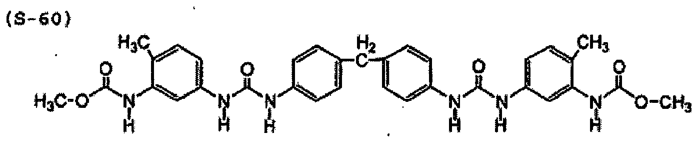

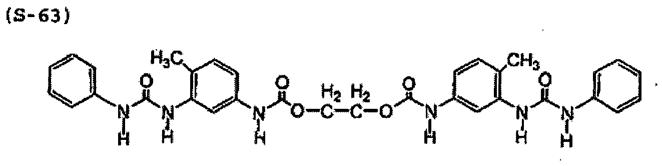

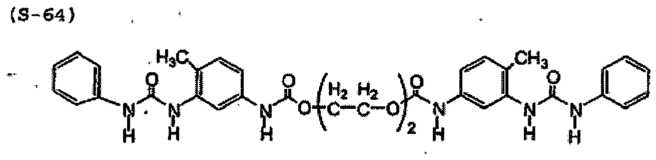

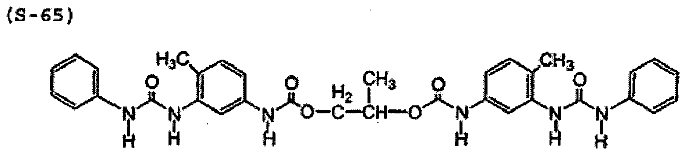

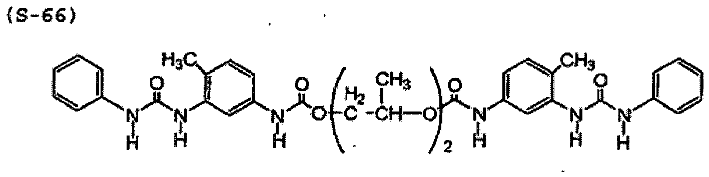

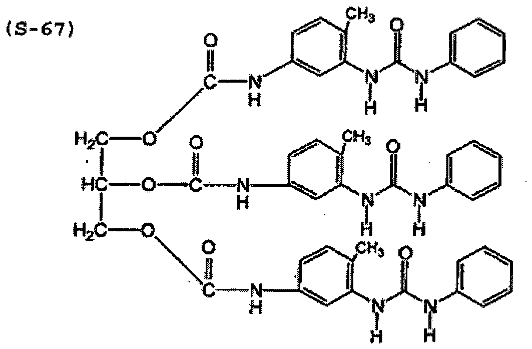

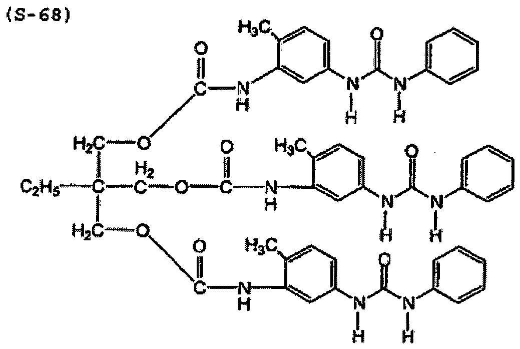

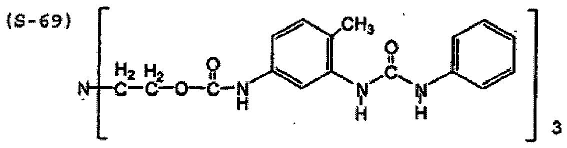

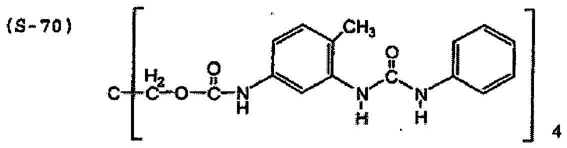

2. Composition produisant une couleur, comprenant un révélateur comprenant un composé d'urée-uréthane selon la revendication 1, et un précurseur de colorant incolore ou légèrement coloré.

3. Composition produisant une couleur selon la revendication 2, dans laquelle le révélateur comprend en outre un révélateur acide.

4. Utilisation de la composition produisant une couleur selon la revendication 2 ou 3 pour la production d'un matériau d'enregistrement sensible à la chaleur.
